(19) Europäisches Patentamt
European Patent Office
Office européen des brevets

(11) EP 4 471 014 A1

(12) EUROPEAN PATENT APPLICATION

(43) Date of publication:
**04.12.2024 Bulletin 2024/49**

(21) Application number: **23175917.6**

(22) Date of filing: **29.05.2023**

(51) International Patent Classification (IPC):
***C07D 241/36*** (2006.01) ***C07D 403/10*** (2006.01)

(52) Cooperative Patent Classification (CPC):
**C07D 403/10; C07D 241/36**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Uniwersytet Gdanski**
**80-309 Gdansk (PL)**

(72) Inventors:
- **Serdiuk, Illia**
  **Gdansk (PL)**
- **Monka, Michal**
  **Gdansk (PL)**
- **Grzywacz, Daria**
  **Gdansk (PL)**

(74) Representative: **Pawlowska-Bajerska, Justyna**
**Kancelaria Patentowa Justyna Pawlowska**
**Wzgorze Bernardowo 263B/6**
**81-583 Gdynia (PL)**

# (54) DERIVATE OF QUINOXALINE WITH THERMALLY ACTIVATED DELAYED FLUORESCENCE

(57) The invention relates to the triarylamine derivatives of quinoxaline with thermally activated delayed fluorescence (TADF) bearing heavy atoms and methods of their synthesis. The emitters are characterized by spectral and photophysical properties with improved TADF parameters as compared to their analogues reported before. The disclosed emitters can convert triplet excitons into singlet ones faster and with higher efficiency. The emitters cover orange, red, and/or near infrared (NIR) spectral ranges. Furthermore, the mixtures of new emitters with fluorescent dopants exhibit narrow-band NIR emission preserving fast triplet-singlet exciton conversion.

The invention concerns functional organic materials which exhibit TADF and can be potentially used for the electroluminescence applications, e.g. in organic light emitting diodes (OLED), and/or applications of materials with long-lived excited states, e.g. photosensibilization, photocatalysis, and/or other fields where excited triplet states or excitons play important role.

EP 4 471 014 A1

## Description

The invention relates to the triarylamine derivatives of quinoxaline with thermally activated delayed fluorescence (TADF) bearing heavy atoms and methods of their synthesis. The emitters are characterized by spectral and photophysical properties with improved TADF parameters as compared to their analogues reported before. The disclosed emitters can convert triplet excitons into singlet ones faster and with higher efficiency. The emitters cover orange, red, and/or near infrared (NIR) spectral ranges. Furthermore, the mixtures of new emitters with fluorescent dopants exhibit narrowband NIR emission preserving fast triplet-singlet exciton conversion.

The invention concerns functional organic materials which exhibit TADF and can be potentially used for the electroluminescence applications, e.g. in organic light emitting diodes (OLED), and/or applications of materials with long-lived excited states, e.g. photosensibilization, photocatalysis, and/or other fields where excited triplet states or excitons play important role.

TADF emitters have recently gained a lot of attention mainly thanks to their potential application in OLEDs. Such organic emitters are capable of efficient triplet harvesting and can achieve 100% internal quantum efficiency of electroluminescence. In optoelectronic devices, TADF emitters can be used directly for light emission or as assistant dopants in combination with narrow-band fluorophores for achieving high color purity.

The key photophysical parameters of TADF emitters are photoluminescence quantum yield (PLQY), lifetime of prompt fluorescence (PF, $\tau_{PF}$) and delayed fluorescence (DF, $\tau_{DF}$) rate constants of radiative deactivation ($k_r$), intersystem crossing (ISC, $k_{ISC}$), and reverse intersystem crossing (rISC, $k_{rISC}$). The $k_{ISC}$ value describes the rate of transformation of singlet excited state to the triplet one. The $k_{rISC}$ value describes the rate of reverse process, so-called triplet harvesting, and, in the case of use of emitter in OLED devices, has strong influence on the external quantum efficiency; $\tau_{DF}$ can be described as a measure of time needed for triplet harvesting.

Due to a broad emission spectrum, to achieve high color purity, TADF emitters are used in combination with narrow-band emission fluorophores. This approach was called hyperfluorescence. In such mixtures triplet harvesting still occurs on the TADF emitter, but light is emitted from the fluorophore additive in the result of Förster resonance energy transfer.

From the point of view of highly required red and NIR organic emitters or mixtures, one of the best emitters or their mixtures are mentioned below.

US2019330162 discloses structure and TADF properties of red/NIR emitters based on dicyanodibenzo[a,c] phenazine acceptor and their use in OLEDs, the examples of photoluminescent and electroluminescent properties of the emitters Px-CNBQx, Ds-CNBQx, Ac-CNBQx, Cz-CNBQx, Px-CNBPz, Da-CNBPz, Ac-CNBPz are described; the industrial applicability of these group of compounds as light emitting materials in light emitting elements or devices is shown. However, prior industrial application in the photophysical properties of these emitters should be improved, namely, $k_{rISC}$ should be increased and $\tau_{DF}$ should be decreased. The color purity should also be improved. In spite of mentioning halogens as possible substituents at positions $R_1$ and $R_2$, it is not disclosed that introduction of bromine and/or iodine affords much higher rates of rISC and higher efficiency to triplet harvesting.

As one of the best examples of organic fluorophores capable of electronic transitions in NIR region, TWI659092 discloses pyrrolopyrrole type dyes with NIR absorption and use of those as a component of NIR absorption filter for a NIR sensor device. Document J. Brodeur, L. Hu, A. Malinge, E. Eizner, W. G. Skene, S. Kéna-Cohen. Highly Efficient and Spectrally Narrow Near-Infrared Fluorescent OLEDs Using a TADF-Sensitized Cyanine Dye. Adv. Opt. Mater. 2019, 7, 1901144.disclose structure of BPPC pyrrolopyrrole-type NIR fluorophore and its use in hyperfluorescent NIR OLED devices. WO2016084008 discloses the use of such fluorophores for heating purposes, but not for generating light.

The goal of this invention is to provide light emitting materials and light emitting elements in red and NIR regions. Fluorescent OLEDs are capable of maximum internal quantum efficiency equal to or below 25% because of loses of triplet excitons. Therefore organic fluorophores like BPPC which do not use triplet exciton energy for light emission are not useful for the state-of-the-art organic light emitting devices and need an assistant dopant for triplet harvesting, for example a TADF emitter.

Most of all-organic TADF emitters, including the above mentioned ones, show $k_{rISC}$ value in the range of $1\times10^3$-$10^5$ $s^{-1}$, and $\tau_{DF}$ in the range of milli- to microseconds. For use in OLED devices both as emitters and as assistant dopants, such parameters are unacceptable as triplet harvesting should occur within submicrosecond and/or nanosecond time domain. There is thus a strong demand for approach to accelerate rISC and shorten $\tau_{DF}$, keeping high PLQY value. This can increase stability and efficiency of OLEDs.

This invention advantage is heavy-atom effect which accelerates spin-flip transitions. In addition the invention is based on heavy-metal-free, biocompatible, cheap, and ecofriendly materials, what results of incorporation of abundant and cheap heavy atoms into TADF emitters in OLEDs.

ISTOTA

In one aspect, the invention provides a compound which is a substituted triarylamine derivative connected to a substituted quinoxaline derivative bearing at least two cyano groups and contains at position $R^a$ and/or $R_1$-$R_8$ at least one atom with atomic weight above 25 Da, preferably chlorine, and/or bromine, and/or iodine connected to the substituted triarylamine derivative at positions $R_1$-$R_8$ and/or substituted quinoxaline derivative at position $R^a$ described by Chemical formula I and II.

Chemical formula I

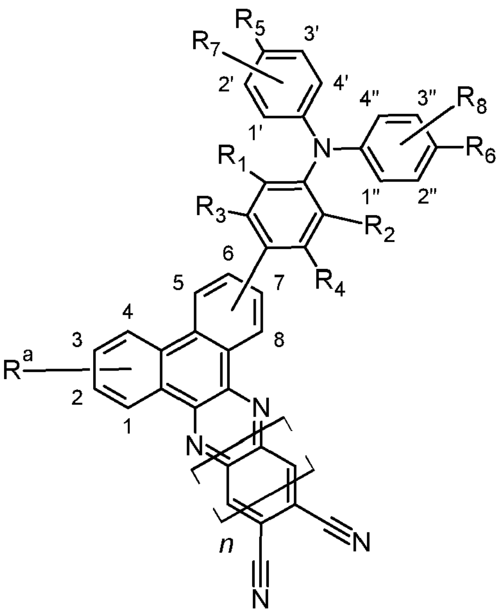

Chemical formula II

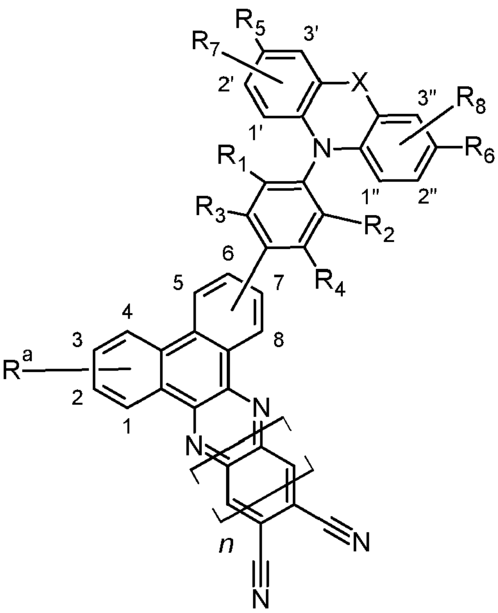

In the structures of Chemical Formula I:

$R^a$ can $R_1$ be H or any electron withdrawing group, namely any with a positive (>0) para and/or meta Hammett sigma constant as defined in the "Glossary of terms used in physical organic chemistry" (IUPAC Recommendations, PAC, 1994, 66, 1077, page 1171). For example, $R^a$ can be halogen or cyano, or nitro, or chalcogen(alkyl), or chalcogen(aryl), or chalcogen(heteroaryl), sulphonyl derivatives, or carboxyl derivative, or carbonyl derivative, or alkyl with electron withdrawing group(s), aryl with electron withdrawing group(s), hetaryl with electron withdrawing group(s);

$R^a$ can be one or several substituents mentioned above at positions 1-4 according to the numeration in Chemical Formula I.

Triarylamine fragment can be attached to the substituted quinoxaline derivative at positions 5-8 according to the numeration in Chemical Formula I.

$R_1$ and $R_3$ can each independently be H or halogen, or $CH_3$, or alkyl, or aryl, or heteroaryl, or chalcogen(alkyl), or chalcogen(aryl), or chalcogen(heteroaryl), or $N(alkyl)_2$, or $N(aryl)_2$, or N(alkyl, aryl), or $N(heteroaryl)_2$, or N(alkyl, heteroaryl), or N(aryl, heteroaryl), or $P(alkyl)_2$, or $P(aryl)_2$, or P(alkyl, aryl), or $P(heteroaryl)_2$, or P(alkyl, heteroaryl), or P(aryl, heteroaryl), or $P(O)(alkyl)_2$, or $P(O)(aryl)_2$, or P(O)(alkyl, aryl), or $P(O)(heteroaryl)_2$, or P(O)(alkyl, heteroaryl), or P(O)(aryl, heteroaryl), or $Si(alkyl)_3$, or $Si(aryl)_3$, or $Si(heteroaryl)_3$, or $Si(alkyl)_2$(aryl), or Si(alkyl)$(aryl)_2$, or $Si(alkyl)_2$ (hetaryl), or Si(alkyl)$(hetaryl)_2$, or $Si(aryl)_2$(hetaryl), or Si(aryl)$(hetaryl)_2$, or Si(alkyl, aryl, heteroaryl), or form an aliphatic (poly)cycle, or form an aromatic (poly)cycle, or form an aliphatic (poly)cycle with heteroatom(s), or form an aromatic (poly)cycle with heteroatom(s).

$R_2$ and $R_4$ can each independently be H or halogen, or $CH_3$, or alkyl, or aryl, or heteroaryl, or chalcogen(alkyl), or chalcogen(aryl), or chalcogen(heteroaryl), or $N(alkyl)_2$, or $N(aryl)_2$, or N(alkyl, aryl), or $N(heteroaryl)_2$, or N(alkyl, heteroaryl), or N(aryl, heteroaryl), or $P(alkyl)_2$, or $P(aryl)_2$, or P(alkyl, aryl), or $P(heteroaryl)_2$, or P(alkyl, heteroaryl), or P(aryl, heteroaryl), or $P(O)(alkyl)_2$, or $P(O)(aryl)_2$, or P(O)(alkyl, aryl), or $P(O)(heteroaryl)_2$, or P(O)(alkyl, heteroaryl),

or P(O)(aryl, heteroaryl), or Si(alkyl)$_3$, or Si(aryl)$_3$, or Si(heteroaryl)$_3$, or Si(alkyl)$_2$(aryl), or Si(alkyl)(aryl)$_2$, or Si(alkyl)$_2$ (hetaryl), or Si(alkyl)(hetaryl)$_2$, or Si(aryl)$_2$(hetaryl), or Si(aryl)(hetaryl)$_2$, or Si(alkyl, aryl, heteroaryl), or form an aliphatic (poly)cycle, or form an aromatic (poly)cycle, or form an aliphatic (poly)cycle with heteroatom(s), or form an aromatic (poly)cycle with heteroatom(s).

$R_5$, $R_6$, $R_7$, and $R_8$ can each independently be H or halogen, or $CH_3$, or alkyl, or aryl, or heteroaryl, or chalcogen(alkyl), or chalcogen(aryl), or chalcogen(heteroaryl), or N(alkyl)$_2$, or N(aryl)$_2$, or N(alkyl, aryl), or N(heteroaryl)$_2$, or N(alkyl, heteroaryl), or N(aryl, heteroaryl), or P(alkyl)$_2$, or P(aryl)$_2$, or P(alkyl, aryl), or P(heteroaryl)$_2$, or P(alkyl, heteroaryl), or P(aryl, heteroaryl), or P(O)(alkyl)$_2$, or P(O)(aryl)$_2$, or P(O)(alkyl, aryl), or P(O)(heteroaryl)$_2$, or P(O)(alkyl, heteroaryl), or P(O)(aryl, heteroaryl), or Si(alkyl)$_3$, or Si(aryl)$_3$, or Si(heteroaryl)$_3$, or Si(alkyl)$_2$(aryl), or Si(alkyl)(aryl)$_2$, or Si(alkyl)$_2$ (hetaryl), or Si(alkyl)(hetaryl)$_2$, or Si(aryl)$_2$(hetaryl), or Si(aryl)(hetaryl)$_2$, or Si(alkyl, aryl, heteroaryl);

$R_1$, $R_2$, $R_3$, $R_4$, $R_5$, $R_6$, $R_7$, and $R_8$ in various combinations can form an aliphatic (poly)cycle(s), or form an aromatic (poly)cycle(s), or form an aliphatic (poly)cycle(s) with heteroatom(s), or form an aromatic (poly)cycle(s) with heteroatom(s).

$R^a$, $R_1$, $R_2$, $R_3$, $R_4$, $R_5$, $R_6$, $R_7$, and $R_8$ cannot be H simultaneously.

At least one of substituents $R^a$, $R_1$, $R_2$, $R_3$, $R_4$, $R_5$, $R_6$, $R_7$, and/or $R_8$, should contain at least one atom with atomic weight above 25 Da.

*n* can be 0, 1 or 2

In the structures of Chemical Formula II:

X can be either C(CH$_3$)$_2$, or C(alkyl)$_2$, or C(aryl)$_2$, or C(alkyl, aryl), or C(heteroaryl)$_2$, or C(alkyl, heteroaryl), or C(aryl, heteroaryl), or a single C-C bond, or C=O, or C=S, or chalcogen, or Si(alkyl)$_2$, or Si(aryl)$_2$, or Si(alkyl, aryl), or Si(heteroaryl)$_2$, or Si(alkyl, heteroaryl), or Si(aryl, heteroaryl), or N(alkyl), or N(aryl), or N(hetaryl), or P(alkyl), or P(aryl), or P(hetaryl), or P(O)(alkyl), or P(O)(aryl), or P(O)(heteroaryl), or P=S, or $SO_2$, or Ge(alkyl)$_2$, or Ge(aryl)$_2$, or Ge(alkyl, aryl), or Ge(heteroaryl)$_2$, or Ge(alkyl, heteroaryl), or Ge(aryl, heteroaryl), or form an aliphatic (poly)cycle, or form an aromatic (poly)cycle, or form an aliphatic (poly)cycle with heteroatom(s), or form an aromatic (poly)cycle with heteroatom(s).

$R^a$ can $R_1$ be H or any electron withdrawing group, namely any with a positive (>0) para and/or meta Hammett sigma constant as defined in the "Glossary of terms used in physical organic chemistry" (IUPAC Recommendations, PAC, 1994, 66, 1077, page 1171). For example, $R^a$ can be halogen or cyano, or nitro, or chalcogen(alkyl), or chalcogen(aryl), or chalcogen(heteroaryl), sulphonyl derivatives, or carboxyl derivative, or carbonyl derivative, or alkyl with electron withdrawing group(s), aryl with electron withdrawing group(s), hetaryl with electron withdrawing group(s);

$R^a$ can be one or several substituents mentioned above at positions 1-4 according to the numeration in Chemical Formula II.

Triarylamine fragment can be attached to the substituted quinoxaline derivative at positions 5-8 according to the numeration in Chemical Formula II.

$R_1$ and $R_3$ can each independently be H or halogen, or $CH_3$, or alkyl, or aryl, or heteroaryl, or chalcogen(alkyl), or chalcogen(aryl), or chalcogen(heteroaryl), or N(alkyl)$_2$, or N(aryl)$_2$, or N(alkyl, aryl), or N(heteroaryl)$_2$, or N(alkyl, heteroaryl), or N(aryl, heteroaryl), or P(alkyl)$_2$, or P(aryl)$_2$, or P(alkyl, aryl), or P(heteroaryl)$_2$, or P(alkyl, heteroaryl), or P(aryl, heteroaryl), or P(O)(alkyl)$_2$, or P(O)(aryl)$_2$, or P(O)(alkyl, aryl), or P(O)(heteroaryl)$_2$, or P(O)(alkyl, heteroaryl), or P(O)(aryl, heteroaryl), or Si(alkyl)$_3$, or Si(aryl)$_3$, or Si(heteroaryl)$_3$, or Si(alkyl)$_2$(aryl), or Si(alkyl)(aryl)$_2$, or Si(alkyl)$_2$ (hetaryl), or Si(alkyl)(hetaryl)$_2$, or Si(aryl)$_2$(hetaryl), or Si(aryl)(hetaryl)$_2$, or Si(alkyl, aryl, heteroaryl), or form an aliphatic (poly)cycle, or form an aromatic (poly)cycle, or form an aliphatic (poly)cycle with heteroatom(s), or form an aromatic (poly)cycle with heteroatom(s).

$R_2$ and $R_4$ can each independently be H or halogen, or $CH_3$, or alkyl, or aryl, or heteroaryl, or chalcogen(alkyl), or chalcogen(aryl), or chalcogen(heteroaryl), or N(alkyl)$_2$, or N(aryl)$_2$, or N(alkyl, aryl), or N(heteroaryl)$_2$, or N(alkyl, heteroaryl), or N(aryl, heteroaryl), or P(alkyl)$_2$, or P(aryl)$_2$, or P(alkyl, aryl), or P(heteroaryl)$_2$, or P(alkyl, heteroaryl), or P(aryl, heteroaryl), or P(O)(alkyl)$_2$, or P(O)(aryl)$_2$, or P(O)(alkyl, aryl), or P(O)(heteroaryl)$_2$, or P(O)(alkyl, heteroaryl),

or P(O)(aryl, heteroaryl), or Si(alkyl)$_3$, or Si(aryl)$_3$, or Si(heteroaryl)$_3$, or Si(alkyl)$_2$(aryl), or Si(alkyl)(aryl)$_2$, or Si(alkyl)$_2$ (hetaryl), or Si(alkyl)(hetaryl)$_2$, or Si(aryl)$_2$(hetaryl), or Si(aryl)(hetaryl)$_2$, or Si(alkyl, aryl, heteroaryl), or form an aliphatic (poly)cycle, or form an aromatic (poly)cycle, or form an aliphatic (poly)cycle with heteroatom(s), or form an aromatic (poly)cycle with heteroatom(s).

$R_5$, $R_6$, $R_7$, and $R_8$ can each independently be H or halogen, or $CH_3$, or alkyl, or aryl, or heteroaryl, or chalcogen(alkyl), or chalcogen(aryl), or chalcogen(heteroaryl), or N(alkyl)$_2$, or N(aryl)$_2$, or N(alkyl, aryl), or N(heteroaryl)$_2$, or N(alkyl, heteroaryl), or N(aryl, heteroaryl), or P(alkyl)$_2$, or P(aryl)$_2$, or P(alkyl, aryl), or P(heteroaryl)$_2$, or P(alkyl, heteroaryl), or P(aryl, heteroaryl), or P(O)(alkyl)$_2$, or P(O)(aryl)$_2$, or P(O)(alkyl, aryl), or P(O)(heteroaryl)$_2$, or P(O)(alkyl, heteroaryl), or P(O)(aryl, heteroaryl), or Si(alkyl)$_3$, or Si(aryl)$_3$, or Si(heteroaryl)$_3$, or Si(alkyl)$_2$(aryl), or Si(alkyl)(aryl)$_2$, or Si(alkyl)$_2$ (hetaryl), or Si(alkyl)(hetaryl)$_2$, or Si(aryl)$_2$(hetaryl), or Si(aryl)(hetaryl)$_2$, or Si(alkyl, aryl, heteroaryl);

$R_1$, $R_2$, $R_3$, $R_4$, $R_5$, $R_6$, $R_7$, and $R_8$ in various combinations can form an aliphatic (poly)cycle(s), or form an aromatic (poly)cycle(s), or form an aliphatic (poly)cycle(s) with heteroatom(s), or form an aromatic (poly)cycle(s) with heteroatom(s).

$R^a$, $R_1$, $R_2$, $R_3$, $R_4$, $R_5$, $R_6$, $R_7$, and $R_8$ cannot be H simultaneously.

At least one of substituents $R^a$, $R_1$, $R_2$, $R_3$, $R_4$, $R_5$, $R_6$, $R_7$, and/or $R_8$, should contain at least one atom with atomic weight above 25 Da.

*n* can be 0, 1 or 2

The compounds given by Chemical Formula I and II emit light under photoexcitation;

The emitted light is characterized by peak maximum wavelength ($PL_{max}$) from 550 nm and higher, photoluminescence quantum yield (PLQY), regions of prompt fluorescence (PF) and delayed fluorescence (DF) in emission decay profile with decay lifetimes $\tau_{PF}$ and $\tau_{DF}$, rate constants of radiative deactivation ($k_r$), intersystem crossing ($k_{ISC}$), and reverse intersystem crossing ($k_{rISC}$), when measured in a mixture with a host material, for example in the 0.1 - 100% (w/w) mixture with a 4,4'-bis(N-carbazolyl)-1,1'-biphenyl (CBP).

The emitted light is characterized by peak maximum wavelength ($PL_{max}$) from 550 nm and higher, PLQY, regions of PF and DF in emission decay profile with decay lifetimes $\tau_{PF}$ and $\tau_{DF}$, rate constants $k_r$, $k_{ISC}$, and $k_{rISC}$, when measured for example in the 10% (w/w) film in 4,4'-bis(N-carbazolyl)-1,1'-biphenyl (CBP) host.

The $k_{ISC}$ and/or $k_{rISC}$ values are increased as compared to those of analogues without heavy atoms. The measure of such increase is given by $k_{ISC}/k_{ISC}$(H) and $k_{rISC}/k_{rISC}$(H) ratios, where $k_{ISC}$(H) and $k_{rISC}$(H) are respective rate constants for analogue emitters without heavy atoms.

According to the present invention the luminescence properties of compounds under Chemical formula I and II convert the triplet excitons to singlet ones faster than the compounds Px-CNBQx, Ds-CNBQx, Ac-CNBQx, Cz-CNBQx, Px-CNBPz, Da-CNBPz, Ac-CNBPz described in US2019330162, outreaching them in their light emitting and triplet harvesting ability, namely by higher $k_{rISC}$ and shorter $\tau_{DF}$, as described in examples.

In another aspect, the present invention provides mixtures containing various amounts of the compound given by Chemical Formula I and/or II and various amounts of known NIR emitting dopant, for example pyrrolopyrrole fluorophore, like BPPC or other; mixtures emit NIR light during at least 0.5 microseconds after excitation wherein the relative mass ratio of the compound given by the Chemical Formula I and/or II and a NIR emitting dopant in a mixture can vary from 1000/1 to 1/10. The emitted light is characterized by $PL_{max}$ and full width at half maximum (FWHM) of the NIR emitting dopant: in the case of BPPC $PL_{max}$ at 790 - 805 nm, the full width at half maximum equal to or less than 50 nm. The emitted light is also characterized by PLQY, regions of PF and DF in emission decay profile with decay lifetimes $\tau_{PF}$ and $\tau_{DF}$, all these parameters can vary depending on the mixture contamination and the type of compound given by Chemical Formula I and II.

The invention is described in detail in the examples and figures where:

Figure 1 presents the graph of comparison of PL spectra of Da-CNBPz-H and e1 (describe in more detailed in Table 1), an example of Chemical Formula I.

Figure 2 presents the graph of comparison of PL spectra of Da-CNBPz and e2 (Table 1), an example of Chemical Formula I.

Figure 3 presents the graph of comparison of PL spectra of Da-CNBPz and e3 (Table 1), an example of Chemical Formula I.

Figure 4 presents the graph of comparison of PL spectra of Da-CNBPz and e4 (Table 1), an example of Chemical Formula I.

Figure 5 presents the graph of comparison of PL spectra of Da-CNBPz and e5 (Table 1), an example of Chemical Formula I.

Figure 6 presents the graph of comparison of PL spectra of Ac-CNBPz and e6 (Table 1), an example of Chemical Formula II.

Figure 7 presents the graph of comparison of PL spectra of Ac-CNBPz and e7 (Table 1), an example of Chemical Formula II.

Figure 8 presents the graph of comparison of PL spectra of Ac-CNBPz and e8 (Table 1), an example of Chemical Formula II.

Figure 9 presents the graph of comparison of PL spectra of Ac-CNBPz and e9 (Table 1), an example of Chemical Formula II.

Figure 10 presents the graph of comparison of PL spectra of Da-CNBPz-H and f1 (Table 1), an example of Chemical Formula I.

Figure 11 presents the graph of comparison of PL spectra of Ac-CNBPz and f2 (Table 1), an example of Chemical Formula II.

Figure 12 presents the graph of comparison of PL spectra of Da-CNBPz-H and g1 (Table 1), an example of Chemical Formula I.

Figure 13 presents the graph of comparison of PL spectra of Da-CNBPz-H and g2 (Table 1), an example of Chemical Formula I.

Figure 14 presents the graph of comparison of PL spectra of Da-CNBPz-H and g3 (Table 1), an example of Chemical Formula I.

Figure 15 presents the graph of comparison of PL spectra of Da-CNBPz-H and g4 (Table 1), an example of Chemical Formula I.

Figure 16 presents the graph of comparison of PL spectra of Da-CNBPz-H and g5 (Table 1), an example of Chemical Formula I.

Figure 17 presents the graph of comparison of PL spectra of Da-CNBPz-H and g6 (Table 1), an example of Chemical Formula I.

Figure 18 presents the graph of comparison of PL spectra of Da-CNBPz-H and g7 (Table 1), an example of Chemical Formula I.

Figure 19 presents the graph of comparison of PL spectra of Da-CNBPz-H and g8 (Table 1), an example of Chemical Formula I.

Figure 20 presents the graph of comparison of PL spectra of Da-CNBPz and g9 (Table 1), an example of Chemical Formula I.

Figure 21 presents the graph of comparison of PL spectra of Da-CNBPz and g10 (Table 1), an example of Chemical Formula I.

Figure 22 presents the graph of comparison of PL spectra of Ac-CNBPz and g11 (Table 1), an example of Chemical Formula II.

Figure 23 presents the graph of comparison of PL spectra of Ac-CNBPz and g12 (Table 1), an example of Chemical

Formula II.

Figure 24 presents the graph of comparison of PL spectra of Ac-CNBPz and g13 (Table 1), an example of Chemical Formula II.

Figure 25 presents the graph of comparison of PL spectra of Ac-CNBPz and g14 (Table 1), an example of Chemical Formula II.

Figure 26 presents the graph of comparison of PL spectra of Ac-CNBPz and g15 (Table 1), an example of Chemical Formula II.

Figure 27 presents the graph of comparison of PL spectra of Ac-CNBPz and g16 (Table 1), an example of Chemical Formula II.

Figure 28 presents the graph of comparison of PL spectra of Ac-CNBPz and g17 (Table 1), an example of Chemical Formula II.

Figure 29 presents the graph of comparison of PL decay of Da-CNBPz-H and e1 (Table 1), an example of Chemical Formula I.

Figure 30 presents the graph of comparison of PL decay of Da-CNBPz and e2 (Table 1), an example of Chemical Formula I.

Figure 31 presents the graph of comparison of PL decay of Da-CNBPz and e3 (Table 1), an example of Chemical Formula I.

Figure 32 presents the graph of comparison of PL decay of Da-CNBPz and e4 (Table 1), an example of Chemical Formula I.

Figure 33 presents the graph of comparison of PL decay of Da-CNBPz and e5 (Table 1), an example of Chemical Formula I.

Figure 34 presents the graph of comparison of PL decay of Ac-CNBPz and e6 (Table 1), an example of Chemical Formula II.

Figure 35 presents the graph of comparison of PL decay of Ac-CNBPz and e7 (Table 1), an example of Chemical Formula II.

Figure 36 presents the graph of comparison of PL decay of Ac-CNBPz and e8 (Table 1), an example of Chemical Formula II.

Figure 37 presents the graph of comparison of PL decay of Ac-CNBPz and e9 (Table 1), an example of Chemical Formula II.

Figure 38 presents the graph of comparison of PL decay of Da-CNBPz-H and f1 (Table 1), an example of Chemical Formula I.

Figure 39 presents the graph of comparison of PL decay of Ac-CNBPz and f2 (Table 1), an example of Chemical Formula II.

Figure 40 presents the graph of comparison of PL decay of Da-CNBPz-H and g1 (Table 1), an example of Chemical Formula I.

Figure 41 presents the graph of comparison of PL decay of Da-CNBPz-H and g2 (Table 1), an example of Chemical Formula I.

Figure 42 presents the graph of comparison of PL decay of Da-CNBPz-H and g3 (Table 1), an example of Chemical Formula I.

Figure 43 presents the graph of comparison of PL decay of Da-CNBPz-H and g4 (Table 1), an example of Chemical Formula I.

Figure 44 presents the graph of comparison of PL decay of Da-CNBPz-H and g5 (Table 1), an example of Chemical Formula I.

Figure 45 presents the graph of comparison of PL decay of Da-CNBPz-H and g6 (Table 1), an example of Chemical Formula I.

Figure 46 presents the graph of comparison of PL decay of Da-CNBPz-H and g7 (Table 1), an example of Chemical Formula I.

Figure 47 presents the graph of comparison of PL decay of Da-CNBPz-H and g8 (Table 1), an example of Chemical Formula I.

Figure 48 presents the graph of comparison of PL decay of Da-CNBPz and g9 (Table 1), an example of Chemical Formula I.

Figure 49 presents the graph of comparison of PL decay of Da-CNBPz and g10 (Table 1), an example of Chemical Formula I.

Figure 50 presents the graph of comparison of PL decay of Ac-CNBPz and g11 (Table 1), an example of Chemical Formula II.

Figure 51 presents the graph of comparison of PL decay of Ac-CNBPz and g12 (Table 1), an example of Chemical Formula II.

Figure 52 presents the graph of comparison of PL decay of Ac-CNBPz and g13 (Table 1), an example of Chemical Formula II.

Figure 53 presents the graph of comparison of PL decay of Ac-CNBPz and g14 (Table 1), an example of Chemical Formula II.

Figure 54 presents the graph of comparison of PL decay of Ac-CNBPz and g15 (Table 1), an example of Chemical Formula II.

Figure 55 presents the graph of comparison of PL decay of Ac-CNBPz and g16 (Table 1), an example of Chemical Formula II.

Figure 56 presents the graph of comparison of PL decay of Ac-CNBPz and g17 (Table 1), an example of Chemical Formula II.

Figure 57 presents the PL spectrum of a mixture of BPPC and e6 (Table 1), an example of Chemical Formula II.

Figure 58. Comparison of the PL decay of a mixture of BPPC with either TPA-DCPP or e1 (Table 1), an example of Chemical Formula I.

Figure 59. Comparison of the PL decay of a mixture of BPPC with either TPA-DCPP or e2 (Table 1), an example of Chemical Formula I.

Figure 60. Comparison of the PL decay of a mixture of BPPC with either TPA-DCPP or e3 (Table 1), an example of Chemical Formula I.

Figure 61. Comparison of the PL decay of a mixture of BPPC with either TPA-DCPP or e6 (Table 1), an example of Chemical Formula II.

Figure 62. Comparison of the PL decay of a mixture of BPPC with either TPA-DCPP or f2 (Table 1), an example of Chemical Formula II.

Figure 63. Comparison of the PL decay of a mixture of BPPC with either TPA-DCPP or g2 (Table 1), an example of Chemical Formula I.

Figure 64. Comparison of the PL decay of a mixture of BPPC with either TPA-DCPP or g5 (Table 1), an example of Chemical Formula I.

Figure 65. Comparison of the PL decay of a mixture of BPPC with either TPA-DCPP or g7 (Table 1), an example of Chemical Formula I.

Figure 66. Comparison of the PL decay of a mixture of BPPC with either TPA-DCPP or g13 (Table 1), an example of Chemical Formula II.

Table 1 presents the examples of substituents providing structures of e1-e5, f1, g1-g10 as the examples of Chemical Formula I, and f2, e6-e9, g11-g17 as the examples of Chemical Formula II, and Da-CNBPz, Da-CNBPz-H, Ac-CNBPz.

Table 2 presents the summary of photoluminescent and photophysical properties of light emitting elements containing e1-e5, f1, g1-g10 (Table 1) as the examples of Chemical Formula I, and f2, e6-e9, g11-g17 (Table 1) as the examples of Chemical Formula II, and Da-CNBPz, Da-CNBPz-H, Ac-CNBPz.

Table 3 presents the summary of photoluminescent properties of light emitting elements containing TPA-DCPP, and e1-e3, g2, g5, g7 (Table 1) as the examples of Chemical Formula I, and f2, e6-e8, g11, g13 (Table 1) as the examples of Chemical Formula II in the presence of BPPC dopant.

The method for preparation of the compounds are shown in scheme 1 and 2 - eneral method for preparation of compounds given by Chemical Formula I and II

Example 1

General method for preparation of compounds given by Chemical Formula I and II

The compounds given by Chemical Formula I and Chemical Formula II can be prepared according to the schemes and general conditions of reactions R1-R6 described below (Scheme 1 and Scheme 2). Heavy-atom substituents can be introduced in reaction R1 and/or R3, and/or R6. The identity and purity of the compounds at each step can be verified through NMR spectroscopy, mass spectroscopy (such a MALDITOF), thin-layer chromatography and other suitable methods.

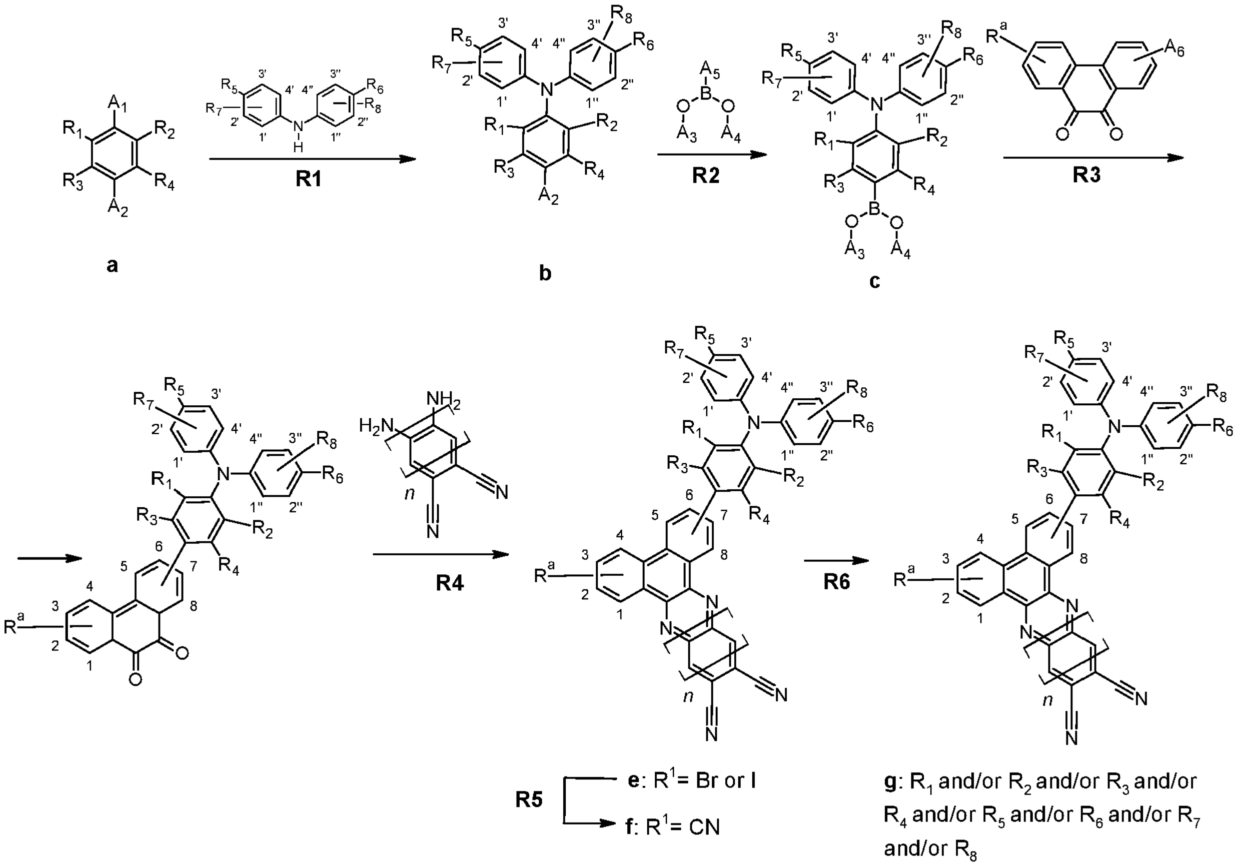


Scheme 1. General method for preparation of compounds given by Chemical Formula I.

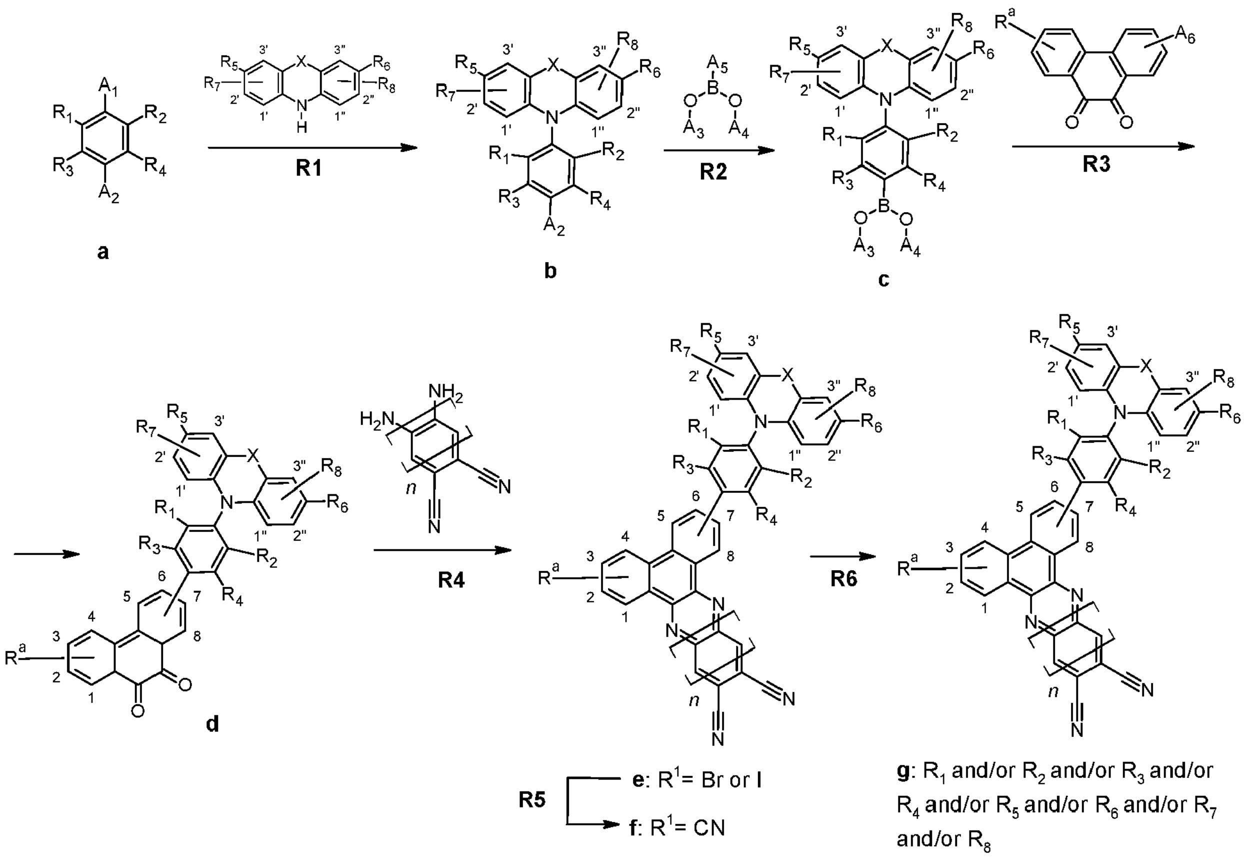


Scheme 2. General method for preparation of compounds given by Chemical Formula II.

In reaction R1

$A_1$ = F, $A_2$ = Br or I

$R_1$ and $R_3$ can each independently be H or halogen, or $CH_3$, or alkyl, or aryl, or heteroaryl, or chalcogen(alkyl), or chalcogen(aryl), or chalcogen(heteroaryl), or N(alkyl)$_2$, or N(aryl)$_2$, or N(alkyl, aryl), or N(heteroaryl)$_2$, or N(alkyl, heteroaryl), or N(aryl, heteroaryl), or P(alkyl)$_2$, or P(aryl)$_2$, or P(alkyl, aryl), or P(heteroaryl)$_2$, or P(alkyl, heteroaryl), or P(aryl, heteroaryl), or P(O)(alkyl)$_2$, or P(O)(aryl)$_2$, or P(O)(alkyl, aryl), or P(O)(heteroaryl)$_2$, or P(O)(alkyl, heteroaryl), or P(O)(aryl, heteroaryl), or Si(alkyl)$_3$, or Si(aryl)$_3$, or Si(heteroaryl)$_3$, or Si(alkyl)$_2$(aryl), or Si(alkyl)(aryl)$_2$, or Si(alkyl)$_2$ (hetaryl), or Si(alkyl)(hetaryl)$_2$, or Si(aryl)$_2$(hetaryl), or Si(aryl)(hetaryl)$_2$, or Si(alkyl, aryl, heteroaryl), or form an aliphatic (poly)cycle, or form an aromatic (poly)cycle, or form an aliphatic (poly)cycle with heteroatom(s), or form an aromatic (poly)cycle with heteroatom(s).

$R_2$ and $R_4$ can each independently be H or halogen, or $CH_3$, or alkyl, or aryl, or heteroaryl, or chalcogen(alkyl), or chalcogen(aryl), or chalcogen(heteroaryl), or N(alkyl)$_2$, or N(aryl)$_2$, or N(alkyl, aryl), or N(heteroaryl)$_2$, or N(alkyl, heteroaryl), or N(aryl, heteroaryl), or P(alkyl)$_2$, or P(aryl)$_2$, or P(alkyl, aryl), or P(heteroaryl)$_2$, or P(alkyl, heteroaryl), or P(aryl, heteroaryl), or P(O)(alkyl)$_2$, or P(O)(aryl)$_2$, or P(O)(alkyl, aryl), or P(O)(heteroaryl)$_2$, or P(O)(alkyl, heteroaryl), or P(O)(aryl, heteroaryl), or Si(alkyl)$_3$, or Si(aryl)$_3$, or Si(heteroaryl)$_3$, or Si(alkyl)$_2$(aryl), or Si(alkyl)(aryl)$_2$, or Si(alkyl)$_2$ (hetaryl), or Si(alkyl)(hetaryl)$_2$, or Si(aryl)$_2$(hetaryl), or Si(aryl)(hetaryl)$_2$, or Si(alkyl, aryl, heteroaryl), or form an aliphatic (poly)cycle, or form an aromatic (poly)cycle, or form an aliphatic (poly)cycle with heteroatom(s), or form an aromatic (poly)cycle with heteroatom(s).

X can be either C($CH_3$)$_2$, or C(alkyl)$_2$, or C(aryl)$_2$, or C(alkyl, aryl), or C(heteroaryl)$_2$, or C(alkyl, heteroaryl), or C(aryl, heteroaryl), or a single C-C bond, or C=O, or C=S, or chalcogen, or Si(alkyl)$_2$, or Si(aryl)$_2$, or Si(alkyl, aryl), or Si(heteroaryl)$_2$, or Si(alkyl, heteroaryl), or Si(aryl, heteroaryl), or N(alkyl), or N(aryl), or N(hetaryl), or P(alkyl), or P(aryl), or P(hetaryl), or P(O)(alkyl), or P(O)(aryl), or P(O)(heteroaryl), or P=S, or $SO_2$, or Ge(alkyl)$_2$, or Ge(aryl)$_2$, or

Ge(alkyl, aryl), or Ge(heteroaryl)$_2$, or Ge(alkyl, heteroaryl), or Ge(aryl, heteroaryl), or form an aliphatic (poly)cycle, or form an aromatic (poly)cycle, or form an aliphatic (poly)cycle with heteroatom(s), or form an aromatic (poly)cycle with heteroatom(s).

$R_5$, $R_6$, $R_7$, and $R_8$ can each independently be H or F, or Cl, or $CH_3$, or alkyl, or aryl, or heteroaryl, or chalcogen(alkyl), or chalcogen(aryl), or chalcogen(heteroaryl), or N(alkyl)$_2$, or N(aryl)$_2$, or N(alkyl, aryl), or N(heteroaryl)$_2$, or N(alkyl, heteroaryl), or N(aryl, heteroaryl), or P(alkyl)$_2$, or P(aryl)$_2$, or P(alkyl, aryl), or P(heteroaryl)$_2$, or P(alkyl, heteroaryl), or P(aryl, heteroaryl), or P(O)(alkyl)$_2$, or P(O)(aryl)$_2$, or P(O)(alkyl, aryl), or P(O)(heteroaryl)$_2$, or P(O)(alkyl, heteroaryl), or P(O)(aryl, heteroaryl), or Si(alkyl)$_3$, or Si(aryl)$_3$, or Si(heteroaryl)$_3$, or Si(alkyl)$_2$(aryl), or Si(alkyl)(aryl)$_2$, or Si(alkyl)$_2$ (hetaryl), or Si(alkyl)(hetaryl)$_2$, or Si(aryl)$_2$(hetaryl), or Si(aryl)(hetaryl)$_2$, or Si(alkyl, aryl, heteroaryl).

$R_1$, $R_2$, $R_3$, $R_4$, $R_5$, $R_6$, $R_7$, and $R_8$ in various combinations can form an aliphatic (poly)cycle(s), or form an aromatic (poly)cycle(s), or form an aliphatic (poly)cycle(s) with heteroatom(s), or form an aromatic (poly)cycle(s) with heteroatom(s).

In reaction R2
$A_3$, $A_4$ can be separately H or any alkyl group or together form a aliphatic cycle, for example, pinacolic ester derived from diol.
$A_5$ can be a $B(OA_3)OA_4$ group with $A_3$ and $A_4$ as described above.
In reaction R3

$A_6$ is Br or I or O-triflate

$R^a$ is H or any electron withdrawing group, namely any substituent with a positive (>0) para and/or meta Hammett sigma constant as defined in the "Glossary of terms used in physical organic chemistry" (IUPAC Recommendations, PAC, 1994, 66, 1077, page 1171). For example, $R^a$ can be halogen or cyano, or nitro, or chalcogen(alkyl), or chalcogen(aryl), or chalcogen(heteroaryl), sulphonyl derivatives, or carboxyl derivative, or carbonyl derivative or alkyl with electron withdrawing group(s), aryl with electron withdrawing group(s), hetaryl with electron withdrawing group(s).

In reaction R4
*n* is 0 or 1, or 2
In reaction R5: substrate e: $R^a$ = F, or Cl, or Br, or I, preferably Br or I; product f: $R^a$ = CN.
In reaction R6

If in a substrate e or f, $R_5$ and/or $R_6$ = H, and N-chlorosuccinimide or *N*-bromosuccinimide or *N*-iodosuccinimide is used as reagent, then, in a product g, $R_5$ and/or $R_6$ = Cl, or Br, or I, respectively.

If in substrate e or f, $R_5$ and/or $R_6$ = $CH_3$, or alkyl, or aryl, or heteroaryl and *N*-chlorosuccinimide or *N*-bromosuccinimide or *N*-iodosuccinimide is used as reagent, then, in product g, $R_7$ and/or $R_8$ = Cl, or Br, or I, respectively, at position(s) 1' and 1".

If in substrate e or f: $R_1$ = H, and $R_5$ and/or $R_6$ = $CH_3$, or alkyl, or aryl, or heteroaryl, and *N*-chlorosuccinimide or *N*-bromosuccinimide or N-iodosuccinimide is used as reagent in amount of at least 3 equivalent, then, in product g, $R_1$ = Cl, or Br, or I, respectively.

If in substrate e or f: $R_5$ and/or $R_6$ = $OCH_3$, O-alkyl, or O-aryl, or O-heteroaryl, or S-alkyl, or S-aryl, or S-heteroaryl, or N-(alkyl)$_2$, or N-(aryl)$_2$, or N-(alkyl, aryl), or N-(heteroaryl)$_2$, or N-(alkyl, heteroaryl), or N-(aryl, heteroaryl) and *N*-chlorosuccinimide or *N*-bromosuccinimide or *N*-iodosuccinimide is used as reagent, then, in product g, $R_7$ and/or $R_8$ = Cl, or Br, or I, respectively, at position(s) 2' and 2".

Example 2. Specific examples of selected canonic structures of heavy atom derivatives of triphenylamine - quinoxaline compounds disclosed here are shown in the Table 1 below. The invention is not limited to compounds e1-9, f1-2, and g1-17 disclosed in detailed in the example 2..

Table 1. Examples of compounds according to the Chemical formula I and II bearing heavy atoms.

| Compound | $R^a$ | Position of triarylamine fragment | $R_1$ | $R_2$ | $R_3$ | $R_4$ | $R_5$ | $R_6$ | $R_7$ | $R_8$ |
|---|---|---|---|---|---|---|---|---|---|---|
| Da-CNBPz | H | 6 | H | H | H | H | $CH_3$ | $CH_3$ | H | H |
| Da-CNBPz-H | H | 6 | H | H | H | H | H | H | H | H |
| Examples of Compounds given by Chemical formula I ($n = 1$) | | | | | | | | | | |
| g1 | H | 6 | H | H | H | H | Br | Br | H | H |
| g2 | H | 6 | H | H | H | H | I | I | H | H |
| g3 | H | 6 | H | H | H | H | Br | H | H | H |
| g4 | H | 6 | H | H | H | H | I | H | H | H |
| e1 | 3-Br | 6 | H | H | H | H | H | H | H | H |
| g5 | 3-Br | 6 | H | H | H | H | Br | Br | H | H |
| g6 | 3-Br | 6 | H | H | H | H | I | I | H | H |
| f1 | 3-CN | 6 | H | H | H | H | H | H | H | H |
| g7 | 3-CN | 6 | H | H | H | H | Br | Br | 1'-Br | 1''-Br |
| g8 | H | 6 | H | H | H | H | Br | Br | 1'-Br | H |
| e2 | H | 6 | Cl | H | H | H | $CH_3$ | $CH_3$ | H | H |
| e3 | H | 6 | Cl | Cl | H | H | $CH_3$ | $CH_3$ | H | H |
| g9 | H | 6 | Cl | H | H | H | $CH_3$ | $CH_3$ | 1'-Br | H |
| g10 | H | 6 | Cl | Cl | H | H | $CH_3$ | $CH_3$ | 1'-Br | H |
| e4 | 2-Br | 7 | Cl | Cl | H | H | $OCH_3$ | $OCH_3$ | H | H |
| e5 | 3-Br | 6 | Cl | Cl | H | H | OC | OC | H | H |

| | | | | | | | | | | |
|---|---|---|---|---|---|---|---|---|---|---|
| | | | | | | | $H_3$ | $H_3$ | | |

| Compound | X | $R^a$ | Position of triarylamine fragment | $R_1$ | $R_2$ | $R_3$ | $R_4$ | $R_5$ | $R_6$ | $R_7$ | $R_8$ |
|---|---|---|---|---|---|---|---|---|---|---|---|
| Ac-CNBPz | $C(CH_3)$ | H | 6 | H | H | H | H | H | H | H | H |
| Examples of Compounds given by Chemical formula II ($n$ = 1) | | | | | | | | | | | |
| g11 | $C(CH_3)$ | H | 6 | H | H | H | H | Br | Br | H | H |
| g12 | $C(CH_3)$ | H | 6 | H | H | H | H | I | I | H | H |
| e6 | $C(CH_3)$ | 3-Br | 6 | H | H | H | H | H | H | H | H |
| g13 | $C(CH_3)$ | 3-Br | 6 | H | H | H | H | Br | Br | H | H |
| g14 | $C(CH_3)$ | 3-Br | 6 | H | H | H | H | I | I | H | H |
| f2 | $C(CH_3)$ | 3-CN | 6 | H | H | H | H | H | H | H | H |
| g15 | $C(CH_3)$ | 3-CN | 6 | H | H | H | H | Br | Br | H | H |
| e7 | $C(CH_3)$ | H | 6 | Cl | Cl | H | H | H | H | H | H |
| e8 | $C(CH_3)$ | 3-Br | 6 | Cl | Cl | H | H | H | H | H | H |
| g16 | $C(CH_3)$ | H | 6 | Cl | Cl | H | H | Br | Br | H | H |
| g17 | $C(CH_3)$ | 3-Br | 6 | Cl | Cl | H | H | Br | Br | H | H |
| e9 | $C(CH_3)$ | 2-Br | 7 | H | H | H | H | H | H | H | H |

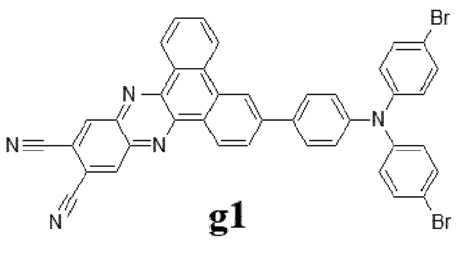

g1

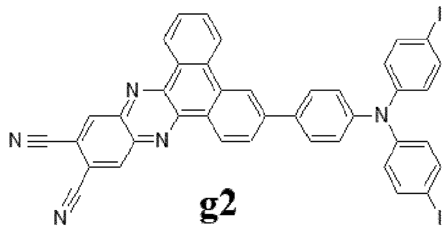

g2

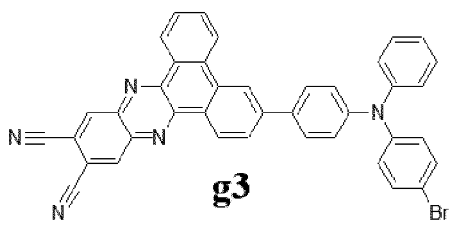

g3

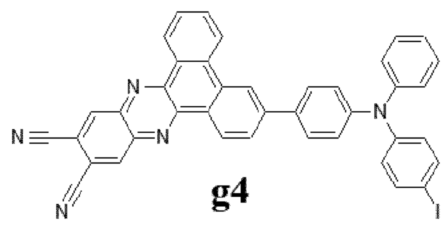

g4

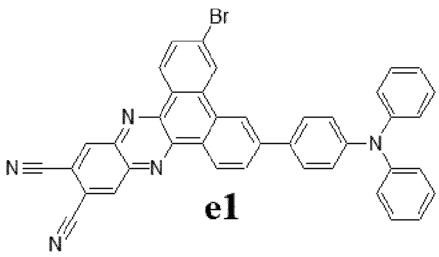
e1

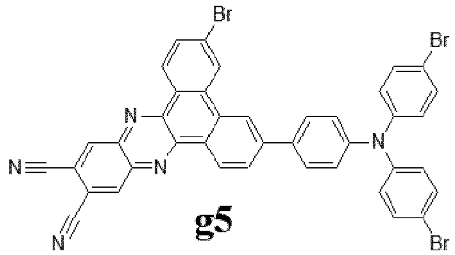
g5

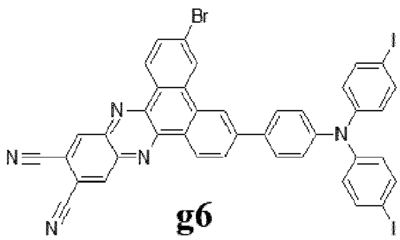
g6

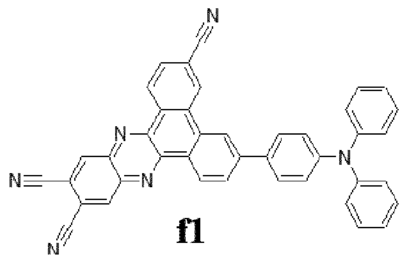
f1

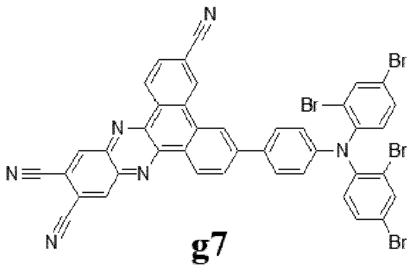
g7

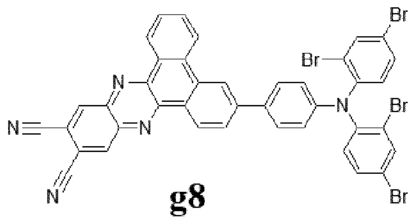
g8

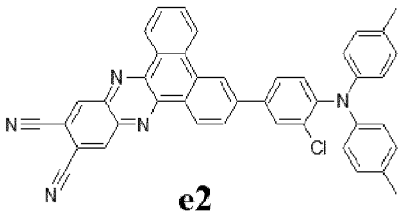
e2

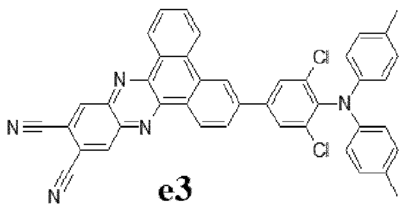
e3

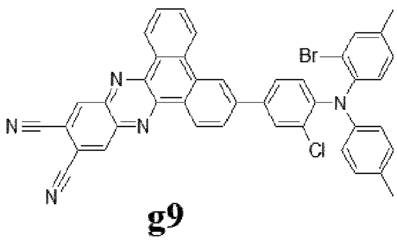
g9

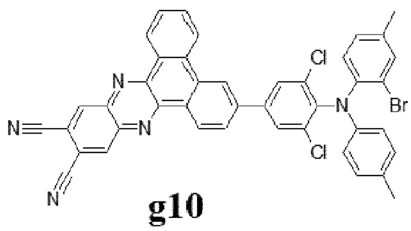
g10

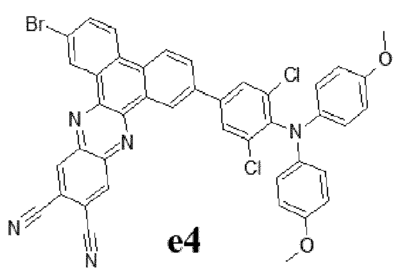
e4

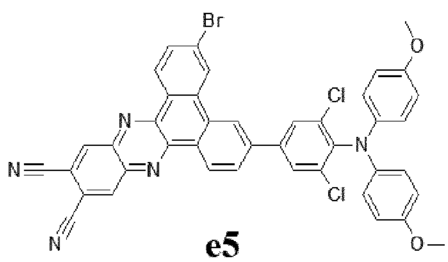
e5

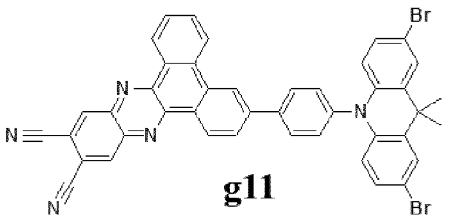

g11

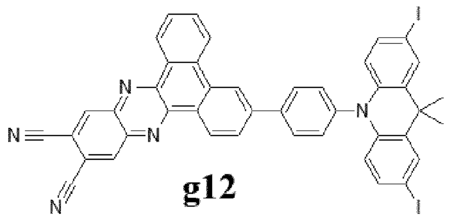

g12

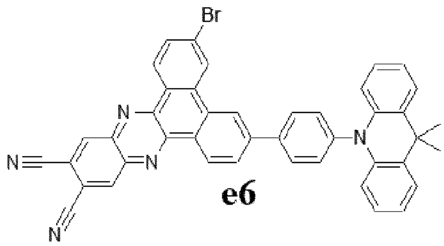

e6

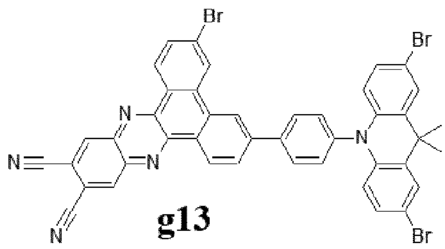

g13

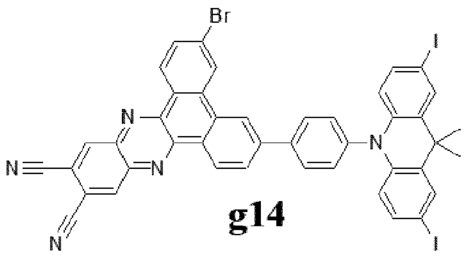

g14

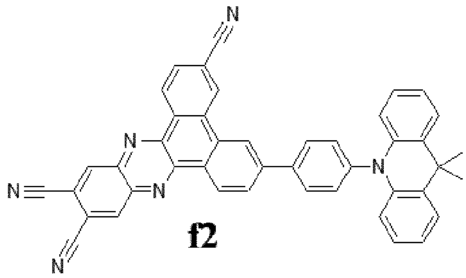

f2

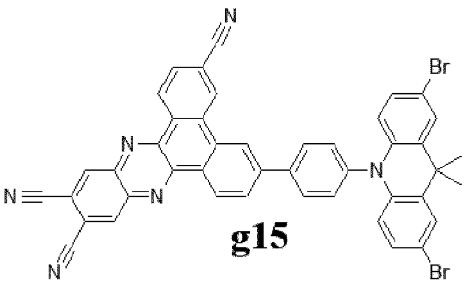

g15

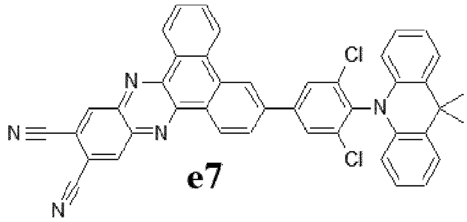

e7

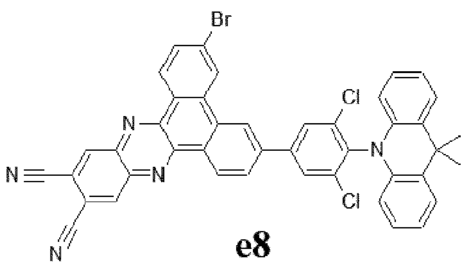

e8

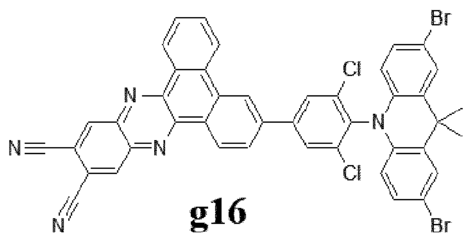

g16

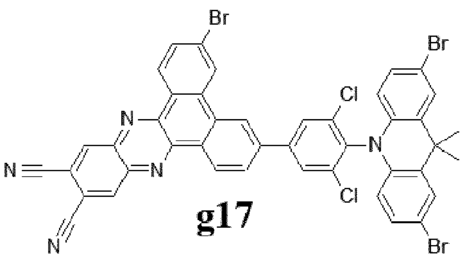

g17

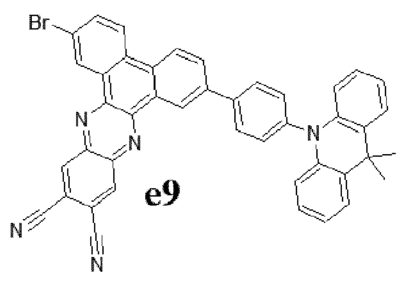

e9

Synthesis examples for preparation of compounds e1-9, f1-2, g1-17 from Table 1.

Examples for R1

Synthesis Example 1

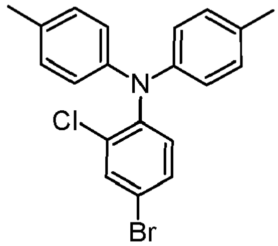

A mixture of a 4,4'-dimethyldiphenylamine (1.97 g, 10 mmol) and NaH (0.26 g, 11 mmol) was dissolved in dimethylformamide and stirred under an inert atmosphere until gas evolution ends. Then, 4-bromo-2-chloro-1-fluorobenzene (a1) (2.72 g, 13 mmol) was added and the mixture was heated at 150 °C with continuous stirring. The reaction progress was monitored by thin-layer chromatography (TLC). After completion of the reaction, the solvent was evaporated under reduced pressure and the residue was treated with 0.1 M HCl in water, followed by extraction with dichloromethane. The product was purified by column chromatography to obtain b1 as a white powder (yield 51%). MALDITOF-MS: *m*/*z:* calcd for $C_{20}H_{17}NBrCl$ 386.7, found 387.5 $[M+1]^+$.

Synthesis Example 2

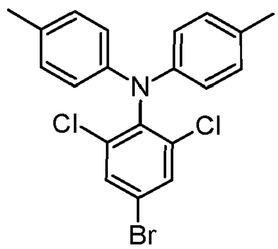

b2: A product was obtained in the same manner as in Synthesis Example 1 except that 5-bromo-1,3-dichloro-2-fluorobenzene (3.17 g, 13 mmol) was used instead of a1. After the purification, a white powder was obtained (yield 60%). $^1H$ NMR (500 MHz, $CDCl_3$-*d*): δ ppm 2.29 (s, 6 H), 6.84 (d, *J*=8.4 Hz, 2 H), 7.04 (d, *J*=8.4 Hz, 2 H), 7.55 (s, 2 H). $^{13}C$ NMR (125 MHz, $CDCl_3$-*d*): δ ppm 142.7, 140.0, 137.9, 132.3, 131.6, 129.7, 120.5, 120.0, 20.7. MALDITOF-MS: *m*/*z:* calcd for $C_{20}H_{16}NBrCl_2$ 421.2, found 421.2 $[M]^+$.

Synthesis Example 3

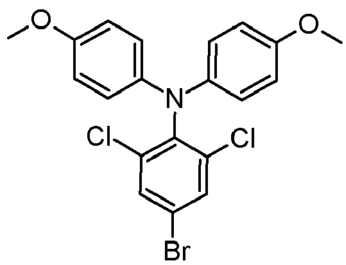

b3: A product was obtained in the same manner as in Synthesis Example 1 except that 4,4'-dimethoxydiphenylamine (2.29

g, 10 mmol) was used instead of 4,4'-dimethyldiphenylamine. After the purification, a pale yellow powder was obtained (yield 67%). $^1$H NMR (500 MHz, $CDCl_3$-*d*): 3.77 (s, 6 H), 6.78 (d, *J*=9.00 Hz, 4 H), 6.86 (d, *J*=9.00 Hz, 4 H), 7.54 (s, 2 H). MALDITOF-MS: *m*/*z*: calcd for $C_{20}H_{16}NO_2BrCl_2$ 452.0, found 453.3 [*M*+1]$^+$.

Synthesis Example 4

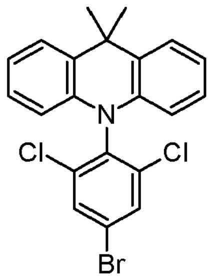

b4: A product was obtained in the same manner as in Synthesis Example 1 except that 9,10-dihydro-9,9-dimethylacridine (2.09 g, 10 mmol) was used instead of 4,4'-dimethyldiphenylamine. After the purification, a white powder was obtained (yield 55%). $^1$H NMR (500 MHz, $CDCl_3$-*d*): 1.72 (s, 6 H), 6.07 (d, *J*=8.0 Hz, 2 H), 6.96 - 7.04 (m, 4 H), 7.50 (d, *J*=7.5 Hz, 2 H), 7.76 (s, 2H). MALDITOF-MS: *m*/*z*: calcd for $C_{21}H_{16}NBrCl_2$ 431.0, found 415.3 *[M*- $CH_3$]$^+$.

Examples for R2

Synthesis Example 5

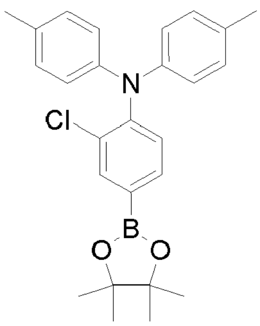

A mixture of 4-bromo-2-chloro-N,N-bis(4-methylphenyl)aniline (b1) (3.87 g, 10 mmol), bis(pinacolato)diboron (2.79 g, 11 mmol), potassium acetate (3.93 g, 40 mmol) and (1,1'-bis(diphenylphosphino)ferrocene)palladium(II) dichloride (0.073 g, 0.1 mmol) in dioxane (40 mL), was heated with continuous stirring under an inert atmosphere. The reaction progress was monitored by TLC. After the completion of the reaction, the solvent was evaporated under reduced pressure. The residue was then treated with water and extracted with dichloromethane. The product was purified by column chromatography to obtain c1 as a white powder (yield: 51%). $^1$H NMR (500 MHz, $CDCl_3$-*d*): δ ppm 1.34 (s, 12 H), 2.29 (s, 6 H), 6.84 (d, *J*=8.24 Hz, 4 H), 7.02 (d, *J*=8.24 Hz, 4 H), 7.15 (d, *J*=7.93 Hz, 1 H), 7.61 (d, *J*=7.93 Hz, 1 H), 7.84 (s, 1 H). MALDITOF-MS: *m/z:* calcd for $C_{26}H_{29}NO_2BCl$ 433.78, found 433.1 [*M*]$^+$.

Synthesis Example 6

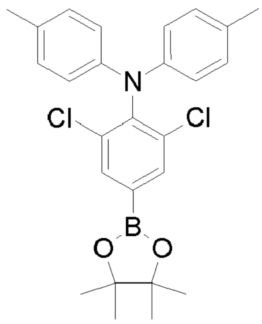

c2: A product was obtained in the same manner as in Synthesis Example 5 except that b2 (4.21 g, 10 mmol) was used instead of b1. After the purification, a white powder was obtained (yield: 60%). $^1$H NMR (500 MHz, $CDCl_3$-*d*): δ ppm 1.34 (s, 12 H), 2.28 (s, 6 H), 6.84 (d, *J*=8.4 Hz, 4 H), 7.02 (d, *J*=8.4 Hz, 4 H), 7.80 (s, 2 H). $^{13}$C NMR (125 MHz, $CDCl_3$-*d*): δ ppm 143.0, 142.8, 136.6, 135.5, 131.4, 129.6, 120.6, 120.4, 84.5, 24.9, 20.8. MALDITOF-MS: *m*/*z:* calcd for $C_{26}H_{28}NO_2BCl_2$ 468.2, found 468.4 [*M*]$^+$.

Synthesis Example 7

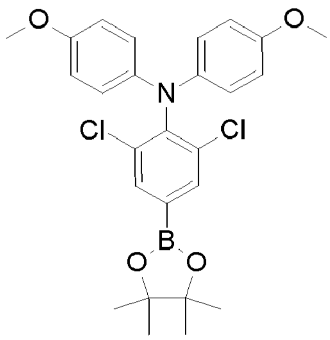

c3: A product was obtained in the same manner as in Synthesis Example 5 except that b3 (4.53 g, 10 mmol) was used instead of b1. After the purification, a pale yellow powder was obtained (yield 67%). $^1$H NMR (500 MHz, $CDCl_3$-*d*): δ ppm 1.34 (s, 12 H), 3.77 (s, 6 H), 6.78 (d, *J*=9.00, 4 H), 6.85 (d, *J*=9.00, 4 H), 7.79 (s, 2 H). MALDITOF-MS: *m*/*z:* calcd for $C_{26}H_{28}NO_4BCl_2$ 499.1, found 499.2 [*M*]$^+$.

Synthesis Example 8

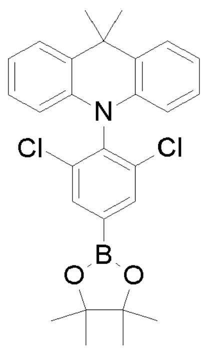

c4: A product was obtained in the same manner as in Synthesis Example 5 except that b4 (4.33 g, 10 mmol) was used instead of b1. After the purification, a pale yellow powder was obtained (yield 67%). $^1$H NMR (500 MHz, $CDCl_3$-*d*): δ ppm 1.39 (s, 12 H), 1.73 (s, 6 H), 6.06 (d, *J*=7.78, 2 H), 6.93 - 7.00 (m, 4 H), 7.49 (dd, *J*=7.48, 1.68 Hz, 2 H), 7.99 (s, 2 H). MALDITOF-MS: *m*/*z:* calcd for $C_{27}H_{28}NO_2BCl_2$ 479.2, found 464.2 *[M-* $CH_3$]$^+$.

Examples for R3

Synthesis Example 9

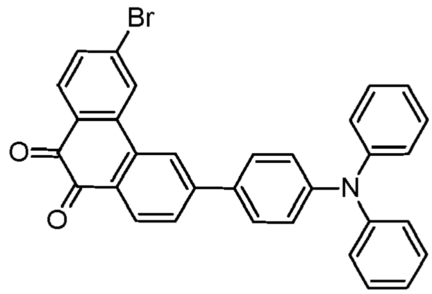

The mixture of 3,6-dibromophenanthrene-9,10-dione (3.66 g, 10 mmol), 4-(diphenylamino)phenylboronic acid (1.45 g, 5 mmol), $Pd(PPh_3)_4$ (0.12 g, 0.1 mmol) and 2 M aqueous $K_2CO_3$ solution in dioxane (20 mL), was refluxed under an inert atmosphere. The reaction progress was monitored by TLC. After the completion of the reaction, the solvent was evaporated under reduced pressure. The residue was then treated with 0.1 M HCl in water and extracted with dichloromethane. The product was purified by column chromatography to obtain d1 as a dark red powder (yield: 56%). $^1H$ NMR (500 MHz, $CDCl_3$-*d*): δ ppm 7.11 (t, J=7.32 Hz, 2 H), 7.16 - 7.20 (m, 6 H), 7.78 (t, *J*=7.38, 4 H), 7.58 (d, *J*=8.54 Hz, 2 H), 7.62 (dd, *J*=8.24, *J*=1.37, 1 H), 7.69 (d, *J*=8.09 Hz, 1 H), 8.07 (d, *J*=8.24 Hz, 1 H), 8.10 (s, 1 H), 8.24 (d, *J*=8.24 Hz, 1 H), 8.25 (d, *J*=8.24 Hz, 1H). MALDITOF-MS: *m*/*z*: calcd for $C_{32}H_{20}NO_2Br$ 530.4, found 531.1 $[M+1]^+$.

Synthesis Example 10

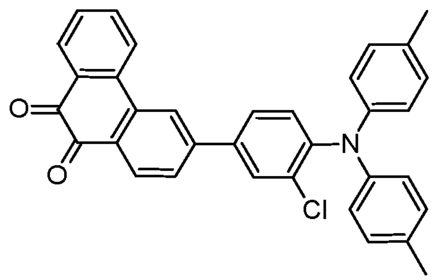

d2: A product was obtained in the same manner as in Synthesis Example 9 except that 3-bromophenanthrene-9,10-dione (2.87 g, 10 mmol) was used instead of 3,6-dibromophenanthrene-9,10-dione, and c1 (4.33 g, 10 mmol) was used instead of 4-(diphenylamino)phenylboronic acid. After the purification, a dark red powder was obtained (yield 90%). $^1H$ NMR (500 MHz, $CDCl_3$-*d*): δ ppm 2.32 (s, 6 H), 6.91 (d, *J*=8.7 Hz, 4 H), 7.07 (d, *J*=8.7 Hz, 4 H), 7.30 (d, *J*=8.4 Hz, 1 H), 7.50 - 7.55 (m, 3H), 7.62 (d, *J*=7.5 Hz, 1 H), 7.73 - 7.76 (m, 1H), 7.98 (d, *J*=7.9 Hz, 1 H), 8.05 (d, *J*=8.2 Hz, 1 H), 8.18 (s, 1H), 8.21 (d, *J*=7.8 Hz, 1 H). MALDITOF-MS: *m/z:* calcd for $C_{34}H_{24}NO_2Cl$ 513.1, found 514.3 $[M+1]^+$.

Synthesis Example 11

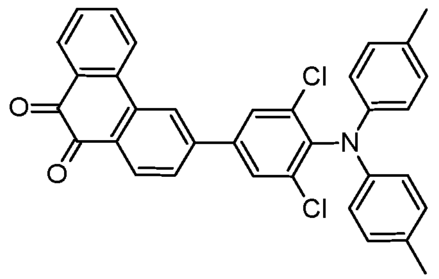

d3: A product was obtained in the same manner as in Synthesis Example 9 except that c2 (4.68 g, 10 mmol) was used instead of 4-(diphenylamino)phenylboronic acid. After the purification, a red powder was obtained (yield 86%). $^1H$ NMR (500 MHz, $CDCl_3$-*d*): δ ppm 2.28 (s, 6 H), 6.88 (d, *J*=8.7 Hz, 4 H), 7.80 (s, 2 H), 7.02 (d, *J*=8.7 Hz, 4 H), 7.52 (t, *J*=7.5 Hz, 1 H), 7.62 (d, J=8.3 Hz, 1 H), 7.75 (t, *J*=8.0 Hz, 1 H), 7.98 (d, *J*=7.8 Hz, 1 H), 8.05 (d, *J*=8.4 Hz, 1 H), 8.18 (s, 1H), 8.22 (d, *J*=7.8 Hz, 1 H). MALDITOF-MS: *m*/*z:* calcd for $C_{34}H_{23}NO_2Cl_2$ 548.1, found 549.2 $[M+1]^+$.

Synthesis Example 12

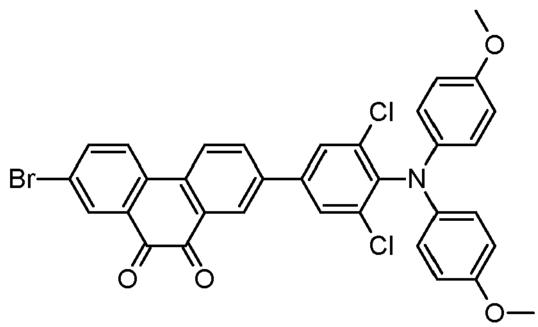

d4: A product was obtained in the same manner as in Synthesis Example 9 except that 2,7-dibromophenanthrene-9,10-dione (4.33 g, 10 mmol) was used instead of 3,6-dibromophenanthrene-9,10-dione, and c3 (2.50 g, 5 mmol) was used instead of 4-(diphenylamino)phenylboronic acid. After the reaction, a dark brown powder was obtained (yield 43%). The product was recrystallized, subjected to MALDITOF analysis, and used in the next step without further purification. MALDITOF-MS: *m*/*z:* calcd for $C_{34}H_{22}NO_4Cl_2Br$ 660.0, found 661.1 $[M+1]^+$.

Synthesis Example 13

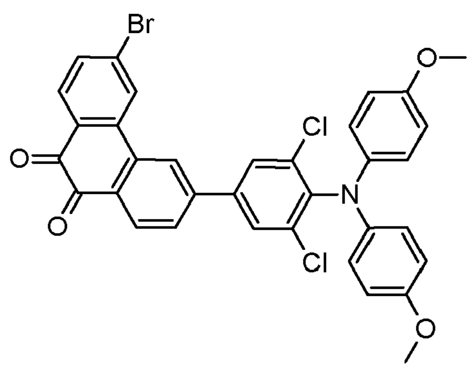

d5: A product was obtained in the same manner as in Synthesis Example 9 except that c3 (2.5 g, 5 mmol) was used instead of 4-(diphenylamino)phenylboronic acid. After the purification, a dark brown powder was obtained (yield 86%). $^1H$ NMR (500 MHz, $CDCl_3$-*d*): δ ppm 3.79 (s, 6 H), 6.81 (d, *J*=8.85 Hz, 4 H), 6.93 (d, *J*=8.85 Hz, 4 H), 7.66 - 7.71 (m, 2 H), 7.72 (s, 2 H) 8.08 (d, *J*=7.93 Hz, 2 H), 8.27 (s, 1 H), 8.31 (d, *J*=8.09 Hz, 1 H). MALDITOF-MS: *m*/*z:* calcd for $C_{34}H_{22}NO_4Cl_2Br$ 660.0,

found 661.1 [*M*+1]+.

Synthesis Example 14

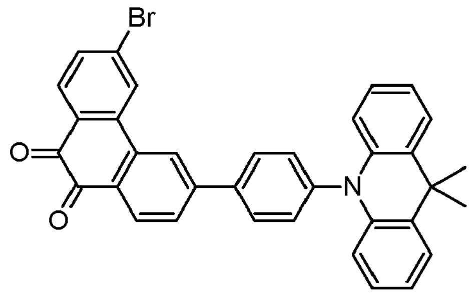

d6: A product was obtained in the same manner as in Synthesis Example 9 except that 9,9-dimethyl-10-(4-(4,4,5,5-tetramethyl-1,3,2-dioxaborolan-2-yl)phenyl)-9,10-dihydroacridine (2.05 g, 5 mmol) was used instead of 4-(diphenylamino)phenylboronic acid. After the purification, a pale orange powder was obtained (yield 63%). $^1$H NMR (500 MHz, $CDCl_3$-*d*): δ ppm 1.77 (s, 6 H), 6.17 (d, *J*=8.0 Hz, 2 H), 6.99 - 7.07 (m, 4 H), 7.53 - 7.58 (m, 3 H), 7.76 (d, *J*=7.9 Hz, 1 H), 7.80 (t, *J*=7.7 Hz, 1 H), 7.91 (s, 2H), 8.20 (d, *J*=7.9 Hz, 1 H), 8.26 - 8.29 (m, 2H), 8.35 (d, *J*=8.3 Hz, 1 H). MALDITOF-MS: *m/z:* calcd for $C_{35}H_{24}NO_2Br$ 571.1, found 556.1 *[M-* $CH_3]^+$.

Synthesis Example 15

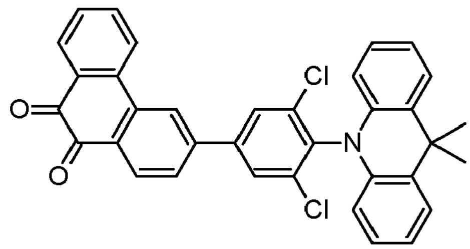

d7: A product was obtained in the same manner as in Synthesis Example 9 except that that 3-bromophenanthrene-9,10-dione (2.87 g, 10 mmol) was used instead of 3,6-dibromophenanthrene-9,10-dione and c4 (4.80 g, 10 mmol) was used instead of 4-(diphenylamino)phenylboronic acid. After the purification, a pale orange powder was obtained (yield 76%). $^1$H NMR (500 MHz, $CDCl_3$-*d*): 1.77 (s, 6 H), 6.17 (d, *J*=8.0 Hz, 2 H), 6.99 - 7.07 (m, 4 H), 7.53 - 7.58 (m, 3 H), 7.76 (d, *J*=7.9 Hz, 1 H), 7.80 (t, *J*=7.7 Hz, 1 H), 7.91 (s, 2H), 8.20 (d, *J*=7.9 Hz, 1 H), 8.26 - 8.29 (m, 2H), 8.35 (d, *J*=8.3 Hz, 1 H). MALDITOF-MS: *m/z:* calcd for $C_{35}H_{23}NO_2Cl_2$ 561.1, found 546.1 *[M-* $CH_3]^+$.

Synthesis Example 16

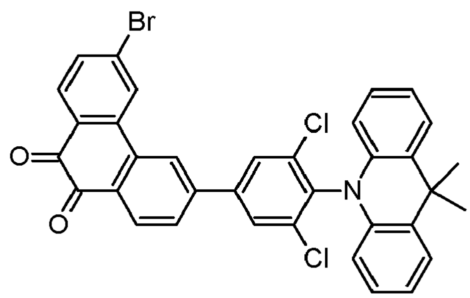

d8: A product was obtained in the same manner as in Synthesis Example 9 except that c4 (2.40 g, 5 mmol) was used instead of 4-(diphenylamino)phenylboronic acid. After the reaction, an orange powder was obtained (yield 46%). The product was recrystallized, subjected to MALDITOF analysis, and used in the next step without further purification. MALDITOF-MS: *m/z:* calcd for $C_{35}H_{22}NO_2Cl_2Br$ 640.9, found 625.9 *[M-* $CH_3]^+$.

Synthesis Example 17

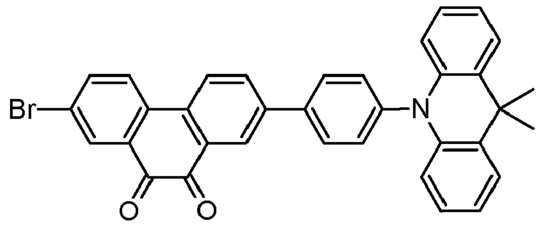

d9: A product was obtained in the same manner as in Synthesis Example 9 except that 2,7-dibromophenanthrene-9,10-dione (4.33 g, 10 mmol) was used instead of 3,6-dibromophenanthrene-9,10-dione and 9,9-dimethyl-10-(4-(4,4,5,5-tetramethyl-1,3,2-dioxaborolan-2-yl)phenyl)-9,10-dihydroacridine (2.10 g, 5 mmol) was used instead of 4-(diphenylamino)phenylboronic acid. After the reaction, an orange powder was obtained (yield 79%). The product was recrystallized, subjected to MALDITOF analysis, and used in the next step without further purification. MALDITOF-MS: *m/z:* calcd for $C_{35}H_{24}NO_2Br$ 569.1, found 588.1 [*M* + $H_3O]^+$.

Examples for R4

Synthesis Example 18

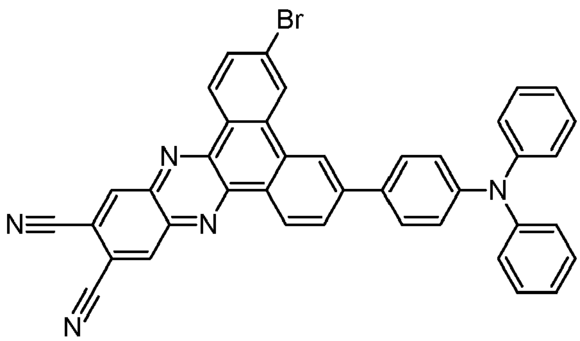

e1: Compound d1 (0.53 g, 1 mmol), 4,5-diaminophthalonitrile (0.16 g, 1 mmol) and concentrated HCl (0.5 mL) were dissolved in a mixture of tetrahydrofuran with ethanol (1:1, v:v, 35 mL) and stirred at room temperature. The reaction progress was monitored by TLC. After the completion of the reaction, the solvent was evaporated under reduce pressure, and the resiude was purified by silica gel column chromatography and subsequently recrystallized to obtain e1 as a dark red powder (yield 95%). $^1H$ NMR (500 MHz, $CDCl_3$-*d*): δ ppm 7.10 (t, *J*=8.8 Hz, 2 H), 7.18 - 7.25 (m, 6 H), 7.29 - 7.37 (m, 4H), 7.69 (d, *J*=9.3 Hz, 2 H), 7.88 (d, *J*=8.1 Hz, 1 H), 7.98 (d, *J*=8.1 Hz, 1 H), 8.57 (s, 1 H), 8.70 - 8.78 (m, 3 H), 9.16 (d, *J*=8.7 Hz, 1 H), 9.30 (d, *J*=8.7 Hz, 1 H). MALDITOF-MS: *m/z:* calcd for $C_{40}H_{22}N_5$. MALDITOF-MS: *m/z:* calcd for $C_{40}H_{22}N_5Br$ 651.1, found 651.1 [*M*]$^+$.

Synthesis Example 19

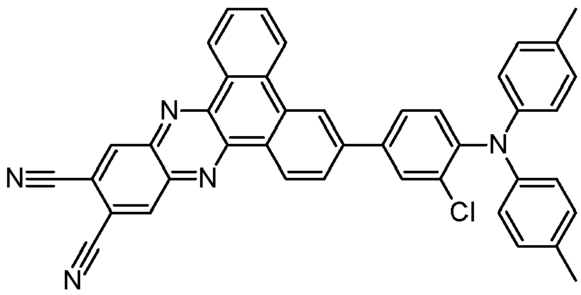

e2: A product was obtained in the same manner as in Synthesis Example 18 except that d2 (0.51 g, 1 mmol) was used instead of d1. After the purification, a dark red powder was obtained (yield 90%). $^1$H NMR (500 MHz, $CDCl_3$-*d*): δ ppm 2.32 (s, 6 H), 6.94 (d, J=8.4 Hz, 4 H), 7.08 (d, *J*=8.4 Hz, 4 H), 7.35 (d, *J*=8.1 Hz, 1 H), 7.66 (dd, *J*=8.1 Hz, *J*=2.2 Hz, 1 H), 7.81 (t, *J*=7.4 Hz, 1 H), 7.87 (d, J=2.0 Hz, 1 H), 7.91 (t, *J*=7.4 Hz, 1 H), 7.97 (d, *J*=8.1 Hz, 1 H), 8.64 (d, *J*=8.3 Hz, 1 H), 8.70 (d, *J*=8.3 Hz, 1 H), 8.74 (s, 2H), 9.32 - 9.37 (m, 2H). MALDITOF-MS: *m*/*z:* calcd for $C_{42}H_{26}N_5Cl$ 635.2, found 635.2 [*M*]$^+$.

Synthesis Example 20

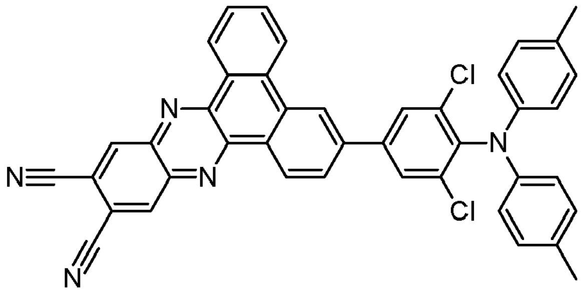

e3: A product was obtained in the same manner as in Synthesis Example 18 except that d3 (0.55 g, 1 mmol) was used instead of d1. After the purification, a red powderwas obtained (yield 86%). $^1$H NMR (500 MHz, $CDCl_3$-*d*): δ ppm 2.31 (s, 6 H), 6.95 (d, J=8.6 Hz, 4 H), 7.07 (d, J=8.6 Hz, 4 H), 7.84 (s, 2H), 7.90 (t, J=7.2 Hz, 1 H), 7.94 (t, J=7.2 Hz, 1 H), 7.98 (d, J=8.3 Hz, 1 H), 8.66 (d, J=8.3 Hz, 1 H), 8.77 (d, J=8.7 Hz, 1 H), 8.79 (s, 2H), 9.37 (d, J=7.5 Hz, 1 H), 9.41 (d, J=7.9 Hz, 1H). MALDITOF-MS: *m*/*z:* calcd for $C_{42}H_{25}N_5Cl_2$ 669.1, found 670.2 [*M*+1]$^+$.

Synthesis Example 21

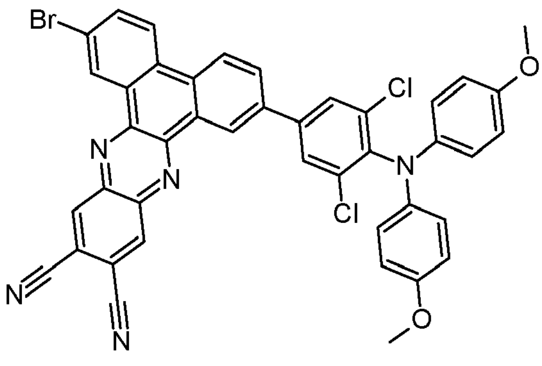

e4: A product was obtained in the same manner as in Synthesis Example 18 except that d4 (0.55 g, 1 mmol) was used instead of d1. After the purification, a brown powder was obtained (yield 87%). $^1$H NMR (500 MHz, $CDCl_3$-*d*): δ ppm 3.80 (s, 6 H), 6.84 (d, J=9.00 Hz, 4 H), 6.98 (d, *J*=9.00 Hz, 4 H), 7.88 (s, 2 H), 8.03 (dd, *J*=8.70, 1.98 Hz, 1H), 8.11 (d, *J*=8.55 Hz, 1H), 8.47 (d, J=8.85 Hz, 1 H), 8.62 (d, *J*=8.55 Hz, 1 H), 8.85 (s, 1 H), 8.91 (s, 1 H), 9.49 (d, *J*=1.98 Hz, 1 H), 9.55 (d, *J*=1.68 Hz, 1 H). MALDITOF-MS: *m*/*z:* calcd for $C_{42}H_{24}N_5O_2Cl_2$ 781.0, found 781.0 [*M*]$^+$.

Synthesis Example 22

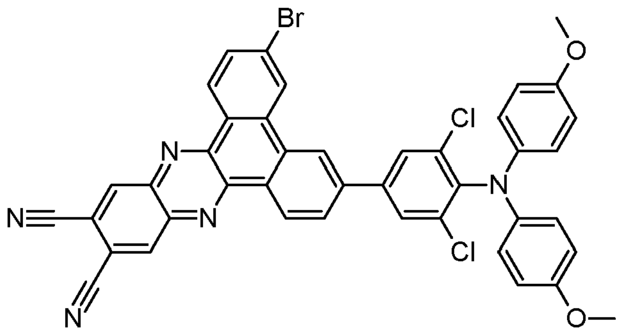

e5: A product was obtained in the same manner as in Synthesis Example 18 except that d5 (0.66 g, 1 mmol) was used instead of d1. After the purification, a dark brown powder was obtained (yield 89%). $^1$H NMR (500 MHz, $CDCl_3$-*d*): δ ppm 3.80 (s, 6 H), 6.83 (d, J=8.85 Hz, 4 H), 6.97 (d, J=8.85 Hz, 4 H), 7.83 (s, 2 H), 7.91 (*d*, J=8.70 Hz, 1 H), 7.98 (*d*, J=8.39 Hz, 1 H), 8.57 (s, 1 H), 8.73 (s, 1 H), 8.75 (s, 1 H), 8.78 (s, 1 H), 9.16 (d, J=8.70 Hz, 1 H), 9.36 (*d*, J=8.39 Hz, 1 H). MALDITOF-MS: *m*/*z:* calcd for $C_{42}H_{24}N_5O_2Cl_2$ 781.0, found 781.0 [*M*]$^+$.

Synthesis Example 23

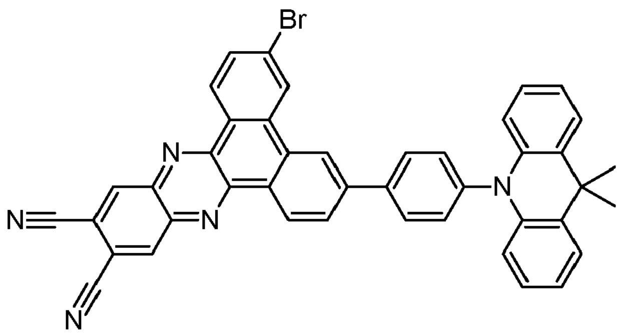

e6: A product was obtained in the same manner as in Synthesis Example 18 except that d6 (0.56 g, 1 mmol) was used instead of d1. After the purification, an orange powder was obtained (yield 90%). $^1$H NMR (500 MHz, $CDCl_3$-*d*): δ ppm 1.30 (s, 3 H), 1.65 (s, 3 H), 6.28 (m, *J*=8.09 Hz, 2 H), 6.88 - 6.95 (m, 4 H), 7.42 (dd, *J*=7.55, 1.30 Hz, 2 H), 7.46 (d, *J*=8.24 Hz, 2 H), 7.60 (dt, *J*=8.35, 1.77 Hz, 2 H), 7.76 (dd, *J*=8.01, 1.30 Hz, 1 H), 7.90 (d, *J*=8.24 Hz, 2 H), 7.98 - 8.05 (m, 2 H), 8.18 (s, 1 H), 8.24 (d, *J*=1.37, 1 H), 8.29 (d, J=8.09 Hz, 1 H). MALDITOF-MS: *m/z:* calcd for $C_{43}H_{26}N_5Br$ 691.14, found 676.13 *[M-* $CH_3$]$^+$.

Synthesis Example 24

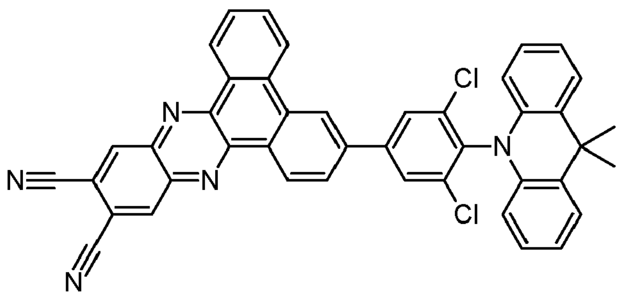

e7: A product was obtained in the same manner as in Synthesis Example 18 except that d7 (0.56 g, 1 mmol) was used instead of d1. After the purification, a yellow powder was obtained (yield 81%). $^1$H NMR (500 MHz, $CDCl_3$-*d*): δ ppm 1.43 (s, 3 H), 1.78 (s, 3 H), 6.21 (d, *J*=8.2 Hz, 2 H), 7.00 (t, *J*=8.0 Hz, 2 H), 7.04 (t, *J*=9.0 Hz, 2 H), 7.54 (d, J=7.5 Hz, 2 H), 7.86 (t, *J*=7.7 Hz, 1 H), 7.97 (t, *J*=7.7 Hz, 1 H), 8.03 (s, 2H), 8.07 (d, *J*=8.0 Hz, 1 H), 8.73 (d, J=8.2 Hz, 1 H), 8.82 - 8.85 (m, 3 H), 9.42 (d, *J*=8.0 Hz, 1 H), 9.50 (d, *J*=8.0 Hz, 1 H). MALDITOF-MS: *m*/*z:* calcd for $C_{43}H_{25}N_5Cl_2$ 681.1, found 666.2 *[M*- $CH_3]^+$.

Synthesis Example 25

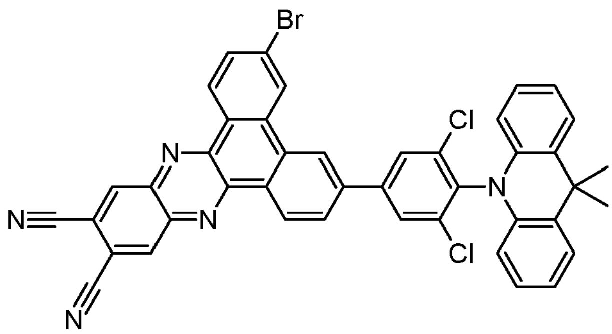

e8: A product was obtained in the same manner as in Synthesis Example 18 except that d8 (0.64 g, 1 mmol) was used instead of d1. After the purification, an orange powder was obtained (yield 80%). $^1$H NMR (500 MHz, $CDCl_3$-*d*): δ ppm 1.28 (s, 3 H), 1.78 (s, 3 H), 6.22 (d, *J*=8.0 Hz, 2 H), 6.98 - 7.08 (m, 4 H), 7.54 (d, *J*= 8.2 Hz, 2 H), 7.93 (d, *J*= 9.2 Hz, 2 H), 7.97 (d, *J*= 9.2 Hz, 1 H), 8.02 (s, 1 H), 8.10 (d, *J*= 9.2 Hz, 2 H), 8.78 - 8.85 (m, 3 H), 9.26 (d, *J*=8.3 Hz, 1 H), 9.49 (d, *J*=8.3 Hz, 1 H). MALDITOF-MS: *m*/*z:* calcd for $C_{43}H_{24}N_5BrCl_2$ 761.5, found 746.2 [*M* - $CH_3]^+$.

Synthesis Example 26

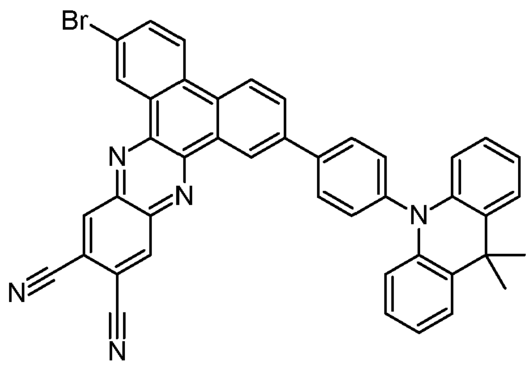

e9: A product was obtained in the same manner as in Synthesis Example 18 except that d9 (0.57 g, 1 mmol) was used instead of d1. After the purification, an orange powder was obtained (yield 86%). $^1$H NMR (500 MHz, $CDCl_3$-*d*): δ ppm 2.35 (s, 6 H), 6.40 (d, J=7.78 Hz, 2 H), 6.98 - 7.05 (m, 4 H), 7.35 (s, 2 H), 7.50 - 7.75 (m, 4 H), 8.03 (dd, *J*= 8.55 Hz, *J*= 1.83 Hz 1 H), 8.25 (dd, *J*= 7.93 Hz, *J*= 1.37 Hz, 1 H), 8.50 (d, *J*= 8.70 Hz, 1 H), 8.66 (d, J=8.70 Hz, 1 H), 8.87 (s, 1 H), 8.91 (s, 1 H), 9.47 (d, J=1.98 Hz, 1 H), 9.52 (d, J=1.83 Hz, 1 H). MALDITOF-MS: *m*/*z:* calcd for $C_{43}H_{26}N_5Br$ 692.6, found 678.1 [M - $CH_3]^+$.

Examples for R5

Synthesis Example 27

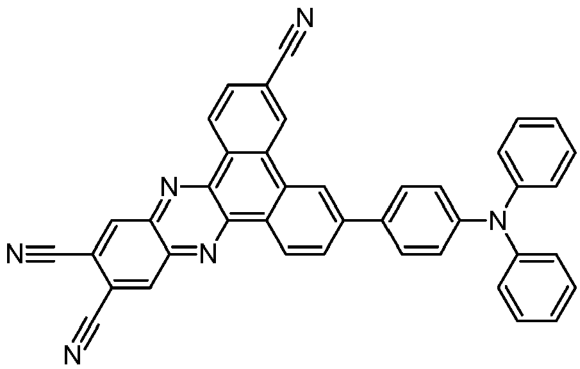

f1: A mixture of e1 (0.65 g, 1 mmol) and CuCN (0.18 g, 2 mmol) was dissolved in *N*-methyl-pyrrolidone and heated at 150 °C. The reaction progress was monitored using TLC. After the completion of the reaction, methanol was added to the reaction mixture. The resulting precipitated solid was collected by filtration and purified through silica gel column chromatography to obtain f1 as a black powder, yield 65%. $^1H$ NMR (500 MHz, $CDCl_3$-*d*): 7.12 (t, *J*=8.5 Hz, 2 H), 7.20 - 7.26 (m, 6 H), 7.32 - 7.39 (m, 4H), 7.68 - 7.72 (m, 2 H), 7.97 - 8.03 (m, 2 H), 8.59 (s, 1 H), 8.78 - 8.81 (m, 2 H), 8.90 (s, 1 H), 9.35 - 9.38 (m, 1 H), 9.43 (d, J=8.7 Hz, 1 H). MALDITOF-MS: *m/z:* calcd for $C_{41}H_{22}N_6$ 598.2, found 598.2 $[M]^+$.

Synthesis Example 28

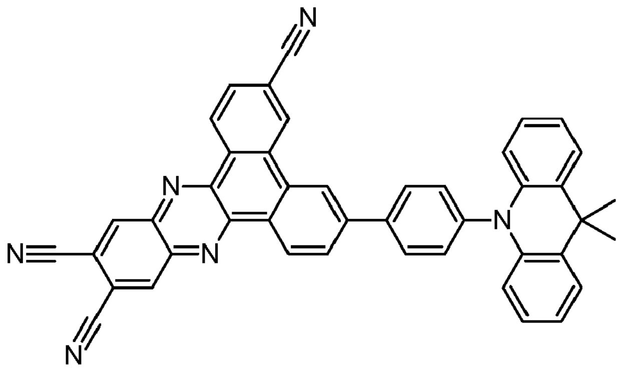

f2: A product was obtained in the same manner as in Synthesis Example 27 except that e6 (0.70 g, 1 mmol) was used instead of e1. After the purification, an orange powder was obtained (yield 86%). $^1H$ NMR (500 MHz, $CDCl_3$-*d*): δ ppm 1.70 (s, 6H), 6.33 (d, *J*=8.39 Hz, 2 H), 6.88 - 6.92 (m, 4 H), 7.42 - 7.47 (m, 4 H), 8.00 - 8.04 (m, 3 H), 8.16 (d, *J*=8.09 Hz, 1 H), 8.77 (s, 1 H), 8.82 (d, *J*=6.71 Hz, 2 H), 8.97 (s, 1 H), 9.45 (t, *J*=9.08 Hz, 2 H). MALDITOF-MS: *m/z:* calcd for $C_{44}H_{26}N_6$ 638.2, found 623.2 $[M- CH_3]^+$.

Examples for R6

Synthesis Example 29

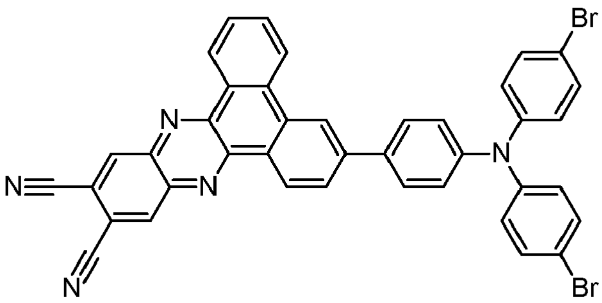

g1: A mixture of Da-CNBPz-H (0.57 g, 1 mmol) and N-bromosuccinimide (0.37 g, 2.1 mmol) in chloroform (15 mL) was stirred at room temperature in the absence of light. The reaction progress was monitored by TLC. After the completion of the reaction, the solution was concentrated and MeOH was added to the residue. The precipitated solid was filtered, purified by silica gel column chromatography and recrystallized several times to obtain g1 as a red powder (yield 91%). $^1H$ NMR (500 MHz, $CDCl_3$-*d*): δ ppm 7.08 (d, *J*=8.51 Hz, 4 H), 7.25 (d, *J*=8.79 Hz, 2 H), 7.45 (d, *J*=8.51 Hz, 4 H), 7.77 (d, *J*=8.51 Hz, 3 H), 7.88 (s, 2 H), 8.04 (d, *J*=7.41 Hz, 3 H), 8.69 (d, *J*=8.24 Hz, 1 H), 8.75 (s, 1 H), 9.47 (d, *J*=8.24 Hz, 2 H). MALDITOF-MS: *m*/*z:* calcd for $C_{40}H_{21}N_5Br_2$ 731.0, found 731.0 [*M*]$^+$.

Synthesis Example 30

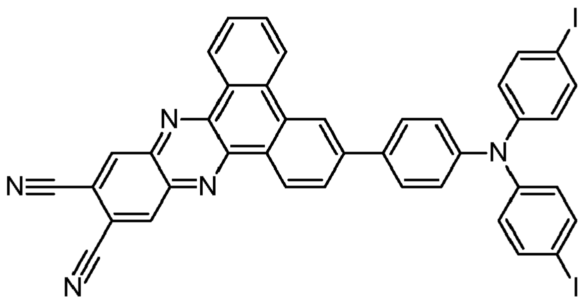

g2: A product was obtained in the same manner as in Synthesis Example 29 except that *N*-iodosuccinimide (0.47 g, 2.1 mmol) was used instead of *N*-bromosuccinimide. After the purification, a dark red powder was obtained (yield 79%). $^1H$ NMR (500 MHz, $CDCl_3$-*d*): δ ppm 6.93 (d, *J*=9.3 Hz, 4 H), 7.21 (d, *J*=8.2 Hz, 2 H), 7.60 (d, *J*=9.3 Hz, 4 H), 7.73 (d, *J*=8.2 Hz, 2 H), 7.81 (t, *J*=7.5 Hz, 1 H), 7.92 (t, *J*=7.5 Hz, 1 H), 7.98 (d, *J*=8.6 Hz, 1 H), 8.65 (d, *J*=8.6 Hz, 1 H), 8.70 - 8.79 (m, 3 H), 9.35 (d, *J*=8.3 Hz, 2 H). MALDITOF-MS: *m*/*z:* calcd for $C_{40}H_{21}N_5I_2$ 825.0, found 825.0 [*M*]$^+$.

Synthesis Example 31

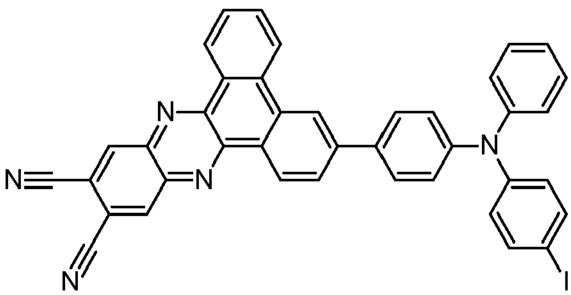

g3: A product was obtained in the same manner as in Synthesis Example 29 except that a reduced amount of *N*-bromosuccinimide (0.18 g, 1.1 mmol) was used. After the purification, a red powder was obtained (yield 73%). MALDITOF-MS: *m*/*z:* calcd for $C_{40}H_{22}N_5Br$ 652.5, found 653.1 [*M*]$^+$. NMR spectrum not obtained due to weak solubility.

Synthesis Example 32

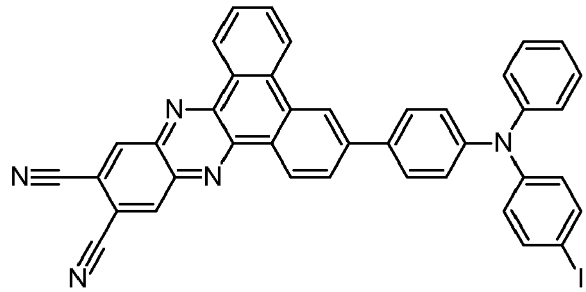

g4: A product was obtained in the same manner as in Synthesis Example 29 except that N-iodosuccinimide (0.23 g, 1.1 mmol) was used instead of N-bromosuccinimide. After the purification a dark red powder was obtained (yield 78%). MALDITOF-MS: *m/z:* calcd for $C_{40}H_{22}N_5I$ 699.5, found 700.3 [*M*+H]$^+$. NMR spectrum not obtained due to weak solubility.

Synthesis Example 33

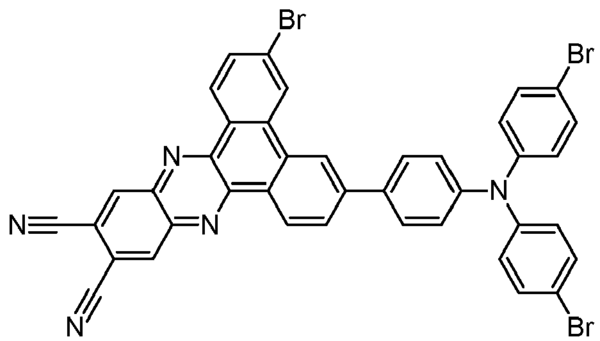

g5: A product was obtained in the same manner as in Synthesis Example 29 except that e1 (0.65 g, 1 mmol) was used instead of Da-CNBPz-H. After the purification, a red powder was obtained (yield 88%). $^1$H NMR (500 MHz, $CDCl_3$-*d*) δ ppm 7.05 (m, *J*=6.10 Hz, 4 H) 7.24 (d, 2 H) 7.43 (d, *J*=7.32 Hz, 4 H) 7.72 (d, 2 H) 7.88 (2 H) 7.98 (1 H) 8.57 (br. s., 1 H) 8.76 (d, *J*=6.71 Hz, 1 H) 9.16 (br. s., 1 H) 9.31 (br. s., 1 H). MALDITOF-MS: *m/z* calcd for $C_{40}H_{20}N_5Br_3$ 807.9, found 808.9 [M+1]$^+$.

Synthesis Example 34

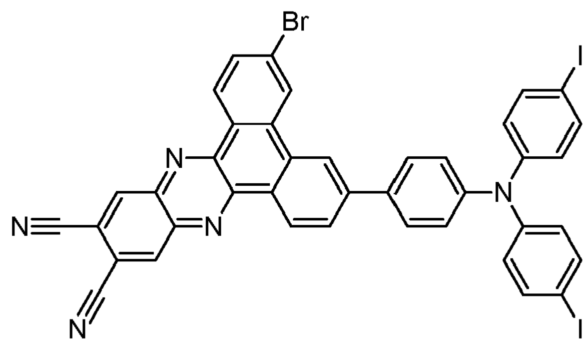

g6: A product was obtained in the same manner as in Synthesis Example 29 except that N-iodosuccinimide (0.47 g, 2.1 mmol) was used instead of *N*-bromosuccinimide, and e1 (0.65 g, 1 mmol) was used instead of Da-CNBPz-H. After the purification, an orange powder was obtained (yield 83%). $^1$H NMR (500 MHz, $CDCl_3$-*d*) δ ppm 6.94 (d, 4 H) 7.21 - 7.24 (m, 2

H) 7.61 (d, *J*=7.02 Hz, 4 H) 7.72 (2 H) 7.90 (1 H) 7.98 (2 H) 8.58 (br. s., 1 H) 8.77 (2 H) 9.17 (br. s., 1 H) 9.32 (br. s., 1 H). MALDITOF-MS: *m*/*z* calcd for $C_{40}H_{20}N_5BrI_2$ 902.90, found 904.96 $[M+1]^+$.

Synthesis Example 35

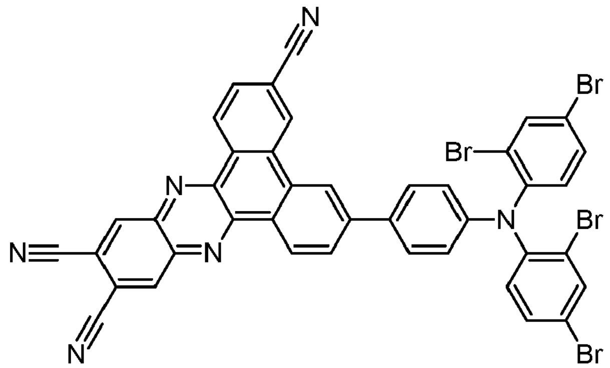

g7: A product was obtained in the same manner as in Synthesis Example 29 except that an increased amount of *N*-bromosuccinimide (0.73 g, 4.1 mmol) was used, and f1 (0.65 g, 1 mmol) was used instead of Da-CNBPz-H. After the purification, an orange powder was obtained (yield 64%). $^1$H NMR (500 MHz, $CDCl_3$-*d*) δ ppm 6.60 (d, *J*=8.8 Hz, 1 H), 6.63 (d, *J*=8.8 Hz, 1 H), 6.98 (s, 1H), 7.31 (d, *J*=7.7 Hz, 2 H), 7.40 (s, 1H), 7.54 (s, 1 H), 7.63 (d, *J*=9.3 Hz, 1 H), 7.74 (d, *J*=9.3 Hz, 1 H), 7.97 - 8.03 (m, 3 H), 8.61 (s, 1 H), 8.80 (br. s., 2 H), 8.90 (s, 1 H), 9.36 - 9.40 (m, 1 H), 9.44 (d, *J*=8.8 Hz, 1 H). MALDITOF-MS: *m*/*z* calcd for $C_{41}H_{18}Br_4N_6$ 914.8, found 915.9 $[M+1]^+$.

Synthesis Example 36

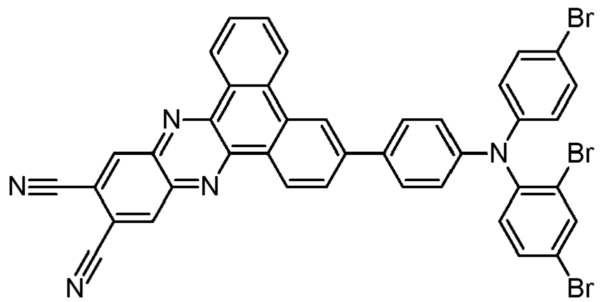

g8: A product was obtained in the same manner as in Synthesis Example 29 except that an increased amount of N-bromosuccinimide (0.55 g, 3.1 mmol) was used. After the purification, an orange powder was obtained (yield 91%). $^1$H NMR spectrum not obtained due to too low solubility. MALDITOF-MS: *m*/*z* calcd for $C_{40}H_{20}Br_3N_5$ 808.9, found 808.9 $[M]^+$.

Synthesis Example 37

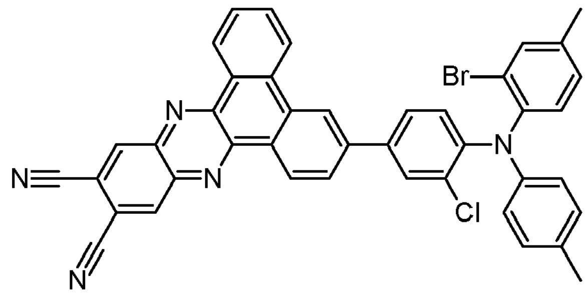

g9: A product was obtained in the same manner as in Synthesis Example 29 except that an reduced amount of *N*-bromosuccinimide (0.18 g, 1.1 mmol) was used, and e2 (0.64 g, 1 mmol) was used instead of Da-CNBPz-H. After the purification, a dark red powder was obtained (yield 90%). $^1$H NMR (500 MHz, $CDCl_3$-*d*) δ ppm 2.32 (s, 6 H), 6.80 - 6.90 (m, 3H), 6.99 (d, *J*=8.4 Hz, 1 H), 7.06 (d, *J*=8.4 Hz, 2 H), 7.46 (br. s, 2 H), 7.59 (d, *J*=8.4 Hz, 1 H), 7.79 - 7.84 (m, 2H), 7.79 (t, *J*=7.0 Hz, 1 H), 7.96 (d, *J*=8.4 Hz, 1 H), 8.49 (d, *J*=7.7 Hz, 1 H), 8.63 (d, *J*=7.7 Hz, 1 H), 8.69 (s, 2H), 9.31 - 9.36 (m, 2H). MALDITOF-MS: *m*/*z:* calcd for $C_{42}H_{25}N_5ClBr$ 713.1, found 714.2 [*M*+1]$^+$.

Synthesis Example 38

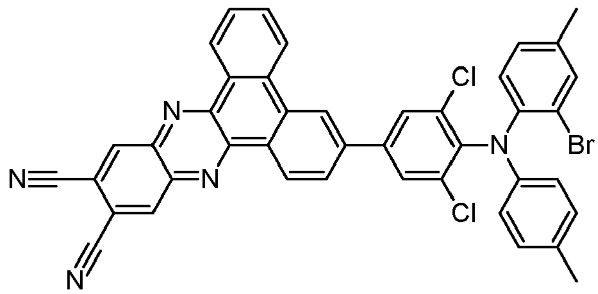

g10: A product was obtained in the same manner as in Synthesis Example 29 except that an reduced amount of *N*-bromosuccinimide (0.18 g, 1.1 mmol) was used, and e3 (0.67 g, 1 mmol) was used instead of Da-CNBPz-H. After the purification, a red powder was obtained (yield 86%). $^1$H NMR (500 MHz, $CDCl_3$-*d*) δ ppm 2.32 (s, 6 H), 6.69 (d, *J*=8.5 Hz, 2 H), 6.99 - 7.06 (m, 5 H), 7.80 (s, 2H), 7.88 (t, *J*=7.5 Hz, 1 H), 7.92 (t, *J*=7.4 Hz, 1 H), 7.96 (d, *J*=8.5 Hz, 1 H), 8.66 (d, *J*=8.5 Hz, 1 H), 8.75 (s, *J*=8.5 Hz, 1 H), 8.79 (s, 2H), 9.34 (d, *J*=8.5 Hz, 1 H), 9.38 (d, *J*=8.7 Hz, 1H). MALDITOF-MS: *m*/*z:* calcd for $C_{42}H_{24}N_5Cl_2Br$ 748.5, found 748.1 [*M*+1]$^+$.

Synthesis Example 39

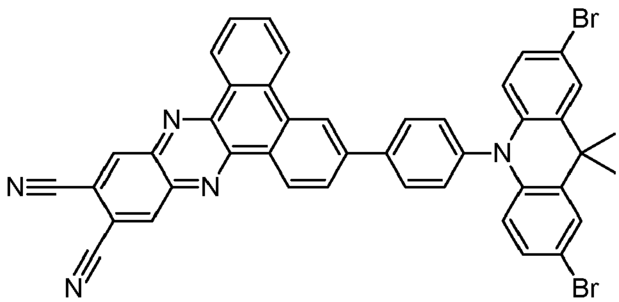

g11: A product was obtained in the same manner as in Synthesis Example 29 except that Ac-CNBPz (0.61 g, 1 mmol) was used instead of Da-CNBPz-H. After the purification, an orange powder was obtained (yield 91%). $^1$H NMR (500 MHz, $CDCl_3$-*d*): δ ppm 1.69 (s, 6 H), 6.27 (d, *J*=8.8 Hz, 2 H), 7.11 (dd, *J*=8.8 Hz, J=2.2 Hz, 2 H), 7.50 (d, *J*=8.2 Hz, 2 H), 7.55 (d, *J*=2.2 Hz, 2 H), 7.86 (t, *J*=8.0 Hz, 1 H), 7.96 (t, *J*=8.0 Hz , 1 H), 8.08 - 8.14 (m, 3 H), 8.73 (d, *J*=8.0 Hz, 1 H), 8.83 (d, *J*=1.8 Hz, 2 H), 8.87 (s, 1 H), 9.40 (d, *J*=8.0 Hz, 1 H), 9.47 (d, *J*=8.4 Hz, 1 H). MALDITOF-MS: *m*/*z:* calcd for $C_{43}H_{25}N_5Br_2$ 769.0, found

754.1 [*M* - $CH_3$]$^+$.

Synthesis Example 40

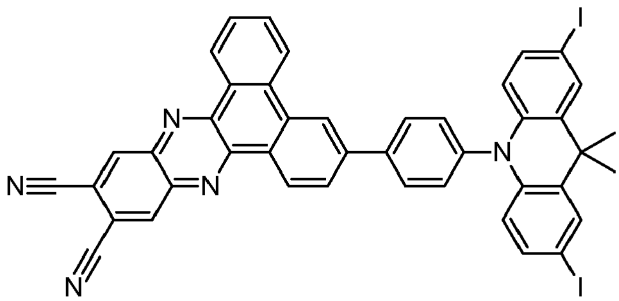

g12: A product was obtained in the same manner as in Synthesis Example 29 except that *N*-iodosuccinimide (0.47 g, 2.1 mmol) was used instead of *N*-bromosuccinimide, and Ac-CNBPz (0.63 g, 1 mmol) was used instead of Da-CNBPz-H. After the purification, an orange powder was obtained (yield 91%). $^1$H NMR (500 MHz, $CDCl_3$-*d*): δ ppm 1.68 (s, 6 H), 6.15 (d, *J*=8.9 Hz, 2 H), 7.27 - 7.31 (m, 2 H), 7.49 (d, *J*=8.8 Hz, 2 H), 7.71 (d, *J*=2.5 Hz, 2 H), 7.85 (t, *J*=7.7 Hz, 1 H), 7.96 (t, *J*=7.7 Hz, 1 H), 8.07 - 8.14 (m, 3 H), 8.73 (d, *J*=8.0 Hz, 1 H), 8.82 (d, *J*=1.8 Hz, 2 H), 8.87 (s, 1 H), 9.40 (d, *J*=8.0 Hz, 1 H), 9.46 (d, *J*=8.4 Hz, 1 H). MALDITOF-MS: *m/z:* calcd for $C_{43}H_{25}N_5I_2$ 865.0, found 849.9[M - $CH_3$]$^+$.

Synthesis Example 41

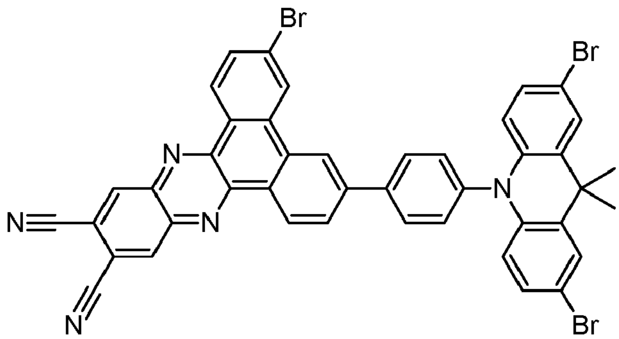

g13: A product was obtained in the same manner as in Synthesis Example 29 except that e6 (0.69 g, 1 mmol) was used instead of Da-CNBPz-H. After the purification, an orange powder was obtained (yield 91%). $^1$H NMR (500 MHz, $CDCl_3$-*d*): δ ppm 1.70 (s, 6H), 6.28 (d, *J*=8.85 Hz, 2 H), 7.12 (dd, J=8.85, 1.98 Hz, 2 H), 7.52 (d, *J*=8.09 Hz, 2 H), 7.56 (d, *J*=1.98 Hz, 2 H), 7.95 (d, *J*=8.70 Hz, 1 H), 8.10 (m, *J*=8.09 Hz, 2 H), 8.14 (d, *J*=8.39 Hz, 1 H), 8.76 (s, 1 H), 8.81 - 8.83 (m, 3 H), 9.24 (d, *J*=8.55 Hz, 1 H), 9.46 (d, *J*=8.24 Hz, 1 H). MALDITOF-MS: *m/z:* calcd for $C_{43}H_{24}N_5Br_3$ 849.0, found 833.9 [*M* - $CH_3$]$^+$.

Synthesis Example 42

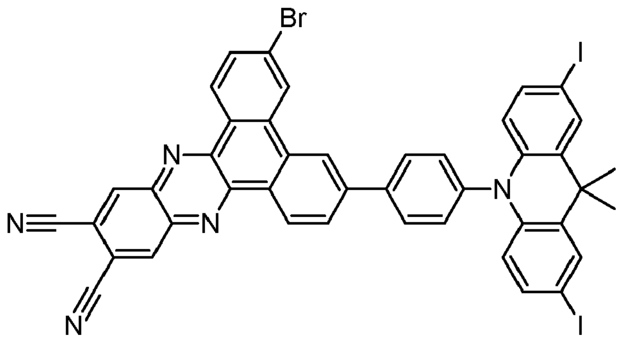

g14: A product was obtained in the same manner as in Synthesis Example 29 except that *N*-iodosuccinimide (0.47 g, 2.1 mmol) was used instead of *N*-bromosuccinimide, and e6 (0.69 g, 1 mmol) was used instead of Da-CNBPz-H. After the purification, an orange powder was obtained (yield 91%). $^{1}$H NMR (500 MHz, $CDCl_3$-*d*): δ ppm 1.70 (s, 6H), 6.27 (d, *J*=8.75 Hz, 2 H), 7.10 (dd, *J*=8.75, 1.90 Hz, 2 H), 7.55 (d, *J*=8.07 Hz, 2 H), 7.56 (d, *J*=1.98 Hz, 2 H), 7.95 (d, *J*=8.70 Hz, 1 H), 8.10 (m, *J*=8.09 Hz, 2 H), 8.14 (d, *J*=8.39 Hz, 1 H), 8.76 (s, 1 H), 8.80 - 8.83 (m, 3 H), 9.24 (d, *J*=8.55 Hz, 1 H), 9.46 (d, *J*=8.24 Hz, 1 H). MALDITOF-MS: *m*/*z:* calcd for $C_{43}H_{24}N_5BrI_2$ 942.4, found 927.4 [*M* - $CH_3$]$^+$.

Synthesis Example 43

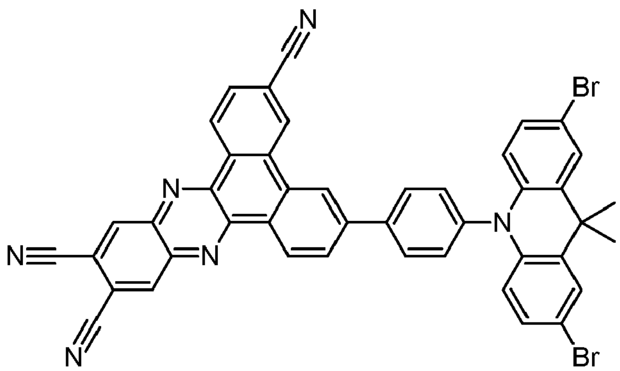

g15: A product was obtained in the same manner as in Synthesis Example 29 except that f2 (0.64 g, 1 mmol) was used instead of Da-CNBPz-H. After the purification, an orange powder was obtained (yield 86%). $^{1}$H NMR (500 MHz, $CDCl_3$-*d*): δ ppm 1.70 (s, 6 H), 6.27 (d, *J*=8.70 Hz, 2 H), 7.12 (dd, *J*=8.77, 2.06 Hz, 2 H), 7.53 (d, *J*=8.24 Hz, 2 H), 7.56 (d, *J*=2.14 Hz, 2 H), 8.07 (d, *J*=8.39 Hz, 1 H), 8.11 (d, *J*=8.24 Hz, 2 H), 8.21 (d, *J*=8.09 Hz, 1 H), 8.83 (s, 1 H), 8.88 (d, *J*=6.26 Hz, 2 H), 9.03 (s, 1 H), 9.52 (t, *J*=8.16 Hz, 2 H). MALDITOF-MS: *m*/*z:* calcd for $C_{44}H_{24}N_6Br_2$ 796.1, found 781.1 *[M*- $CH_3$]$^+$.

Synthesis Example 44

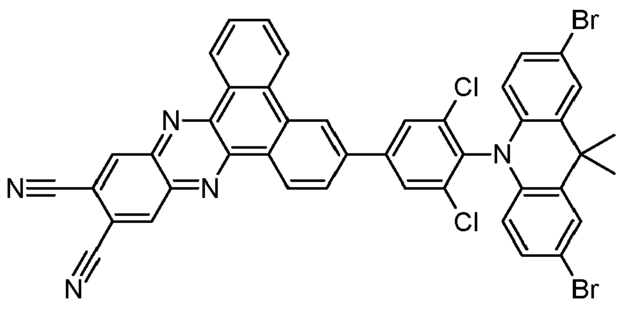

g16: A product was obtained in the same manner as in Synthesis Example 29 except that e3 (0.67 g, 1 mmol) was used instead of Da-CNBPz-H. After the purification, a yellow powder was obtained (yield 81%). $^{1}$H NMR (500 MHz,

$CDCl_3$-*d*): 1.74 (s, 6 H), 6.09 (d, *J*=8.70 Hz, 2 H), 7.16 (dd, *J*=8.77, 2.21 Hz, 2 H), 7.60 (d, *J*=2.14 Hz, 2 H), 7.84 - 7.89 (m, 1 H), 7.94 - 8.00 (m, 1 H), 8.03 (s, 2 H), 8.06 (d, *J*=6.87 Hz, 1 H), 8.72 (d, *J*=8.70 Hz, 1 H), 8.80 - 8.86 (m, 3 H), 9.42 (d, *J*=8.39 Hz, 1 H), 9.50 (d, *J*=8.39 Hz, 1 H). MALDITOF-MS: *m/z:* calcd for $C_{43}H_{23}N_5Br_2Cl_2$ 839.0, found 824.0 [*M*- $CH_3$]$^+$.

Synthesis Example 45

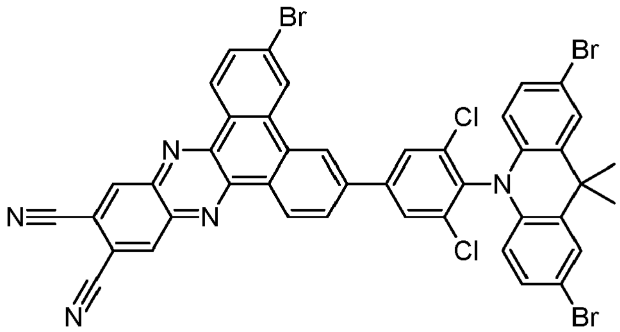

g17: A product was obtained in the same manner as in Synthesis Example 29 except that e8 (0.76 g, 1 mmol) was used instead of Da-CNBPz-H. After the purification, an orange powder was obtained (yield 80%). $^1$H NMR (500 MHz, $CDCl_3$-*d*): δ ppm 1.74 (s, 6 H), 6.06 - 6.17 (d, *J*=8.70 Hz 2 H), 7.16 (d, *J*=8.70 Hz, 2 H), 7.60 (s, 2 H), 7.97 (d, *J*=8.24 Hz, 1 H), 8.02 (s, 2 H), 8.09 (d, *J*=8.85 Hz, 1 H), 8.62 (br. s., 1 H), 8.71 (s, 1 H), 8.78 (s, 2 H), 9.21 (d, *J*=7.63 Hz, 1 H), 9.26 (d, *J*=8.09 Hz, 1 H). MALDITOF-MS: *m/z:* calcd for $C_{43}H_{22}N_5Br_3Cl_2$ 917.0, found 901.9 [*M* - $CH_3$]$^+$.

Compounds Da-CNBPz, Da-CNBPz-H, Ac-CNBPz were synthesized according to the US2019330162.

Example 3 - Examples of Mixtures according to the invention

In the Mixture Examples 1-7 below, the representative organic photoluminescent elements were prepared as films from mixtures of an emitter with a host material - 4,4'-bis(*N*-carbazolyl)-1,1'-biphenyl (CBP) with or without additive of fluorophore dopant. Any other host material can be used for preparation of photoluminescent elements. In the examples below, films were prepared using a spin coating method, and other methods like vacuum deposition, drop casting, etc. can be used.

Mixture Example 1. According to the present invention the light emitting elements containing compounds under Chemical formula I such as e1-e5, f1, and g1-g10 shown in table 1 and Synthesis Examples 18-22, 27, 29-38 outreach the compounds like Da-CNBPz and Da-CNBPz-H described in US2019330162, as well as compounds under Chemical formula II such as e6-9, f2, and g11-17 shown in table 1 and Synthesis Examples 23-26, 28, 39-45 outreach the compounds like Ac-CNBPz described in US2019330162 in the rate of convertion of triplet excitons to siglet ones described by $k_{rISC}$, and the rate of delayed fluorescence described by $\tau_{DF}$, namely in shorter $\tau_{DF}$. The particular compound according to the invention were prepared according to the synthesis example. The film of mixture containing 10% (*w/w*) of e1 and 90 % (w/w) of 4,4'-bis(*N*-carbazolyl)-1,1-biphenyl (CBP) was obtained in such a way that 100 μL of the $CHCl_3$ solution containing 0.1 mg of e1 and 0.9 mg of CBP was poured on a quartz glass at spinning velocity of 50 rotations per second using a spin coater. The films of mixtures containing 10% (w/w) of compounds e2-9, f1-2, g1-17, Da-CNBPz, Da-CNBPz-H, Ac-CNBPz and 90 % (w/w) of CBP were prepared in the same manner as described for e1.

Mixture Example 2. According to the present invention the light emitting elements containing compounds under Chemical formula I and II such as e1-9, f1-2, g1-17 and BPPC type compounds outreach the compounds Px-CNBQx, Ds-CNBQx, Ac-CNBQx, Cz-CNBQx, Px-CNBPz, Da-CNBPz, Ac-CNBPz and others described in US2019330162 in their portion of the light emitted in NIR region and color purity described by the photoluminescence intensity maximum $PL_{max}$ and a full-width at half maximum FWHM and in the rate of delayed fluorescence described by $\tau_{DF}$, namely in shorter $\tau_{DF}$. The film of mixture containing 6% (w/w) of e1, 0.5% (w/w) of BPPC, and 93.5% (w/w) of CBP was obtained in such a way that 100 μL of the $CHCl_3$ solution containing 0.06 mg of e1, 0.005 mg of BPPC, and 0.935 mg of CBP was poured on a quartz glass at spinning velocity of 50 rotations per second using a spin coater. The films of mixtures containing 6% (w/w) of compound g2 or g5 and 0.5% (*w/w*) of BPPC, and 93.5% (*w/w*) of CBP were prepared in the same manner as described for e1.

Mixture Example 3. The film of mixture containing 6% (*w/w*) of e2, 0.75% (*w/w*) of BPPC, and 93.25% (*w/w*) of CBP was obtained in such a way that 100 μL of the $CHCl_3$ solution containing 0.06 mg of e2, 0.0075 mg of BPPC, and 0.9325 mg of CBP was poured on a quartz glass at spinning velocity of 50 rotations per second using a spin coater. The

films of mixtures containing 6% (*w/w*) of compound e6, e8, or g13 and 0.75% (*w/w*) of BPPC, and 93.25% (*w/w*) of CBP were prepared in the same manner as described for e2.

Mixture Example 4. The film of mixture containing 6% (*w/w*) of e3, 1% (*w/w*) of BPPC, and 93% (*w/w*) of CBP was obtained in such a way that 100 μL of the $CHCl_3$ solution containing 0.06 mg of e1, 0.01 mg of BPPC, and 0.93 mg of CBP was poured on a quartz glass at spinning velocity of 50 rotations per second using a spin coater. The films of mixture containing 6% (*w/w*) of compound e7 and 1% (*w/w*) of BPPC, and 93% (*w/w*) of CBP were prepared in the same manner as described for e3.

Mixture Example 5. The film of mixture containing 8% (*w/w*) of f2, 1% (*w/w*) of BPPC, and 91% (*w/w*) of CBP was obtained in such a way that 100 μL of the $CHCl_3$ solution containing 0.08 mg of f2, 0.01 mg of BPPC, and 0.91 mg of CBP was poured on a quartz glass at spinning velocity of 50 rotations per second using a spin coater. The film of mixture containing 8% (*w/w*) of compound g7 and 1% (*w/w*) of BPPC, and 91% (*w*/w) of CBP were prepared in the same manner as described for f2.

Mixture Example 6. The film of mixture containing 8% (*w/w*) of g11, 0.25% (w/w) of BPPC, and 91.75% (*w/w*) of CBP was obtained in such a way that 100 μL of the $CHCl_3$ solution containing 0.08 mg of f2, 0.0025 mg of BPPC, and 0.9175 mg of CBP was poured on a quartz glass at spinning velocity of 50 rotations per second using a spin coater.

Mixture Example 7. According to the present invention the light emitting elements containing compounds under Chemical formula I and II such as e1-9, f1-2, g1-17 and BPPC type compounds prepared as described in the Mixture Examples 2-6 outreach those containing the TPA-DCPP compound and BPPC type compounds described in J. Brodeur, L. Hu, A. Malinge, E. Eizner, W. G. Skene, S. Kéna-Cohen. Highly Efficient and Spectrally Narrow Near-Infrared Fluorescent OLEDs Using a TADF-Sensitized Cyanine Dye. Adv. Opt. Mater. 2019, 7, 1901144.in the rate of delayed fluorescence in NIR region described by $\tau_{DF}$, namely in shorter $\tau_{DF}$. The film of a mixture such as 10% (*w/w*) of compound TPA-DCPP, 0.75% (*w/w*) of BPPC, and 89.25% (*w/w*) of CBP was obtained in such a way that 100 μL of the $CHCl_3$ solution containing 0.1 mg of TPA-DCPP, 0.0075 mg of BPPC, and 0.8925 mg of CBP was poured on a quartz glass at spinning velocity of 50 rotations per second using a spin coater.

Structure of TPA-DCPP.

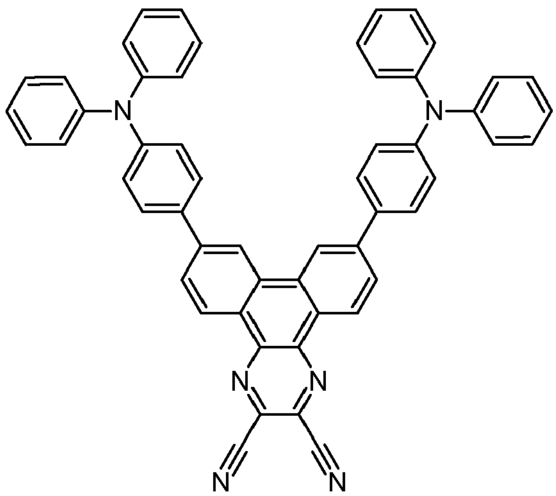

Example 4 - Photoluminescence (PL) examples.

According to the present invention, the compounds under Chemical formula I and II can be used as light emitting materials. These light emitting materials can be used to make a light emitting element. The compounds under Chemical formula I and II have a function of light emission when used solely or when mixed with other materials including host material.

Experimental study provided effect of the invention

PL example 1. The PL spectrum of a film prepared as described in the Mixture Examples 1-7 was measured using Varian Cary Eclipse spectrofluorometer in a front-face excitation geometry at the excitation wavelength 330 nm. Absolute PL quantum yield (PLQY) was measured using the integrating sphere (Quantaurus C11347-11, Hamamatsu).

The PL decay measurements were performed at the excitation wavelength 330 nm using a customized system consisting of a pulsed YAG:Nd laser (PL2251A, EKSPLA) coupled with the optical parametric generator (PG 401/SH) as

excitation light source and 2501S grating spectrometer (Bruker Optics) combined with the streak camera system (C4334-01 Hamamatsu) as a detection unit. The system was equipped with a double-stage high vacuum pump (T-Station 85 Edwards).

The rates and yields of photophysical processes were obtained according to the equations below.

PL decays were fitted with the multi exponential equation:

$$I(t) = A_0 + \sum_{i=1}^{n} A_i \exp\left(-{}^{t}/\tau_{\,i}\right)$$

where $A_i$ is the pre-exponential factor, $\tau_i$ is the decay time and $I(t)$ is emission intensity. Average lifetimes of prompt ($\tau_{PF}$) and delayed fluorescence ($\tau_{DF}$) were determined using the following formula:

$$\tau_{\,PF},\ \tau_{\,DF} = \sum_{i=1}^{n} f_i\,\tau_{\,i},$$

where $f_i$ is fractional contribution of i-th component expressed as:

$$f_i = \frac{A_i\,\tau_{\,i}}{\sum_{i=1}^{n} A_i\,\tau_{\,i}}$$

The ratio of DF and PF quantum yields $\varphi_{DF}/\varphi_{PF}$ was determined as follows:

$$\frac{\varphi_{DF}}{\varphi_{PF}} = \frac{\sum_{i=1}^{n} \tau_{\,DF(i)}\,A_{DF(i)}}{\sum_{j=1}^{n} \tau_{\,PF(j)}\,A_{PF(j)}}$$

where $A_{DF(i)}$ and $A_{PF(j)}$ is the pre-exponential factor of delayed and prompt fluorescence component, respectively; $\tau_{DF(i)}$ and $\tau_{PF(j)}$ is the lifetime of delayed and prompt fluorescence component, respectively. The rate constants of radiative decativation $k_r$ and intersystem crossing $k_{ISC}$ are given by equations:

$$k_r = \frac{\varphi_{PF}}{\tau_{\,PF}}, \qquad \text{(S5)}$$

$$k_{ISC} = \frac{\varphi_{DF}}{\phi\ \tau_{\,PF}}, \qquad \text{(S6)}$$

$$k_{nr} = \frac{1}{\tau_{\,PF}} - (k_r + k_{ISC}). \qquad \text{(S7)}$$

where $\phi$ is PLQY ($\varphi_{DF} + \varphi_{PF}$). Further, the quantum yields for ISC and rISC were calculated as

$$\varphi_{ISC} = k_{ISC}\,\tau_{\,PF}, \qquad \text{(S8)}$$

$$\varphi_{rISC} = \frac{1 - \varphi_{PF}/\phi}{\varphi_{ISC}}. \qquad \text{(S9)}$$

Finally, the rate constant of rISC ($k_{rISC}$) was calculated as

$$k_{rISC} = \frac{\varphi_{rISC}}{\tau_{DF}}\left(\frac{\phi}{\varphi_{PF}}\right). \qquad (S10)$$

In the following PL Examples 2-30 it is demonstrated that the introduction of *electron withdrawing substituent preferably containing heavy atoms* causes the improvement of luminescent properties. The obtained results as well as comparison with the results obtained for Da-CNBPz, Da-CNBPz-H, Ac-CNBPz, and TPA-DCPP under the same conditions are gathered in Figures 1-28 demonstrating PL spectra, Figures 29-56 demonstrating PL decays, and Table 2 demonstrating photophysical parameters of films of mixtures containing 10% (*w*/*w*) of emitters and 90% (*w*/*w*) of CBP. The appearance of the nanosecond prompt fluorescence with the lifetime $\tau_{PF}$ and microsecond delayed fluorescence with the lifetime $\tau_{DF}$ confirms that all emitters e1-9, f1-2, and g1-17 exhibit thermally activated delayed fluorescence TADF. Using the enhancement of $k_{rISC}$ and shortening of $\tau_{DF}$ as comparison criteria, the key TADF parameters of compounds e1-5, f1, g1-10 outreach those of like Da-CNBPz and Da-CNBPz-H, and e6-9, f2, g11-17 outreach those of Ac-CNBPz. This confirms that the compounds under Chemical formula I and II have improved properties as light emitting materials and can have industrial application potential in red or NIR light emitting elements.

The examples of the key effect *of an electron withdrawing substituent with a positive Hammett constant at the quinoxaline fragment* are described below.

PL Example 2. As compared to that containing Da-CNBPz-H, the light emitting element prepared according to the Mixture Example 1 containing a derivative f1 bearing CN group as $R^a$ at position 3 shows emission 11 nm shifted to NIR region, 2.9 times shorter DF lifetime, and 2.9 times higher rISC rate constant (Figures 10 and 38, Table 2).

PL Example 3. As compared to that containing Ac-CNBPz, the light emitting element prepared according to the Mixture Example 1 containing f2 bearing CN group as $R^a$ at position 3 shows emission 30 nm shifted to NIR region, 11.8 times shorter DF lifetime, and 7.1 times higher rISC rate constant (Figures 11 and 39, Table 2).

PL Example 4. As compared to that containing g11, the light emitting element prepared according to the Mixture Example 1 containing g13 bearing CN group as $R^a$ at position 3 shows emission 46 nm shifted to NIR region, 5.1 times shorter DF lifetime, and 2.4 times higher rISC rate constant (Table 2).

PL examples 1-4 indicate that compounds given by Chemical Formula I and II with electron withdrawing substituent with a positive Hammett constant at the quinoxaline fragment emit more light in NIR region and can harvest triplets faster and in shorter time, what is required for efficient NIR electroluminescence applications.

The examples of the key effect of a *heavy atom at the quinoxaline fragment* are described below.

PL Example 5. As compared to that containing Da-CNBPz-H, the light emitting element prepared according to the Mixture Example 1 containing e1 bearing heavy bromine atom as $R^a$ at position 3 shows emission 41 nm shifted to NIR region, 7.7 times shorter DF lifetime, and 12.5 times higher rISC rate constant (Figures 1 and 29, Table 2).

PL Example 6. As compared to that containing Ac-CNBPz, the light emitting element prepared according to the Mixture Example 1 containing e6 bearing heavy bromine atom as $R^a$ at position 3 shows emission 55 nm shifted to NIR region, 15.1 times shorter DF lifetime, and 17.6 times higher rISC rate constant (Figures 6 and 34, Table 2).

PL Example 7. As compared to that containing Ac-CNBPz, the light emitting element prepared according to the Mixture Example 1 containing e9 bearing heavy bromine atom as $R^a$ at position 2 shows emission 43 nm shifted to NIR region, 20 times shorter DF lifetime, and 39 times higher rISC rate constant (Figures 9 and 37, Table 2).

PL examples 5-7 indicate that compounds given by Chemical Formula I and II with heavy atom at the quinoxaline fragment emit more light in NIR region and can harvest triplets faster and in shorter time, what is required for efficient NIR electroluminescence applications.

The examples of the key effect of a *various heavy atoms at various positions of the triphenylamine fragment* ($R_1$-$R_8$) are described below.

PL Example 8. As compared to that containing Da-CNBPz-H, the light emitting element prepared according to the

Mixture Example 1 containing g3 bearing a heavy bromine atom as $R_5$ shows 11.9 times shorter DF lifetime, and 15.5 times higher rISC rate constant (Figures 14 and 42, Table 2).

PL Example 9. As compared to that containing Da-CNBPz-H, the light emitting element prepared according to the Mixture Example 1 containing g1 bearing two heavy bromine atoms as $R_5$ and $R_6$ shows 3.2 times shorter DF lifetime, and 8.5 times higher rISC rate constant (Figures 12 and 40, Table 2).

PL Example 10. As compared to that containing Da-CNBPz-H, the light emitting element prepared according to the Mixture Example 1 containing g4 bearing a heavy iodine atom as $R_5$ shows 7.9 times shorter DF lifetime, and 10.5 times higher rISC rate constant (Figures 15 and 43, Table 2).

PL Example 11. As compared to that containing Da-CNBPz-H, the light emitting element prepared according to the Mixture Example 1 containing g2 bearing two heavy iodine atoms as $R_5$ and $R_6$ shows 2 times shorter DF lifetime, and 11 times higher rISC rate constant (Figures 13 and 41, Table 2).

PL Example 12. As compared to that containing Da-CNBPz-H, the light emitting element prepared according to the Mixture Example 1 containing g8 bearing three heavy bromine atoms as $R_5$, $R_6$, and $R_7$ at position 1' shows 3.5 times shorter DF lifetime, and 13.5 times higher rISC rate constant (Figures 19 and 47, Table 2).

PL Example 13. As compared to that containing Da-CNBPz, the light emitting element prepared according to the Mixture Example 1 containing e2 bearing a heavy chlorine atom as $R_1$ shows 18.5 times shorter DF lifetime, and 30 times higher rISC rate constant (Figures 2 and 30, Table 2).

PL Example 14. As compared to that containing Da-CNBPz, the light emitting element prepared according to the Mixture Example 1 containing e3 bearing two heavy chlorine atoms as $R_1$ and $R_2$ shows 43 times shorter DF lifetime, and 93 times higher rISC rate constant (Figures 3 and 31, Table 2).

PL Example 15. As compared to that containing Da-CNBPz, the light emitting element prepared according to the Mixture Example 1 containing g9 bearing a heavy chlorine atom as $R_1$ and a heavy bromine atom as $R_7$ at position 1' shows 5.4 times shorter DF lifetime, and 14 times higher rISC rate constant (Figures 20 and 48, Table 2).

PL Example 16. As compared to that containing Da-CNBPz, the light emitting element prepared according to the Mixture Example 1 containing g10 bearing two heavy chlorine atoms as $R_1$, $R_2$, and a heavy bromine atom as $R_7$ at position 1' shows 13.5 times shorter DF lifetime, and 47 times higher rISC rate constant (Figures 21 and 49, Table 2).

PL Example 17. As compared to that containing Ac-CNBPz, the light emitting element prepared according to the Mixture Example 1 containing g11 bearing two heavy bromine atoms as $R_5$ and $R_6$ shows 1.2 times shorter DF lifetime, and 2.6 times higher rISC rate constant (Figures 22 and 50, Table 2).

PL Example 18. As compared to that containing Ac-CNBPz, the light emitting element prepared according to the Mixture Example 1 containing g12 bearing two heavy iodine atoms as $R_5$ and $R_6$ shows 1.9 times shorter DF lifetime, and 3.6 times higher rISC rate constant (Figures 23 and 51, Table 2).

PL Example 19. As compared to that containing f2, the light emitting element prepared according to the Mixture Example 1 containing g15 bearing two heavy bromine atoms as $R_5$ and $R_6$ shows 1.4 times shorter DF lifetime, and 2.3 times higher rISC rate constant (Table 2).

PL Example 20. As compared to that containing Da-CNBPz, the light emitting element prepared according to the Mixture Example 1 containing e7 bearing two heavy chlorine atoms as $R_1$ and $R_2$ shows 8.2 times shorter DF lifetime, and 6.6 times higher rISC rate constant (Figures 7 and 35, Table 2).

PL Example 21. As compared to that containing Da-CNBPz, the light emitting element prepared according to the Mixture Example 1 containing g16 bearing two heavy chlorine atoms as $R_1$ and $R_2$, and two heavy bromine atoms as $R_5$ and $R_6$ shows 1.8 times shorter DF lifetime, and 2 times higher rISC rate constant (Figures 27 and 55, Table 2).

The examples 8-21 show that the compounds given by Chemical Formula I and II with heavy atom at the triphenylamine fragment can harvest triplets faster and in shorter time, what is required for efficient electroluminescence applications.

Excellent triplet harvesting properties are also achieved when the *heavy atoms and/or electron withdrawing substituents are introduced simultaneously at various positions of the triphenylamine fragment and quinoxaline fragment:* PL Example 22. As compared to that containing Da-CNBPz, the light emitting element prepared according to the Mixture Example 1 containing e4 bearing a heavy bromine atom as $R^a$ at position 2, two heavy chlorine atoms as $R_1$ and $R_2$, and $CH_3O$ instead of $CH_3$ groups as $R_5$, $R_6$ shows 5.1 times shorter DF lifetime, and 24 times higher rISC rate constant (Figures 4 and 32, Table 2).

PL Example 23. As compared to that containing Da-CNBPz, the light emitting element prepared according to the Mixture Example 1 containing e5 bearing a heavy bromine atom as $R^a$ at position 3, two heavy chlorine atoms as $R_1$ and $R_2$, and $CH_3O$ instead of $CH_3$ groups as $R_5$, $R_6$ shows 54 times shorter DF lifetime, and 66 times higher rISC rate constant (Figures 5 and 33, Table 2).

PL Example 24. As compared to that containing Da-CNBPz-H, the light emitting element prepared according to the Mixture Example 1 containing g5 bearing three heavy bromine atoms as $R^a$ at position 3, $R_5$, and $R_6$ shows 5.6 times shorter DF lifetime, and 20.5 times higher rISC rate constant (Figures 16 and 44, Table 2).

PL Example 25. As compared to that containing Da-CNBPz-H, the light emitting element prepared according to the Mixture Example 1 containing g6 bearing a heavy bromine atom as $R^a$ at position 3, and two heavy iodine atoms at $R_5$ and $R_6$ shows 5.9 times shorter DF lifetime, and 26.5 times higher rISC rate constant (Figures 17 and 45, Table 2).

PL Example 26. As compared to that containing Da-CNBPz-H, the light emitting element prepared according to the Mixture Example 1 containing g7 bearing CN as $R^a$ at position 3, four heavy bromine atoms as $R_5$, $R_6$, $R_7$ at position 1' and $R_8$ at position 1" shows 14.4 times shorter DF lifetime, and 38 times higher rISC rate constant (Figures 18 and 46, Table 2).

PL Example 27. As compared to that containing Ac-CNBPz, the light emitting element prepared according to the Mixture Example 1 containing e8 bearing a heavy bromine atom as $R^a$ at position 3, and two heavy chlorine atoms as $R_1$ and $R_2$ shows 11.3 times shorter DF lifetime, and 18 times higher rISC rate constant (Figures 8 and 36, Table 2).

PL Example 28. As compared to that containing Ac-CNBPz, the light emitting element prepared according to the Mixture Example 1 containing g13 bearing three heavy bromine atoms as $R^a$ at position 3, $R_5$, and $R_6$ shows 3 times shorter DF lifetime, and 5.3 times higher rISC rate constant (Figures 24 and 52, Table 2).

PL Example 29. As compared to that containing Ac-CNBPz, the light emitting element prepared according to the Mixture Example 1 containing g14 bearing a heavy bromine atom as $R^a$ at position 3, and two heavy iodine atoms at $R_5$ and $R_6$ shows 2.2 times shorter DF lifetime, and 5.3 times higher rISC rate constant (Figures 25 and 53, Table 2).

PL Example 30. As compared to that containing Ac-CNBPz, the light emitting element prepared according to the Mixture Example 1 containing g17 bearing three heavy bromine atoms as $R^a$ at position 3, $R_5$, and $R_6$, and two heavy chlorine atoms as $R_1$ and $R_2$ shows 19.4 times shorter DF lifetime, and 28 times higher rISC rate constant (Figures 28 and 56, Table 2).

Table 2. Photophysical parameters of amorphous films of mixtures of 90% CBP and 10% (*w/w*) of Da-CNBPz, Da-CNBPz-H, Ac-CNBPz and their heavy-atom analogues.

| Cmpd | $PL_{max}$ [nm] | PLQY [%] | $\tau_{PF}$ [ns] | TDF [$\mu$s] | $k_r$ [$10^7$ $s^{-1}$] | $k_{ISC}$ [$10^7$ $s^{-1}$] | $k_{rISC}$ [$10^4$ $s^{-1}$] |
|---|---|---|---|---|---|---|---|
| Da-CNBPz | 692 | 83 | 16.7 | 646 | 3.2 | 2.1 | 0.2 |
| Da-CNBPz-H | 639 | 96 | 18.8 | 633 | 3.56 | 1.6 | 0.2 |
| Examples of Compounds given by Chemical formula I | | | | | | | |
| e1 | 680 | 65 | 9.6 | 82 | 3.3 | 5 | 2.5 |
| e2 | 662 | 60 | 32.5 | 35 | 0.9 | 1.6 | 6 |
| e3 | 642 | 36 | 20.3 | 15 | 0.3 | 1.3 | 18.5 |
| e4 | 618 | 43 | 32 | 126 | 0.23 | 2.6 | 4.8 |
| e5 | 680 | 6 | 11.1 | 12 | 0.34 | 3.3 | 13.3 |
| f1 | 650 | 44 | 24.1 | 221 | 1.45 | 0.9 | 0.57 |
| g1 | 618 | 100 | 20.9 | 197 | 1.42 | 3.4 | 1.7 |
| g2 | 622 | 100 | 15 | 317 | 0.98 | 5.7 | 2.2 |
| g3 | 667 | 69 | 18.8 | 53 | 2.26 | 2 | 3.1 |
| g4 | 657 | 63 | 23.2 | 80 | 1.64 | 1.7 | 2.1 |
| g5 | 655 | 66 | 9.8 | 113 | 1.45 | 8 | 4.1 |
| g6 | 655 | 65 | 5.5 | 107 | 2.09 | 15 | 5.3 |

(continued)

| Examples of Compounds given by Chemical formula I | | | | | | | |
|---|---|---|---|---|---|---|---|
| g7 | 612 | 68 | 105 | 44 | 0.12 | 0.7 | 7.6 |
| g8 | 609 | 29 | 47.7 | 181 | 0.13 | 1.7 | 2.7 |
| g9 | 610 | 42 | 37.4 | 120 | 0.26 | 2.1 | 2.8 |
| g10 | 611 | 44 | 52.8 | 48 | 0.18 | 1.5 | 9.4 |
| | | | | | | | |
| Ac-CNBPz | 590 | 71 | 39.4 | 272 | 0.39 | 2 | 1.7 |
| Examples of Compounds given by Chemical formula II | | | | | | | |
| e6 | 645 | 43 | 50.4 | 18 | 0.16 | 1.6 | 30 |
| e7 | 620 | 44 | 106 | 33 | 0.11 | 0.7 | 11.3 |
| e8 | 621 | 50 | 42.2 | 24 | 0.17 | 2 | 30.6 |
| e9 | 633 | 33 | 29.6 | 13.7 | 0.12 | 3 | 67 |
| f2 | 620 | 80 | 80.4 | 23 | 0.36 | 0.8 | 12 |
| g11 | 578 | 97 | 59.7 | 218 | 0.16 | 1.5 | 4.5 |
| g12 | 581 | 54 | 33.3 | 141 | 0.18 | 2.7 | 6.2 |
| g13 | 609 | 59 | 28.7 | 92 | 0.24 | 3.1 | 9 |
| g14 | 605 | 59 | 27.6 | 122 | 0.19 | 3.3 | 9 |
| g15 | 624 | 38 | 39.9 | 16.8 | 0.2 | 2 | 28 |
| g16 | 590 | 20 | 67.4 | 148 | 0.06 | 1.2 | 3.4 |
| g17 | 624 | 35 | 33 | 14 | 0.16 | 2.6 | 47 |

Example 5 - Hyperfluorescence (HF) Examples.

According to the present invention, the compounds under Chemical formula I and II have a function of assistant for the light emission when mixed with other light emitting materials. The following HF Examples 1-12 show the application of compounds in light emitting elements with near infrared emission prepared according to the Mixture Example 2-6. Films containing compounds given by the Chemical Foluma I and/or II, such as e1-e3, e6-e8, f2, g2, g5, g7, g11, g13 with mass concentration of 6-10% (w/w) and a NIR emitting dopant like BPPC with mass concentration of 0.25-1% in CBP were subjected to stationary and time-resolved photoluminescence measurements under the same conditions as described in PL Example 1. The obtained results are shown in Figures 57-66, and Table 3. The color purity is described by the FWHM parameter, superiority of the obtained light emitting elements is shown by comparison with the compounds Px-CNBQx (FWHM = 125 nm), Da-CNBQx (FWHM = 109 nm), Ac-CNBQx (FWHM = 106 nm), Cz-CNBQx (FWHM = 97 nm), Px-CNBPz (FWHM = 158 nm), Da-CNBPz (FWHM = 139 nm), Ac-CNBPz (FWHM = 132 nm) as described in US2019330162. Using $PL_{max}$ and FWHM as comparison criteria, it is concluded that all the light emitting elements containing Chemical formula I and/or II and BPPC show much larger portion of emission in NIR region and higher color purity (FWHM = 45 nm) as compared to emitters reported in US2019330162.

The presence of a microsecond delayed fluorescence with the lifetime $\tau_{DF}$ confirms that the light emitting elements containing Chemical formula I and/or II and BPPC take advantage of thermally activated delayed fluorescence. Using the shortening of $\tau_{DF}$ as a comparison criteria, it is concluded that the light emitting elements outreach those containing compound TPA-DCPP reported in J. Brodeur, L. Hu, A. Malinge, E. Eizner, W. G. Skene, S. Kéna-Cohen. Highly Efficient and Spectrally Narrow Near-infrared Fluorescent OLEDs Using a TADF-Sensitized Cyanine Dye. Adv. Opt. Mater. 2019, 7, 1901144. This confirms that the compounds under Chemical formula I and II have improved properties as assistant for the light emission and have industrial application potential as components of light emitting elements for high color purity NIR emission. The rate of triplet harvesting of the prepared light emitting elements is described by the $\tau_{DF}$ and compared to the light emitting element containing TPA-DCPP and BPPC prepared as described in Mixture Example 7. HF Example 1. The light emitting element containing 6% (w/w) e1 and 0.5 % (*w/w*) of BPPC prepared as described in Mixture Example 2 exhibit emission in the NIR region with the intensity maximum $PL_{max}$ at 795 nm, FWHM of 45 nm, and the 14.2 times lower $\tau_{DF}$ as compared to the light emitting element containing TPA-DCPP and BPPC (Figure 58 and Table

3).

HF Example 2. The light emitting element containing 6% (w/w) e2 and 0.75 % (w/w) of BPPC prepared as described in Mixture Example 3 exhibit emission in the NIR region with the intensity maximum $PL_{max}$ at 795 nm, FWHM of 45 nm, and the 11.3 times lower $\tau_{DF}$ as compared to the light emitting element containing TPA-DCPP and BPPC (Figure 59 and Table 3).

HF Example 3. The light emitting element containing 6% (w/w) e3 and 1% (w/w) of BPPC prepared as described in Mixture Example 4 exhibit emission in the NIR region with the intensity maximum $PL_{max}$ at 795 nm, FWHM of 45 nm, and the 17.2 times lower $\tau_{DF}$ as compared to the light emitting element containing TPA-DCPP and BPPC (Figure 60 and Table 3).

HF Example 4. The light emitting element containing 6 % (w/w) e6 and 0.75 % (w/w) of BPPC prepared as described in Mixture Example 3 exhibit emission in the NIR region with the intensity maximum $PL_{max}$ at 795 nm, FWHM of 45 nm, and the 19.6 times lower $\tau_{DF}$ as compared to the light emitting element containing TPA-DCPP and BPPC (Figures 57 and 61, Table 3).

HF Example 5. The light emitting element containing 6% (w/w) e7 and 1% (w/w) of BPPC prepared as described in Mixture Example 4 exhibit emission in the NIR region with the intensity maximum $PL_{max}$ at 795 nm, FWHM of 45 nm, and the 15 times lower $\tau_{DF}$ as compared to the light emitting element containing TPA-DCPP and BPPC (Table 3).

HF Example 6. The light emitting element containing 6 % (w/w) e8 and 0.75 % (w/w) of BPPC prepared as described in Mixture Example 3 exhibit emission in the NIR region with the intensity maximum $PL_{max}$ at 795 nm, FWHM of 45 nm, and the 12.3 times lower $\tau_{DF}$ as compared to the light emitting element containing TPA-DCPP and BPPC (Table 3).

HF Example 7. The light emitting element containing 8 % (w/w) f2 and 1 % (w/w) of BPPC prepared as described in Mixture Example 5 exhibit emission in the NIR region with the intensity maximum $PL_{max}$ at 795 nm, FWHM of 45 nm, and the 9.2 times lower $\tau_{DF}$ as compared to the light emitting element containing TPA-DCPP and BPPC (Figure 62 and Table 3).

HF Example 8. The light emitting element containing 6 % (w/w) g2 and 0.5 % (w/w) of BPPC prepared as described in Mixture Example 2 exhibit emission in the NIR region with the intensity maximum $PL_{max}$ at 795 nm, FWHM of 45 nm, and the 9 times lower $\tau_{DF}$ as compared to the light emitting element containing TPA-DCPP and BPPC (Figure 63 and Table 3).

HF Example 9. The light emitting element containing 6 % (w/w) g5 and 0.5 % (w/w) of BPPC prepared as described in Mixture Example 2 exhibit emission in the NIR region with the intensity maximum $PL_{max}$ at 795 nm, FWHM of 45 nm, and the 20 times lower $\tau_{DF}$ as compared to the light emitting element containing TPA-DCPP and BPPC (Figure 64 and Table 3).

HF Example 10. The light emitting element containing 8 % (w/w) g7 and 1 % (w/w) of BPPC prepared as described in Mixture Example 5 exhibit emission in the NIR region with the intensity maximum $PL_{max}$ at 795 nm, FWHM of 45 nm, and the 22.4 times lower $\tau_{DF}$ as compared to the light emitting element containing TPA-DCPP and BPPC (Figure 65 and Table 3).

HF Example 11. The light emitting element containing 8 % (w/w) g11 and 0.25 % (*w/w*) of BPPC prepared as described in Mixture Example 6 exhibit emission in the NIR region with the intensity maximum $PL_{max}$ at 795 nm, FWHM of 45 nm, and the 15.9 times lower $\tau_{DF}$ as compared to the light emitting element containing TPA-DCPP and BPPC (Table 3).

HF Example 12. The light emitting element containing 6 % (w/w) g13 and 0.75 % (*w/w*) of BPPC prepared as described in Mixture Example 3 exhibit emission in the NIR region with the intensity maximum $PL_{max}$ at 795 nm, FWHM of 45 nm, and the 8.3 times lower $\tau_{DF}$ as compared to the light emitting element containing TPA-DCPP and BPPC (Table 3).

Table 3. Photophysical parameters of amorphous films of mixtures of 0.75% BPPC, 89.25% CBP and 10% (w/w) of TPA-DCPP, Da-CNBPz, Ac-CNBPz or their heavy-atom analogues.

| TADF emitter | mas conc. of compound given by Chemical Formula I or II (%) | BPPC mas conc. (%) | $PL_{max}$ [nm] | FWHM [nm] | PLQY [%] | $\tau_{PF}$ [ns] | TDF [μs] |
|---|---|---|---|---|---|---|---|
| TPA-DCPP | 10 | 0.75 | 795 | 45 | 37 | 7.4 | 505 |
| Examples of Compounds given by Chemical formula I and II | | | | | | | |
| e1 | 6 | 0.5 | 795 | 45 | 42 | 5.6 | 35.5 |
| e2 | 6 | 0.75 | 795 | 45 | 34 | 11.9 | 44.8 |
| e3 | 6 | 1 | 795 | 45 | 30 | 16.6 | 29.4 |
| e6 | 6 | 0.75 | 795 | 45 | 38 | 6.2 | 10.2 |
| e7 | 6 | 1 | 795 | 45 | 28 | 12.7 | 13.3 |
| e8 | 6 | 0.75 | 795 | 45 | 33 | 24.5 | 16.3 |
| f2 | 8 | 1 | 795 | 45 | 19 | 4.8 | 21.8 |
| g2 | 6 | 0.5 | 795 | 45 | 37 | 3.9 | 56.2 |

(continued)

| Examples of Compounds given by Chemical formula I and II | | | | | | | |
|---|---|---|---|---|---|---|---|
| g5 | 6 | 0.5 | 795 | 45 | 45 | 3.8 | 25.4 |
| g7 | 8 | 1 | 795 | 45 | 28 | 7.7 | 22.5 |
| g11 | 8 | 0.25 | 795 | 45 | 27.2 | 6.8 | 12.6 |
| g13 | 6 | 0.75 | 795 | 45 | 34 | 8.1 | 24.1 |

## Claims

1. Derivates of quinoxaline with thermally activated delayed fluorescence being substituted by a triarylamine derivative and comprising at least one atom with atomic weight above 25 Da described by formula I:

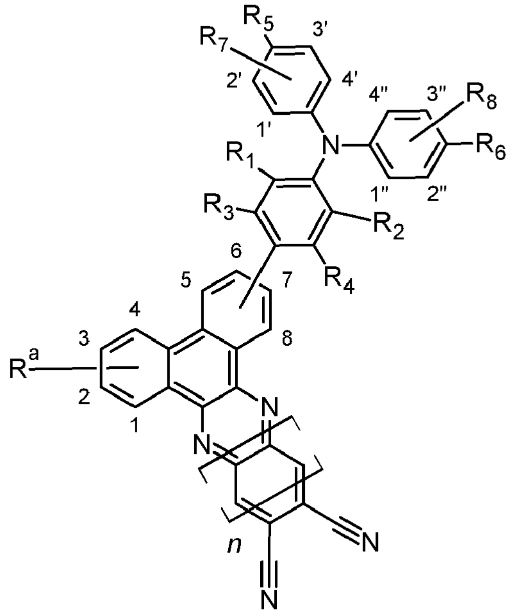

,

while $R^a$ is H or one or several electron withdrawing group(s) with a positive (>0) para and/or meta Hammett sigma constant: halogen or cyano, or nitro, or chalcogen(alkyl), or chalcogen(aryl), or chalcogen(heteroaryl), sulphonyl derivatives, or carboxyl derivative, or carbonyl derivative, or alkyl with electron withdrawing group(s), aryl with electron withdrawing group(s), hetaryl with electron withdrawing group(s);

$R_1$, $R_2$, $R_3$, $R_4$, $R_5$, $R_6$, $R_7$, and $R_8$ is each independently H or halogen, or $CH_3$, or alkyl, or aryl, or heteroaryl, or chalcogen(alkyl), or chalcogen(aryl), or chalcogen(heteroaryl), or N(alkyl)$_2$, or N(aryl)$_2$, or N(alkyl, aryl), or N(heteroaryl)$_2$, or N(alkyl, heteroaryl), or N(aryl, heteroaryl), or P(alkyl)$_2$, or P(aryl)$_2$, or P(alkyl, aryl), or P(heteroaryl)$_2$, or P(alkyl, heteroaryl), or P(aryl, heteroaryl), or P(O)(alkyl)$_2$, or P(O)(aryl)$_2$, or P(O)(alkyl, aryl), or P(O)(heteroaryl)$_2$, or P(O)(alkyl, heteroaryl), or P(O)(aryl, heteroaryl), or Si(alkyl)$_3$, or Si(aryl)$_3$, or Si(heteroaryl)$_3$, or Si(alkyl)$_2$(aryl), or Si(alkyl)(aryl)$_2$, or Si(alkyl)$_2$(hetaryl), or Si(alkyl)(hetaryl)$_2$, or Si(aryl)$_2$(hetaryl), or Si(aryl)(hetaryl)$_2$, or Si(alkyl, aryl, heteroaryl); or in various combinations form aliphatic (poly)cycle(s), or aromatic (poly)cycle(s), or aliphatic (poly)cycle(s) with heteroatom(s), or aromatic (poly)cycle(s) with heteroatom(s);

wherein within $R^a$, $R_1$, $R_2$, $R_3$, $R_4$, $R_5$, $R_6$, $R_7$, and $R_8$ set one or two or three or four or five or six or at least seven or eight R is H simultaneously and at least one of substituents $R^a$, $R_1$, $R_2$, $R_3$, $R_4$, $R_5$, $R_6$, $R_7$, and/or $R_8$ contain at least one atom with atomic weight above 25 Da, and *n* is 0, 1.

2. Derivates of quinoxaline with thermally activated delayed fluorescence being substituted by a triarylamine derivative and containing one or several atom(s) with atomic weight above 25 Da described by formula II

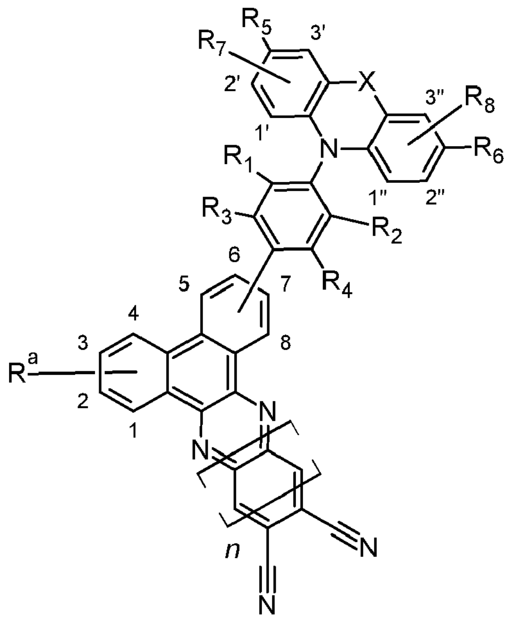

while $R^a$ is H or one or several electron withdrawing group(s) with a positive (>0) para and/or meta Hammett sigma constant: halogen or cyano, or nitro, or chalcogen(alkyl), or chalcogen(aryl), or chalcogen(heteroaryl), sulphonyl derivatives, or carboxyl derivative, or carbonyl derivative, or alkyl with electron withdrawing group(s), aryl with electron withdrawing group(s), hetaryl with electron withdrawing group(s);
X is $C(CH_3)_2$, or $C(alkyl)_2$, or $C(aryl)_2$, or C(alkyl, aryl), or $C(heteroaryl)_2$, or C(alkyl, heteroaryl), or C(aryl, heteroaryl), or a single C-C bond, or C=O, or C=S, or chalcogen, or $Si(alkyl)_2$, or $Si(aryl)_2$, or Si(alkyl, aryl), or $Si(heteroaryl)_2$, or Si(alkyl, heteroaryl), or Si(aryl, heteroaryl), or N(alkyl), or N(aryl), or N(hetaryl), or P(alkyl), or P(aryl), or P(hetaryl), or P(O)(alkyl), or P(O)(aryl), or P(O)(heteroaryl), or P=S, or $SO_2$, or $Ge(alkyl)_2$, or $Ge(aryl)_2$, or Ge(alkyl, aryl), or $Ge(heteroaryl)_2$, or Ge(alkyl, heteroaryl), or Ge(aryl, heteroaryl), or form an aliphatic (poly) cycle, or form an aromatic (poly)cycle, or
form an aliphatic (poly)cycle with heteroatom(s), or form an aromatic (poly)cycle with heteroatom(s);
$R_1$, $R_2$, $R_3$, $R_4$, $R_5$, $R_6$, $R_7$, and $R_8$ is each independently H or halogen, or $CH_3$, or alkyl, or aryl, or heteroaryl, or chalcogen(alkyl), or chalcogen(aryl), or chalcogen(heteroaryl), or $N(alkyl)_2$, or $N(aryl)_2$, or N(alkyl, aryl), or $N(heteroaryl)_2$, or N(alkyl, heteroaryl), or N(aryl, heteroaryl), or $P(alkyl)_2$, or $P(aryl)_2$, or P(alkyl, aryl), or $P(heteroaryl)_2$, or P(alkyl, heteroaryl), or P(aryl, heteroaryl), or $P(O)(alkyl)_2$, or $P(O)(aryl)_2$, or P(O)(alkyl, aryl), or $P(O)(heteroaryl)_2$, or P(O)(alkyl, heteroaryl), or P(O)(aryl, heteroaryl), or $Si(alkyl)_3$, or $Si(aryl)_3$, or $Si(heteroaryl)_3$, or $Si(alkyl)_2(aryl)$, or $Si(alkyl)(aryl)_2$, or $Si(alkyl)_2(hetaryl)$, or $Si(alkyl)(hetaryl)_2$, or $Si(aryl)_2(hetaryl)$, or $Si(aryl)(hetaryl)_2$, or Si(alkyl, aryl, heteroaryl); or in various combinations form aliphatic (poly)cycle(s), or aromatic (poly)cycle(s), or aliphatic (poly)cycle(s) with heteroatom(s), or aromatic (poly)cycle(s) with heteroatom(s), while only one or two or three or four or five or six or seven or eight R as $R^a$, $R_1$, $R_2$, $R_3$, $R_4$, $R_5$, $R_6$, $R_7$, and $R_8$ is H simultaneously and at least one of substituents $R^a$, $R_1$, $R_2$, $R_3$, $R_4$, $R_5$, $R_6$, $R_7$, and/or $R_8$ contain at least one atom with atomic weight above 25 Da, and *n* is 0, 1 or 2.

**3.** Mixtures with thermally activated delayed fluorescence containing compound mixtures of new emitters described according to claim 1 and shown in formula I and/or according to claims 2 and shown in Formula II with at least with one of known fluorescent dopants exhibit narrowband NIR NIR emitting dopant such as pyrrolopyrrole fluorophore, like BPPC, while the mixtures emit NIR light during at least 0.5 microseconds after excitation and the ratio of the compound according to formula I and/or II and a NIR emitting dopant in a mixture vary from 1000/1 to 1/10.

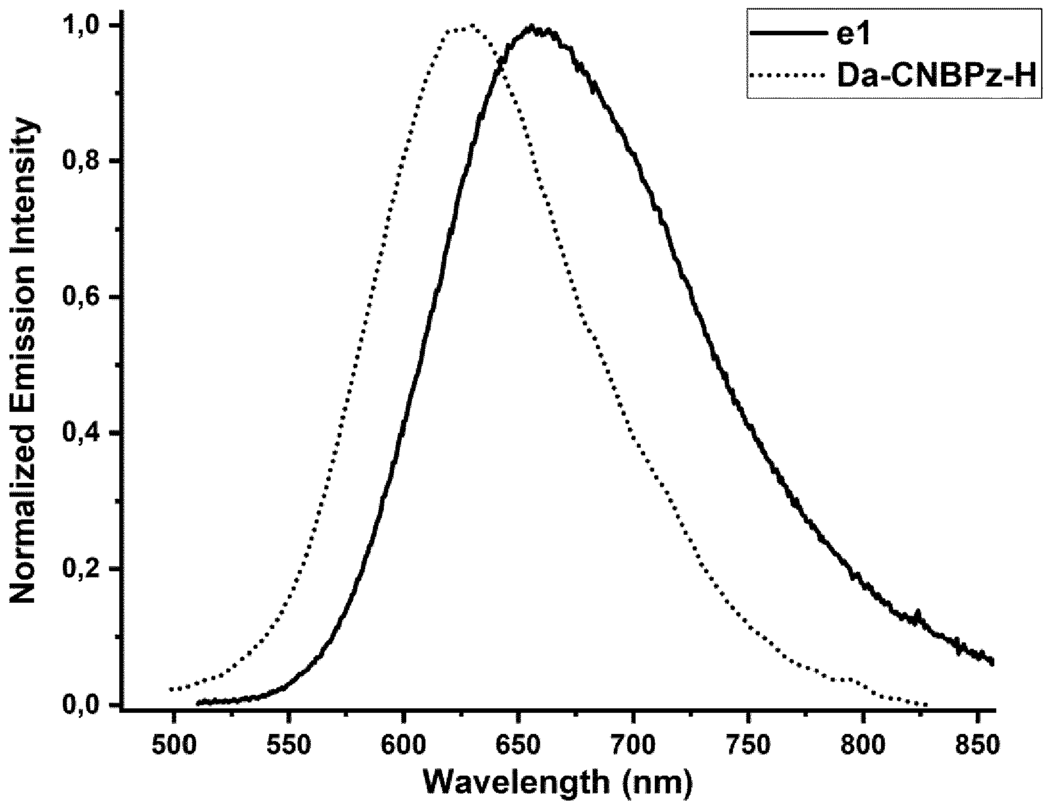


Figure 1. Comparison of PL spectra of Da-CNBPz-H and e1.

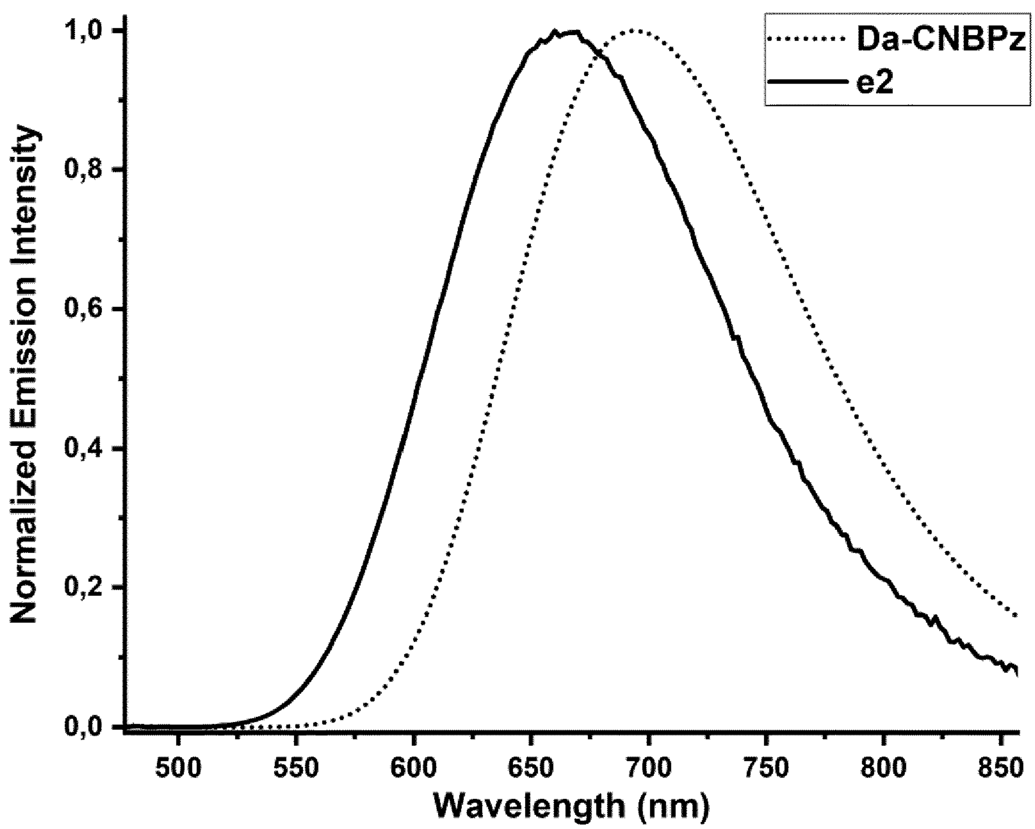

Figure 2. Comparison of PL spectra of Da-CNBPz and e2.

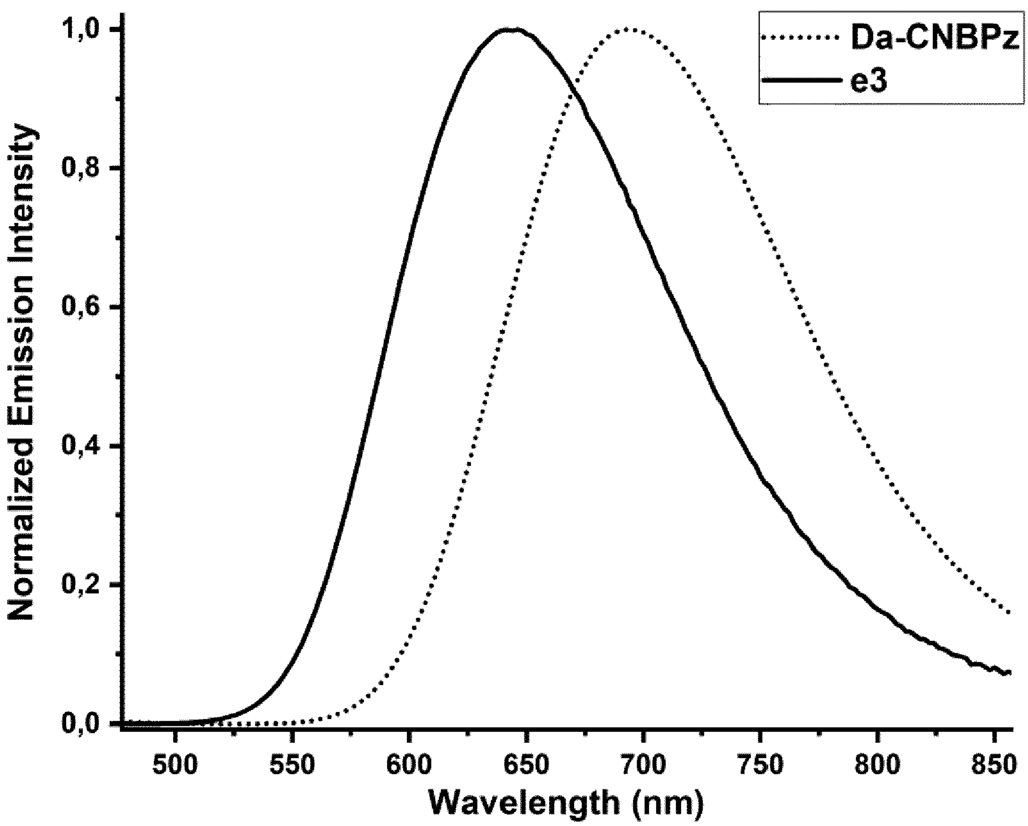


Figure 3. Comparison of PL spectra of Da-CNBPz and e3.

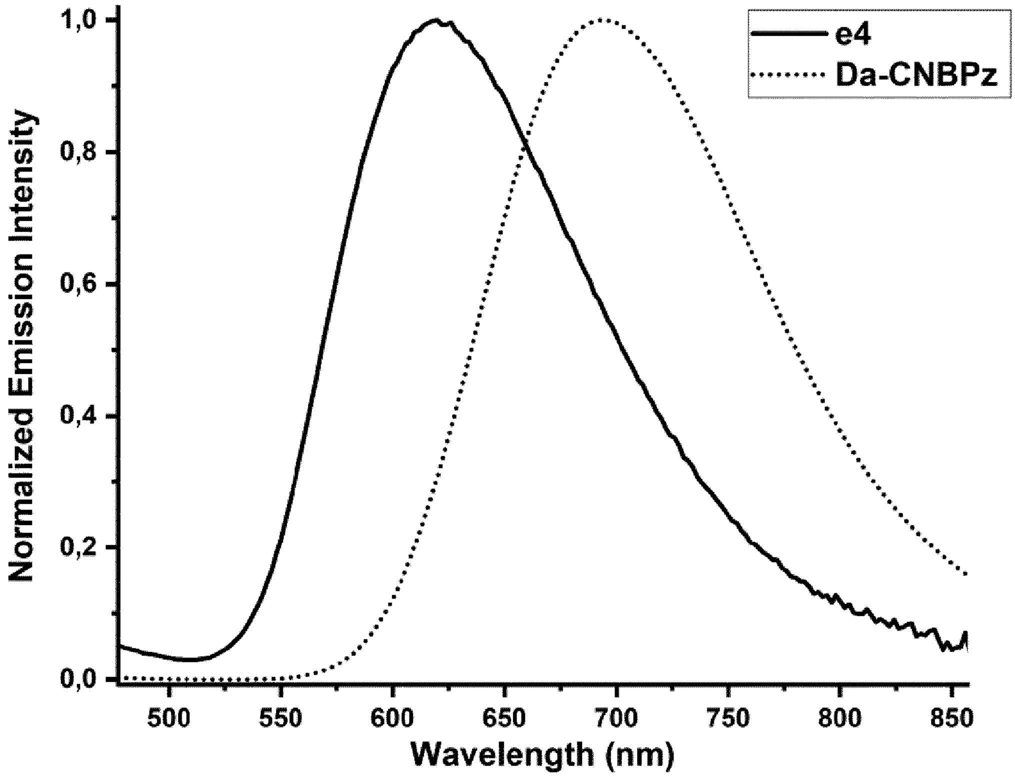


Figure 4. Comparison of PL spectra of Da-CNBPz and e4.

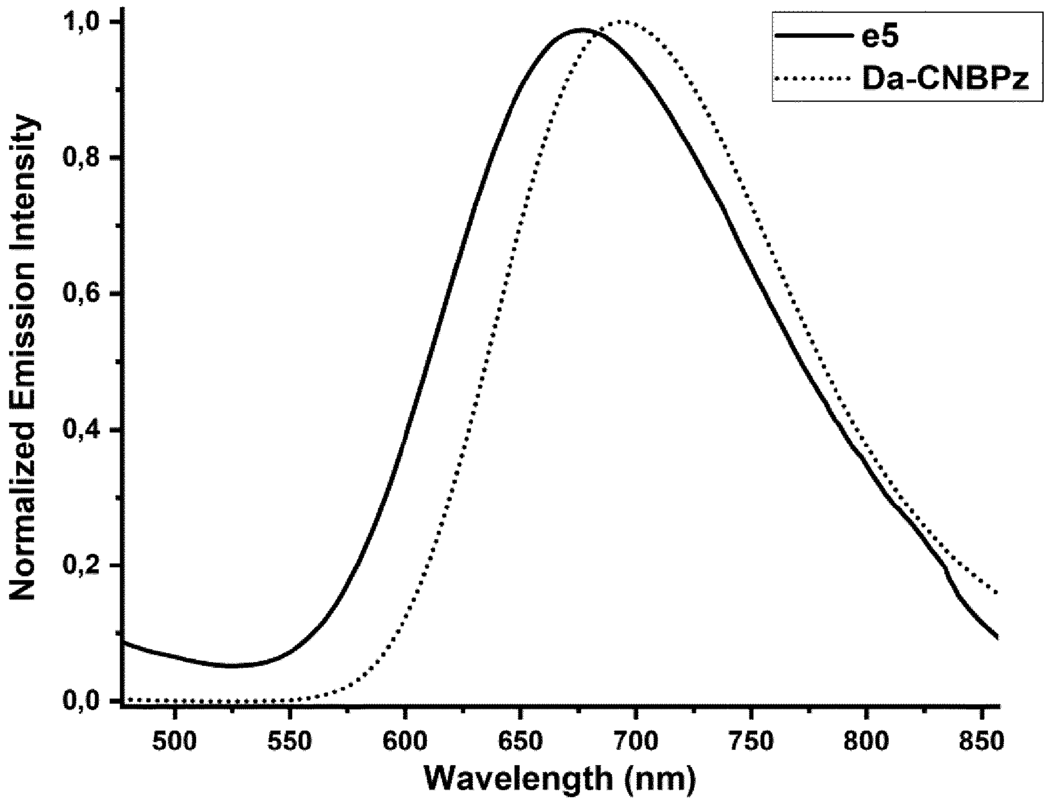


Figure 5. Comparison of PL spectra of Da-CNBPz and e5.

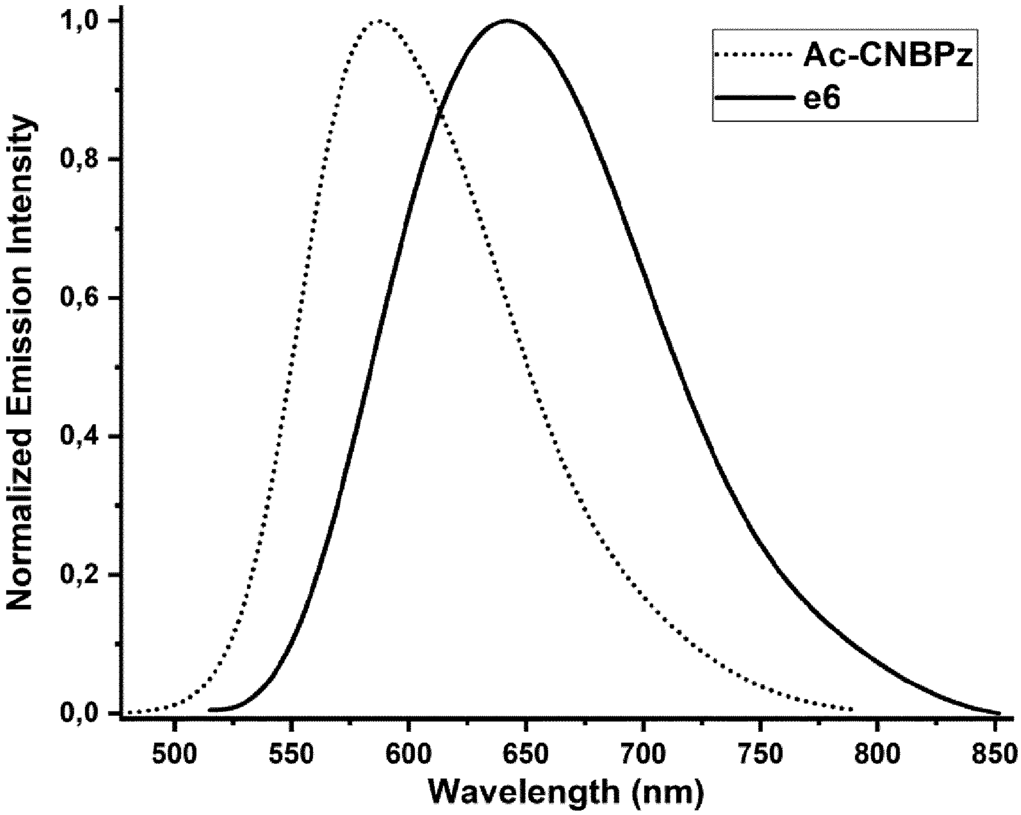


Figure 6. Comparison of PL spectra of Ac-CNBPz and e6.

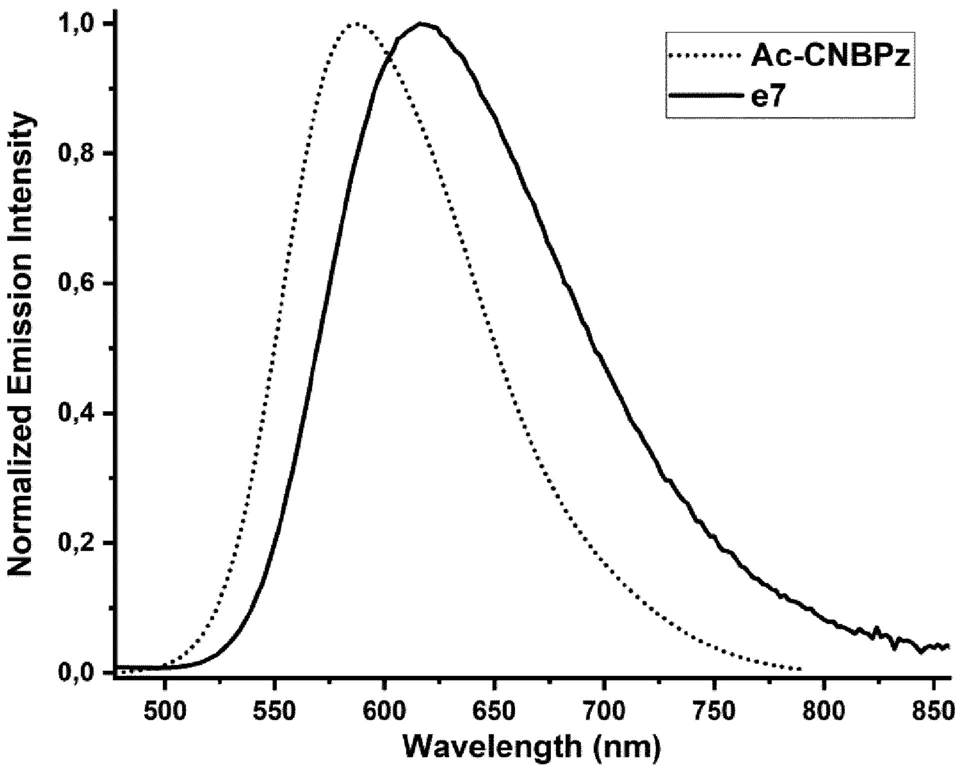


Figure 7. Comparison of PL spectra of Ac-CNBPz and e7.

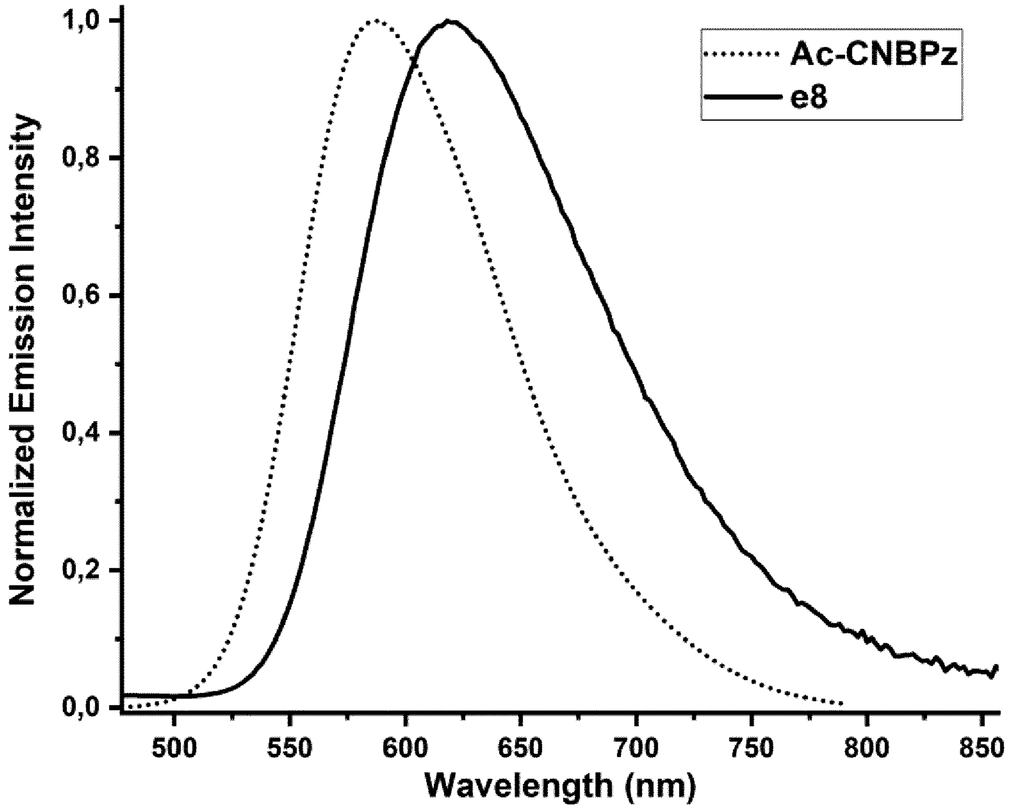


Figure 8. Comparison of PL spectra of Ac-CNBPz and e8.

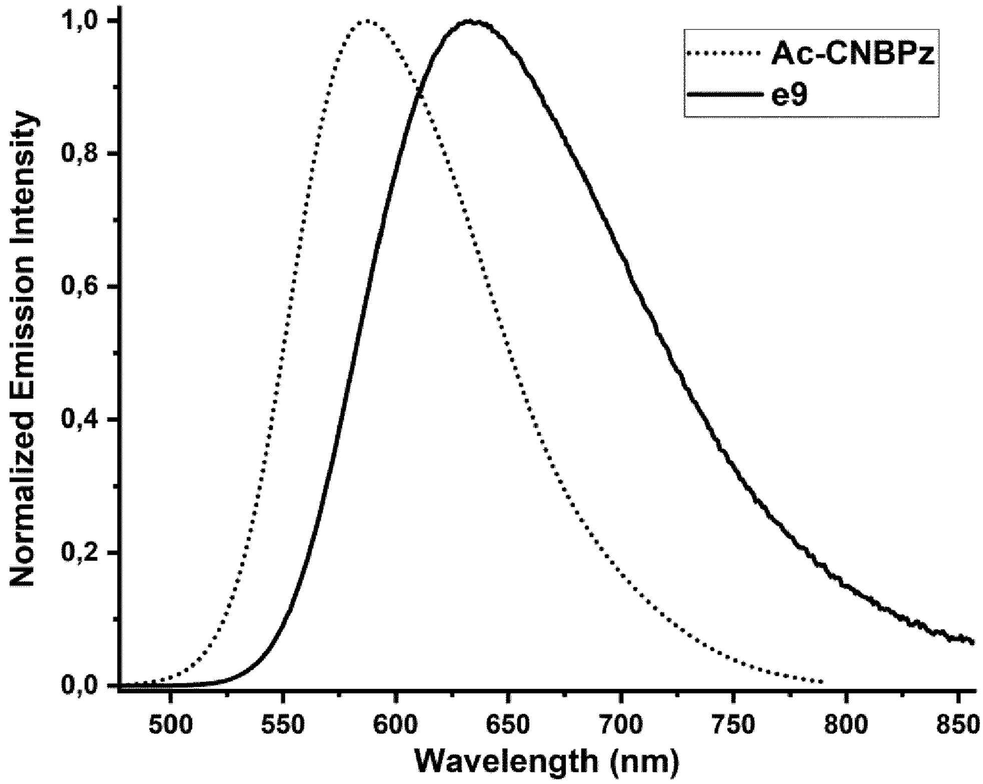


Figure 9. Comparison of PL spectra of Ac-CNBPz and e9.

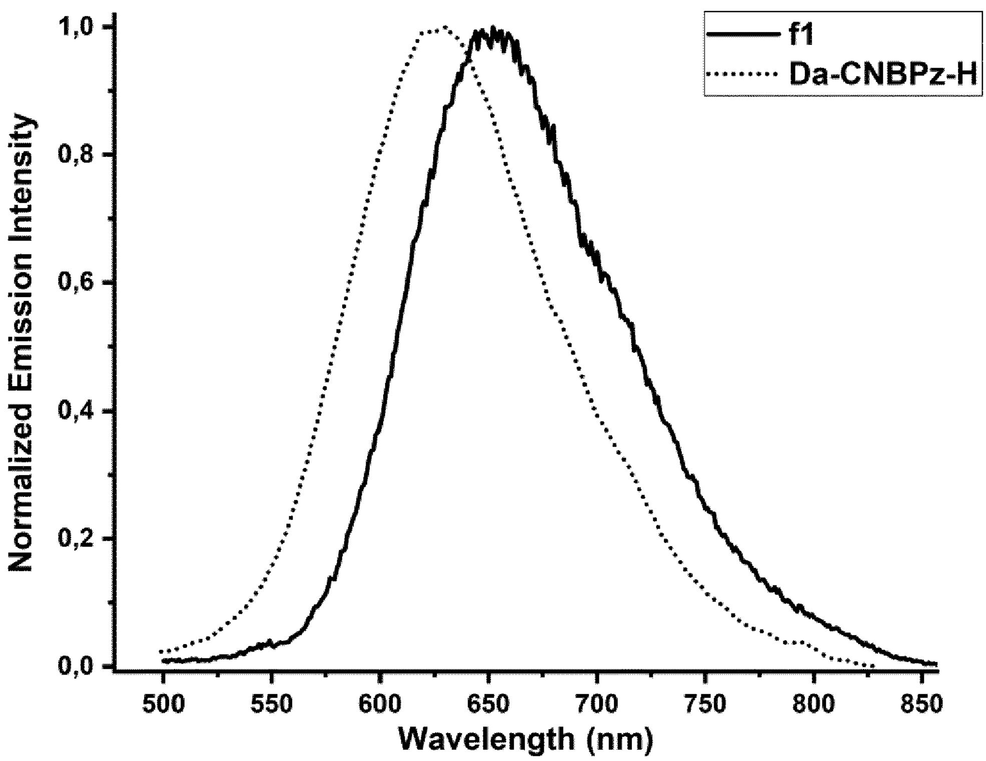


Figure 10. Comparison of PL spectra of Da-CNBPz-H and f1.

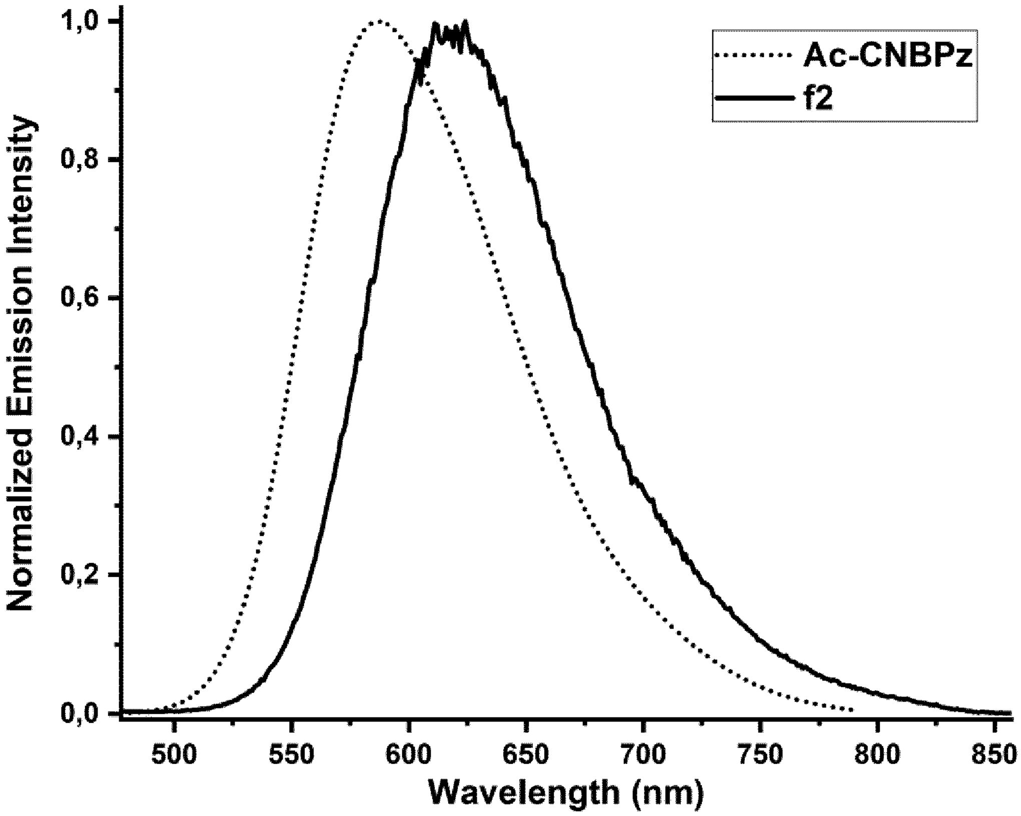


Figure 11. Comparison of PL spectra of Ac-CNBPz and f2.

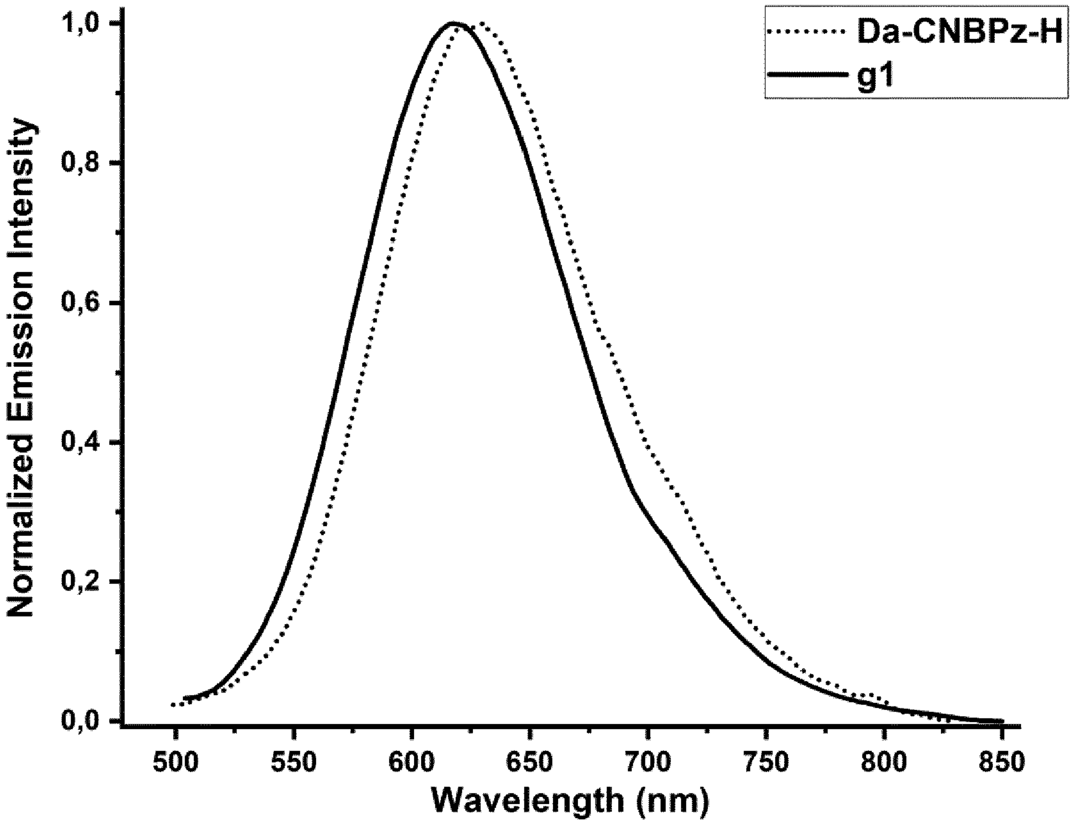

Figure 12. Comparison of PL spectra of Da-CNBPz-H and g1.

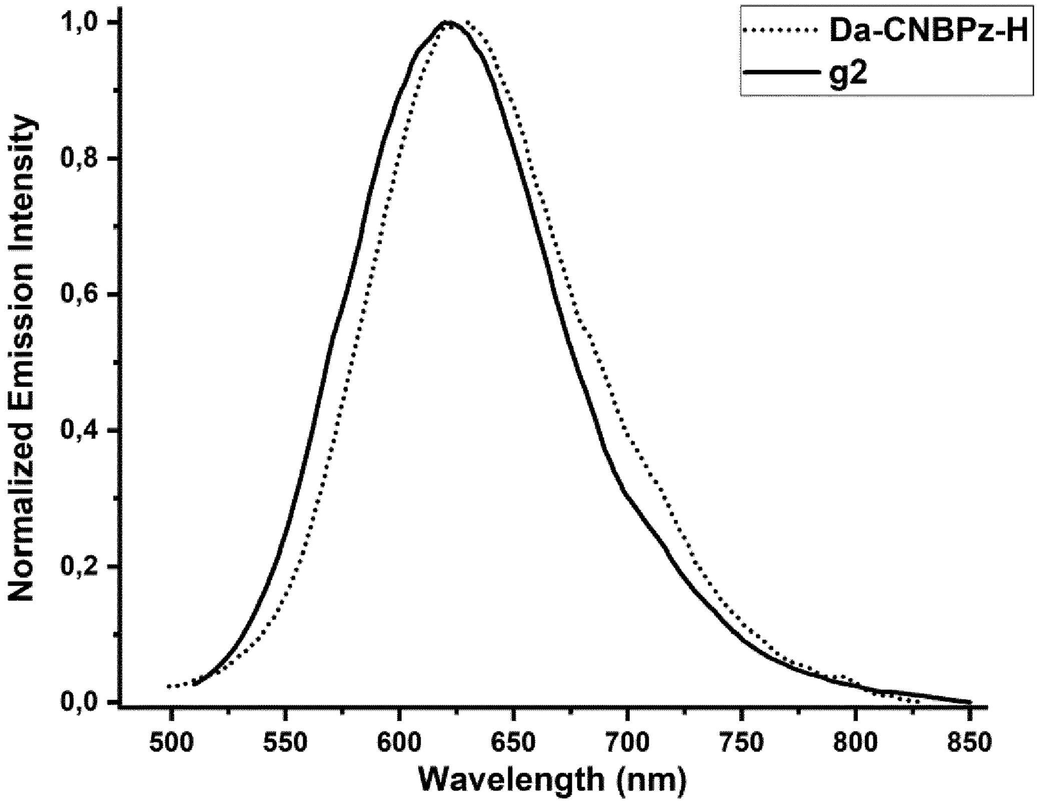


Figure 13. Comparison of PL spectra of Da-CNBPz-H and g2.

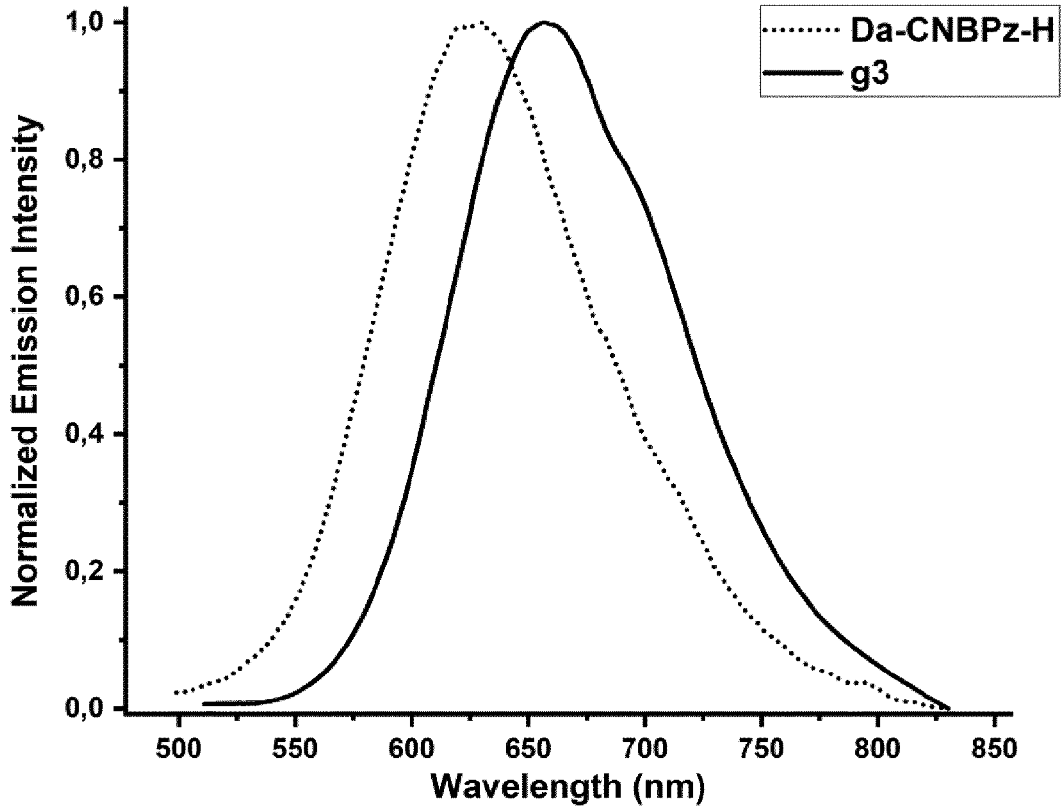


Figure 14. Comparison of PL spectra of Da-CNBPz-H and g3.

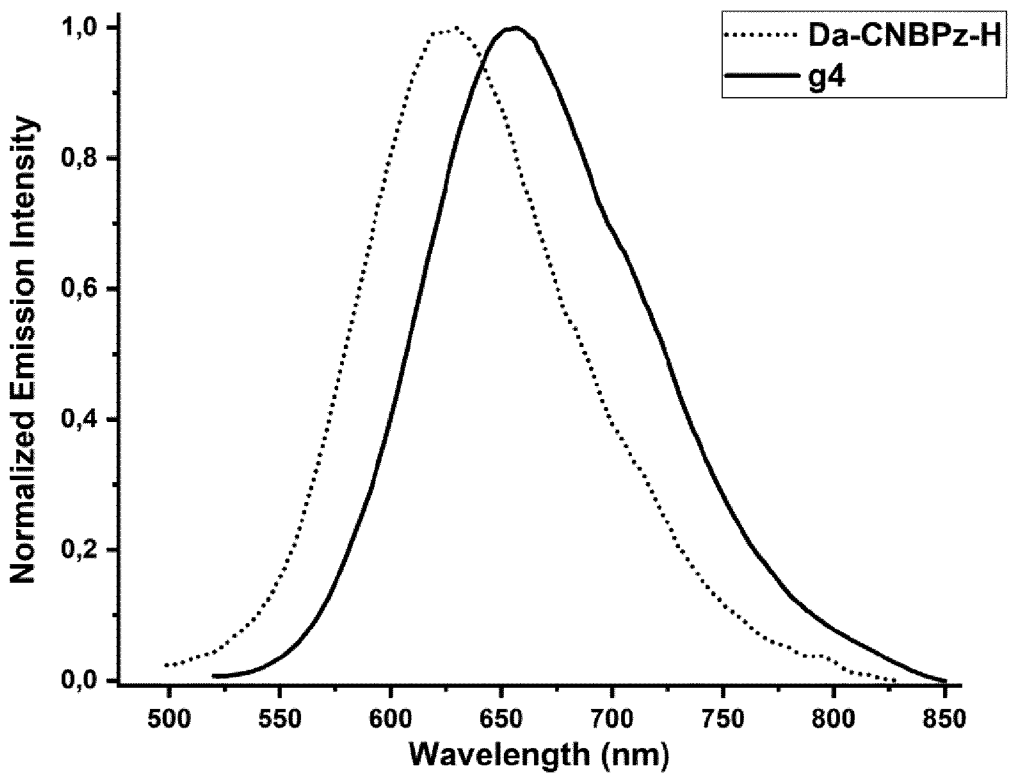


Figure 15. Comparison of PL spectra of Da-CNBPz-H and g4.

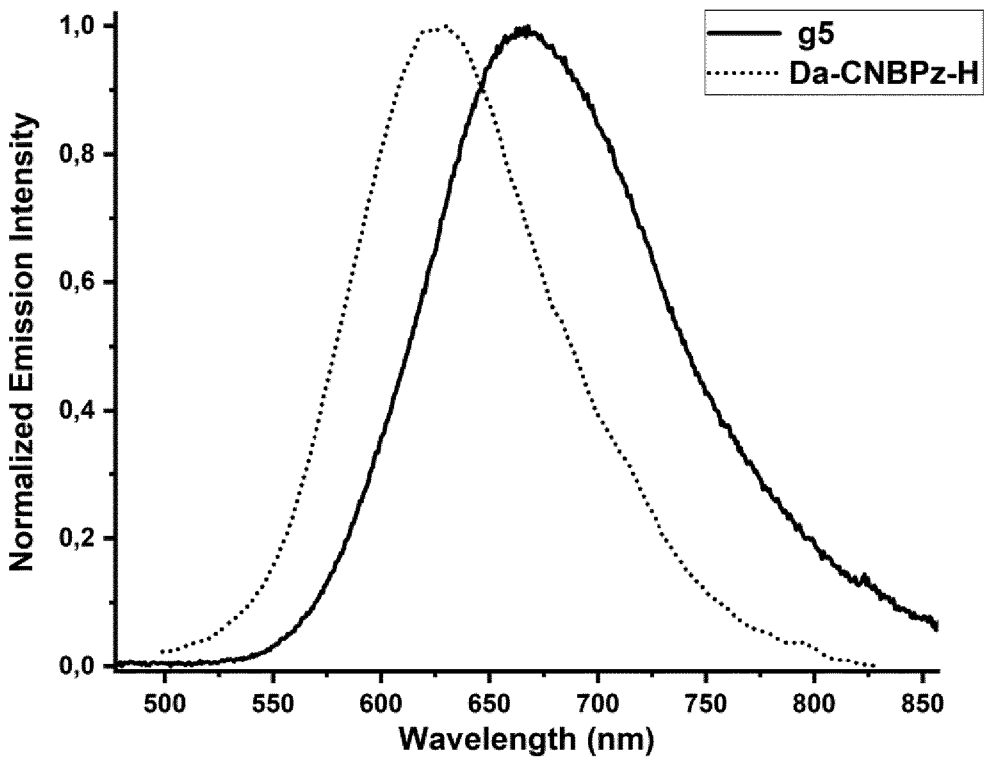


Figure 16. Comparison of PL spectra of Da-CNBPz-H and g5.

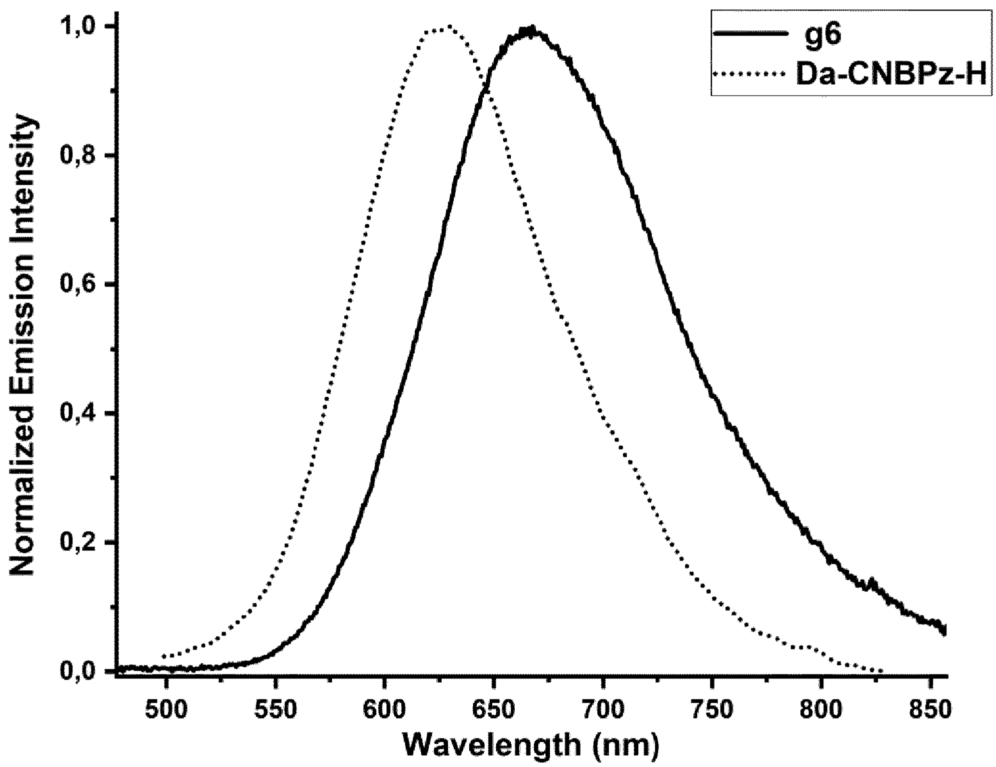


Figure 17. Comparison of PL spectra of Da-CNBPz-H and g6.

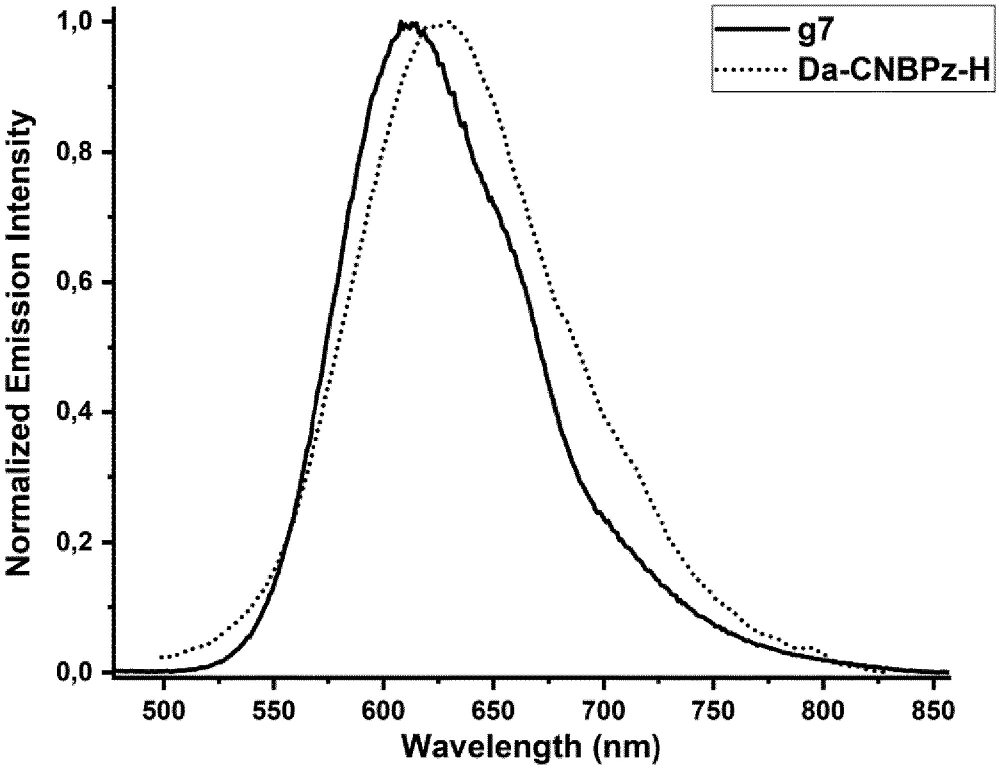


Figure 18. Comparison of PL spectra of Da-CNBPz-H and g7.

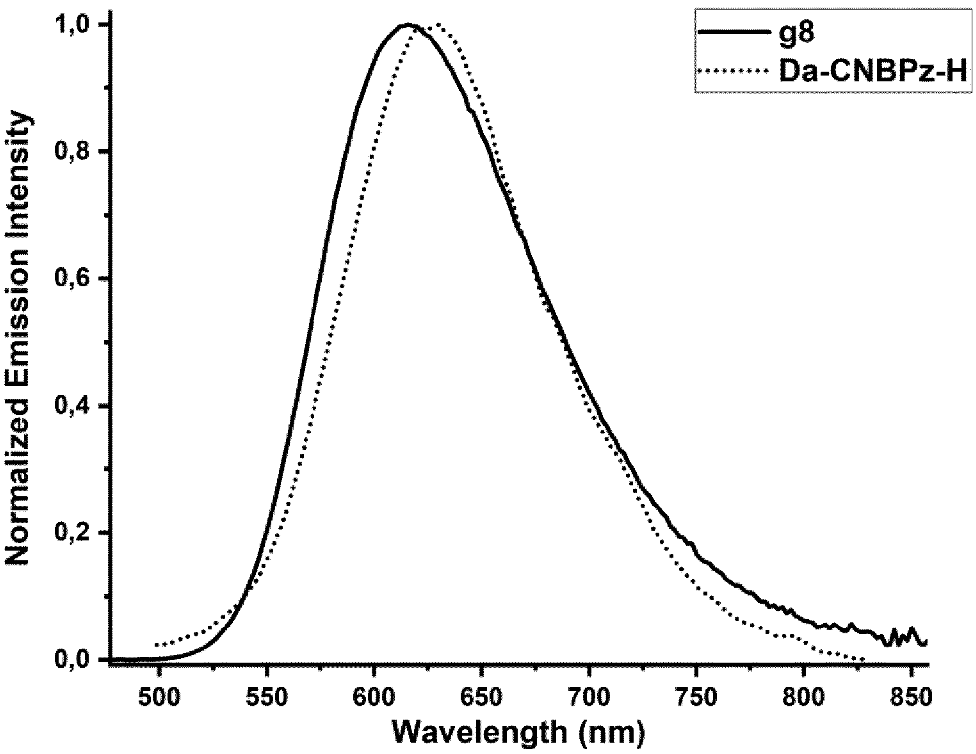


Figure 19. Comparison of PL spectra of Da-CNBPz-H and g8.

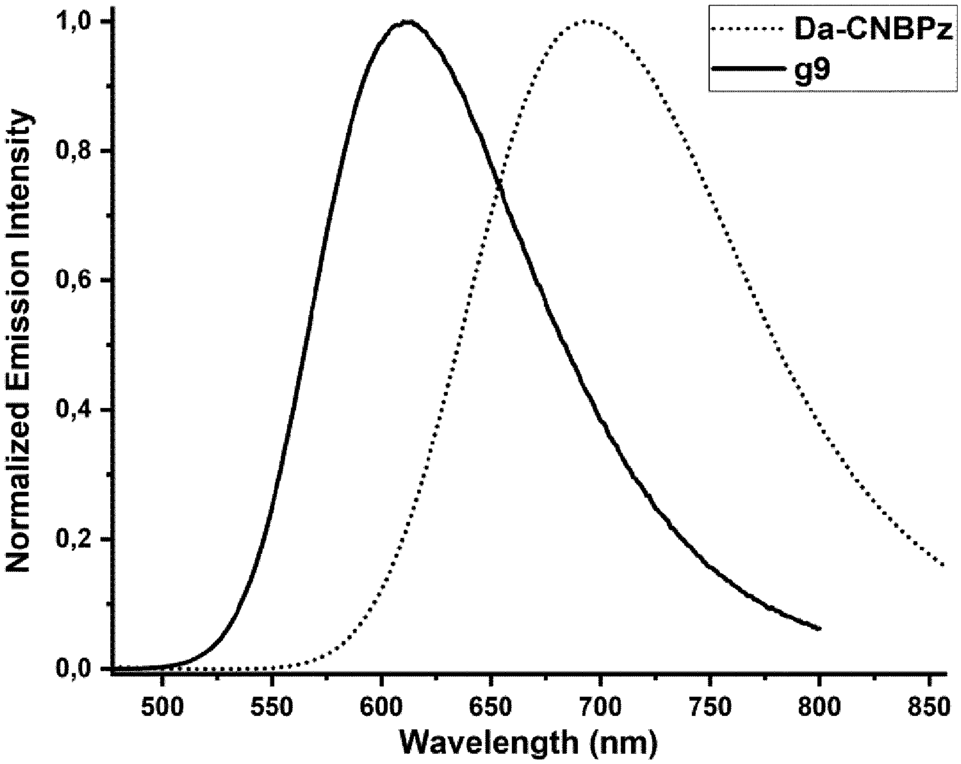


Figure 20. Comparison of PL spectra of Da-CNBPz and g9.

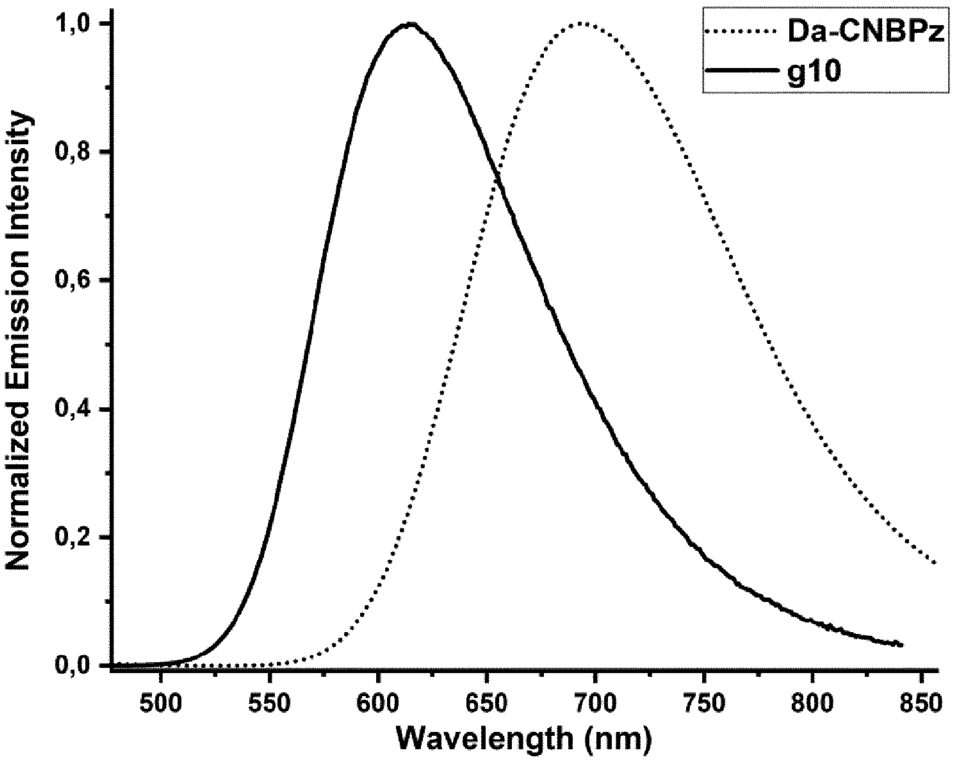


Figure 21. Comparison of PL spectra of Da-CNBPz and g10.

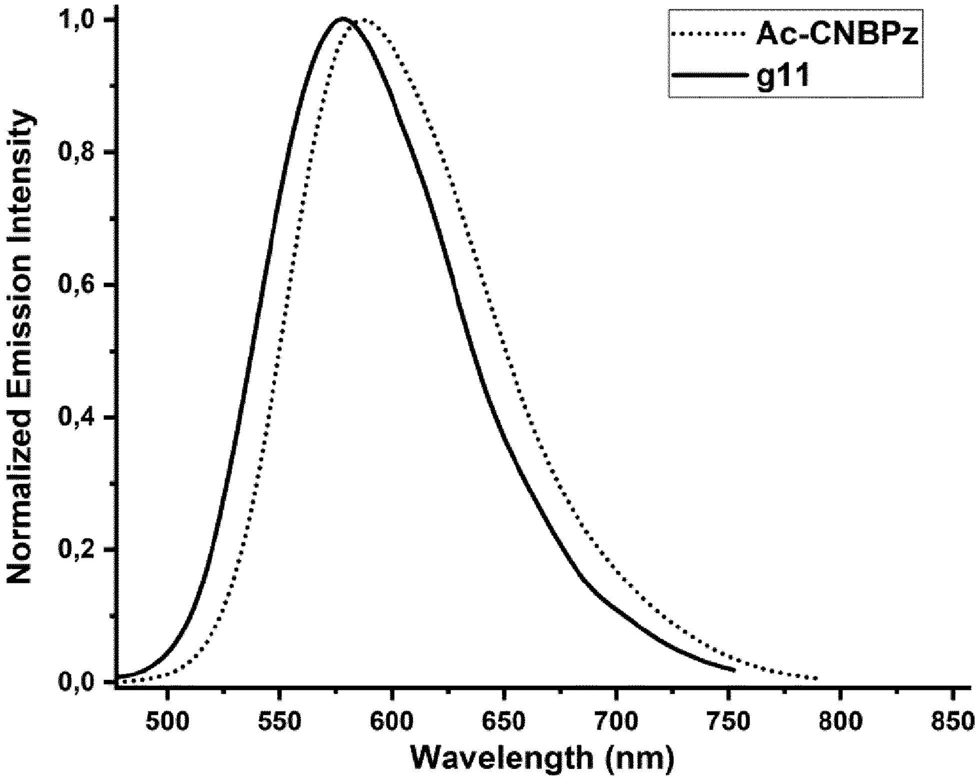


Figure 22. Comparison of PL spectra of Ac-CNBPz and g11.

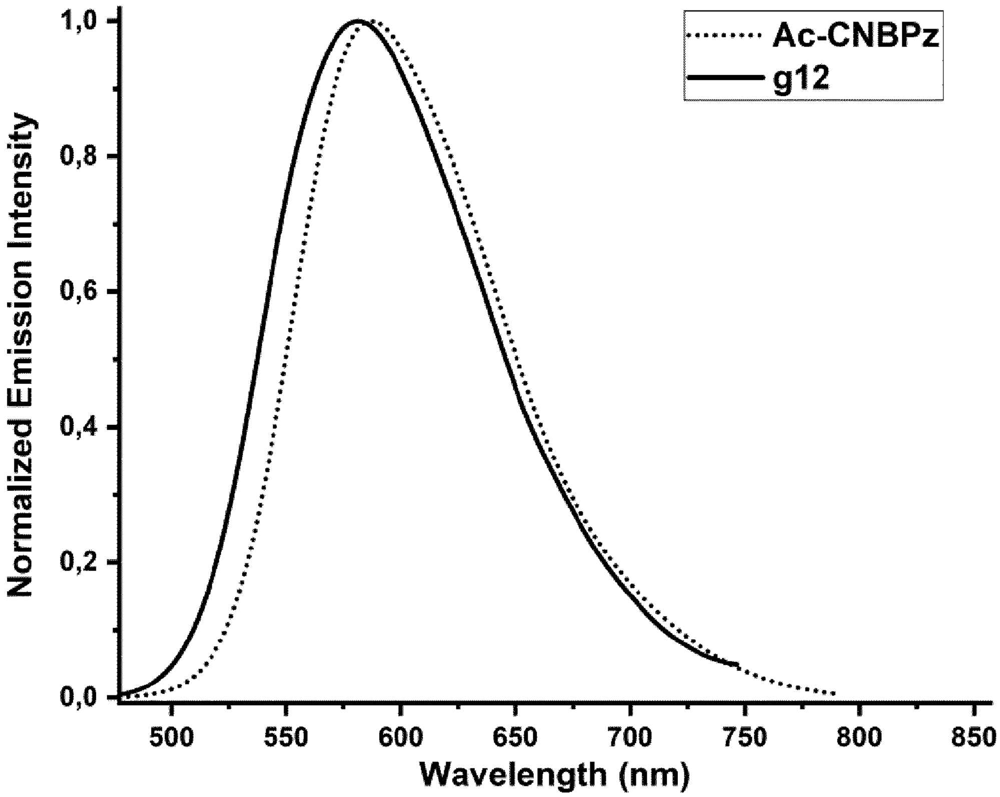


Figure 23. Comparison of PL spectra of Ac-CNBPz and g12.

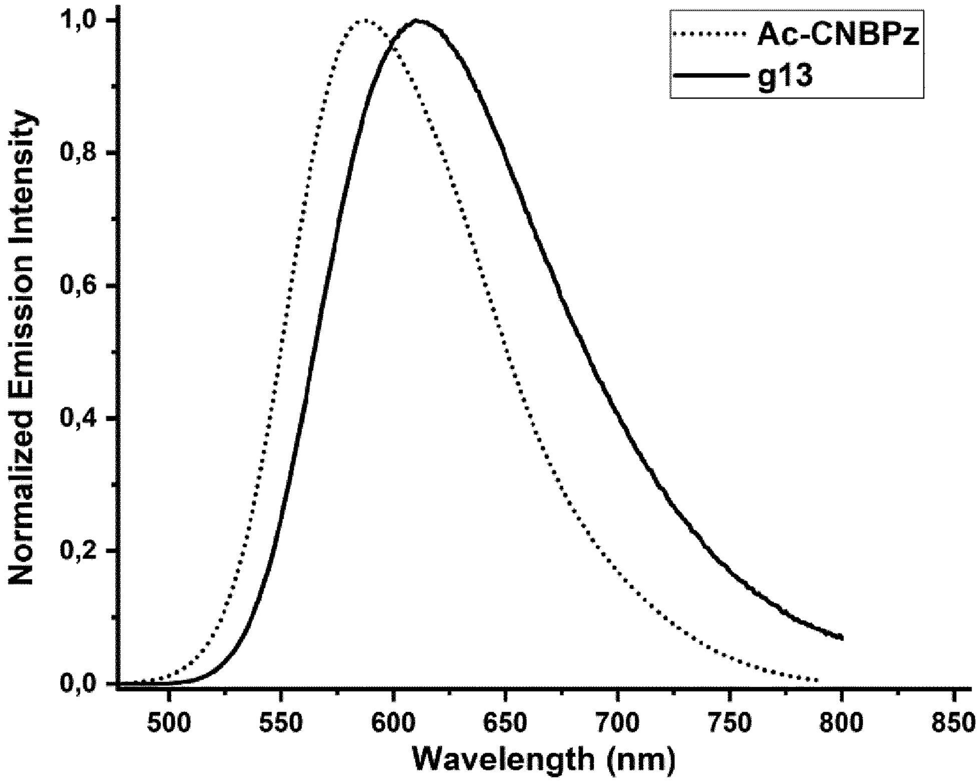


Figure 24. Comparison of PL spectra of Ac-CNBPz and g13.

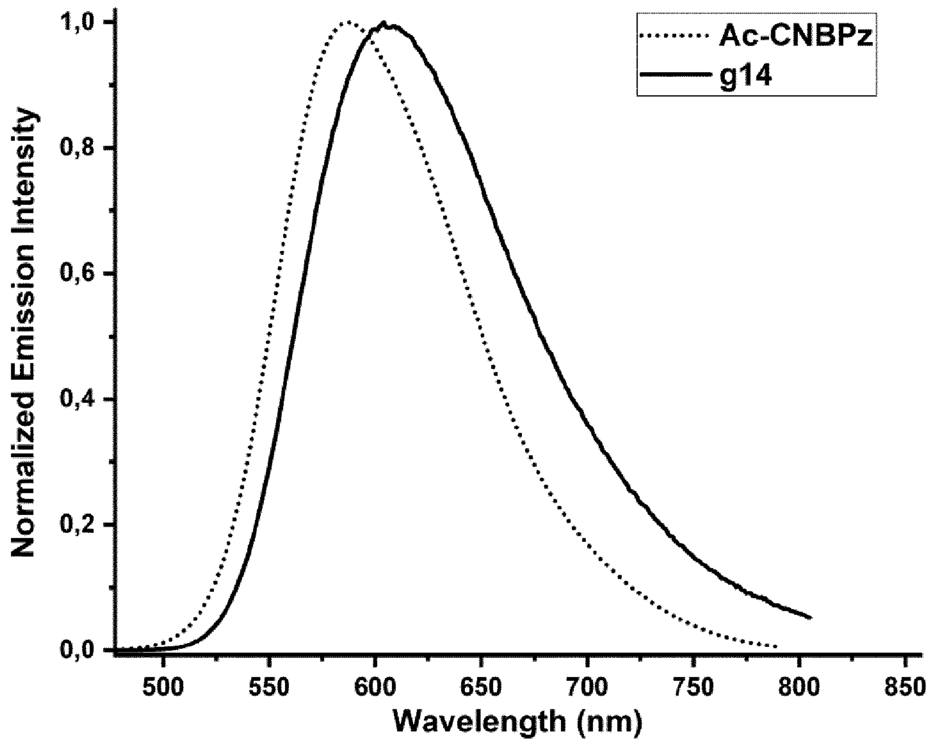


Figure 25. Comparison of PL spectra of Ac-CNBPz and g14.

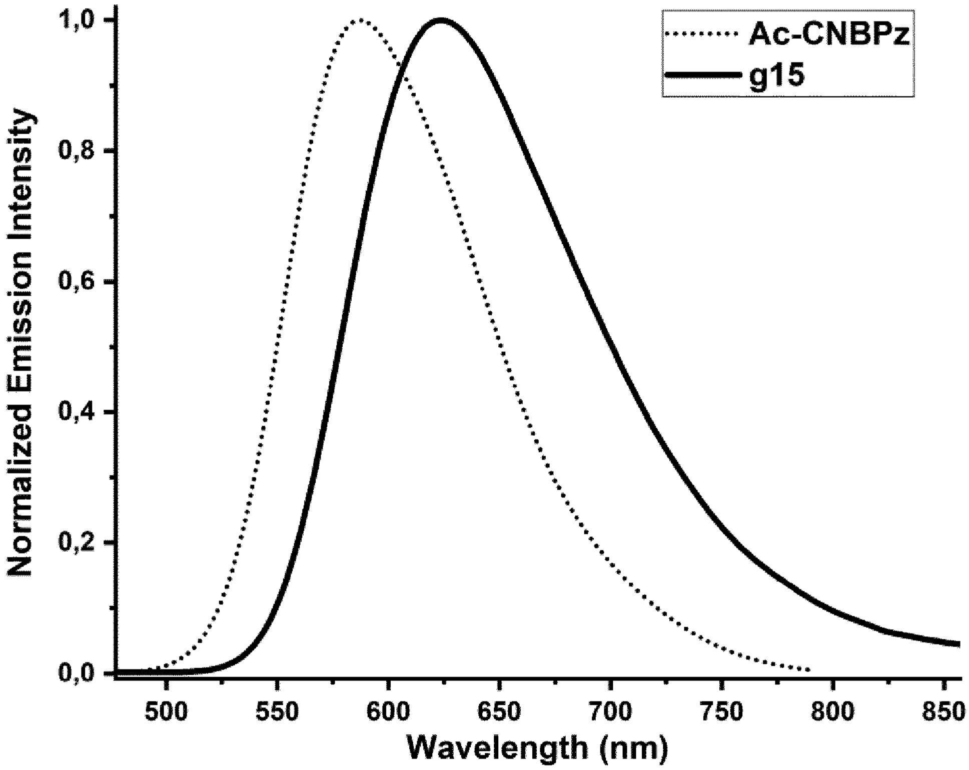


Figure 26. Comparison of PL spectra of Ac-CNBPz and g15.

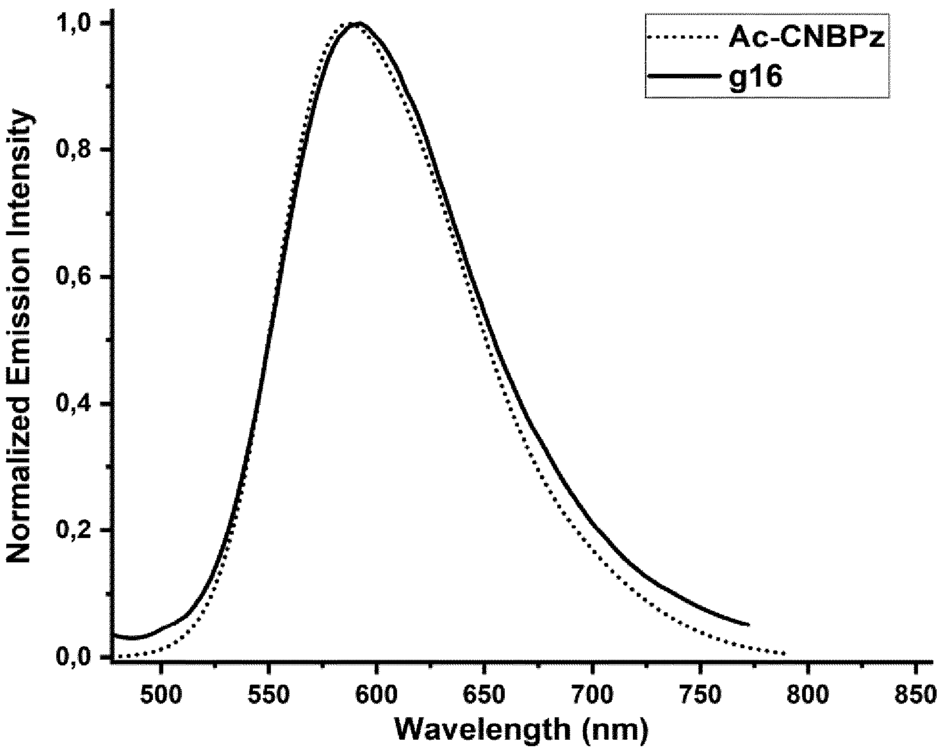


Figure 27. Comparison of PL spectra of Ac-CNBPz and g16.

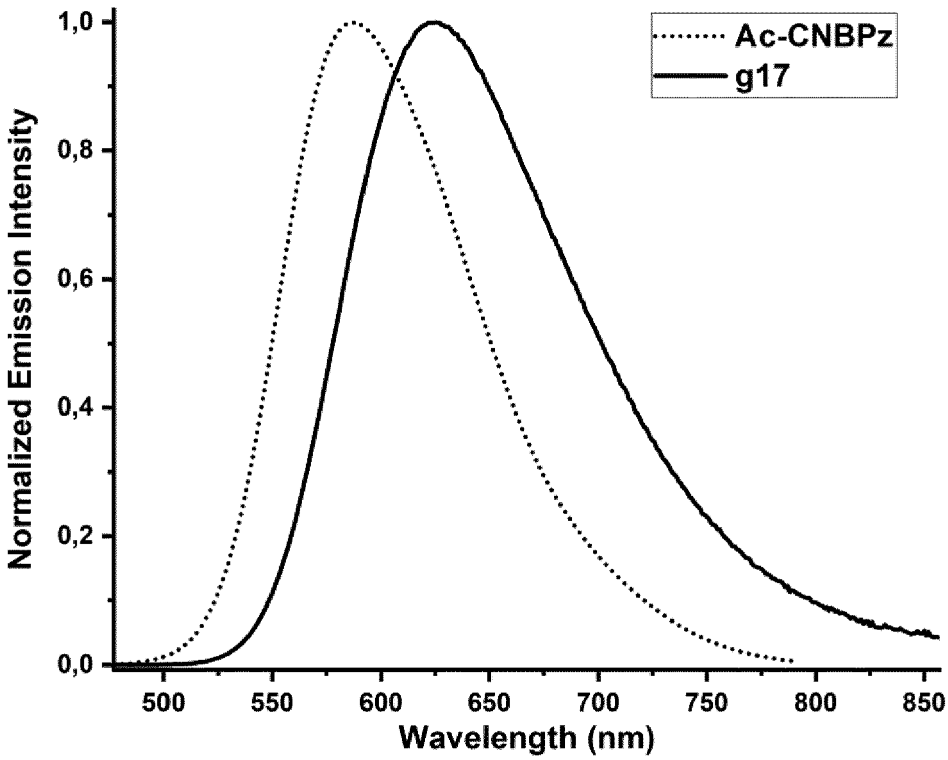


Figure 28. Comparison of PL spectra of Ac-CNBPz and g17.

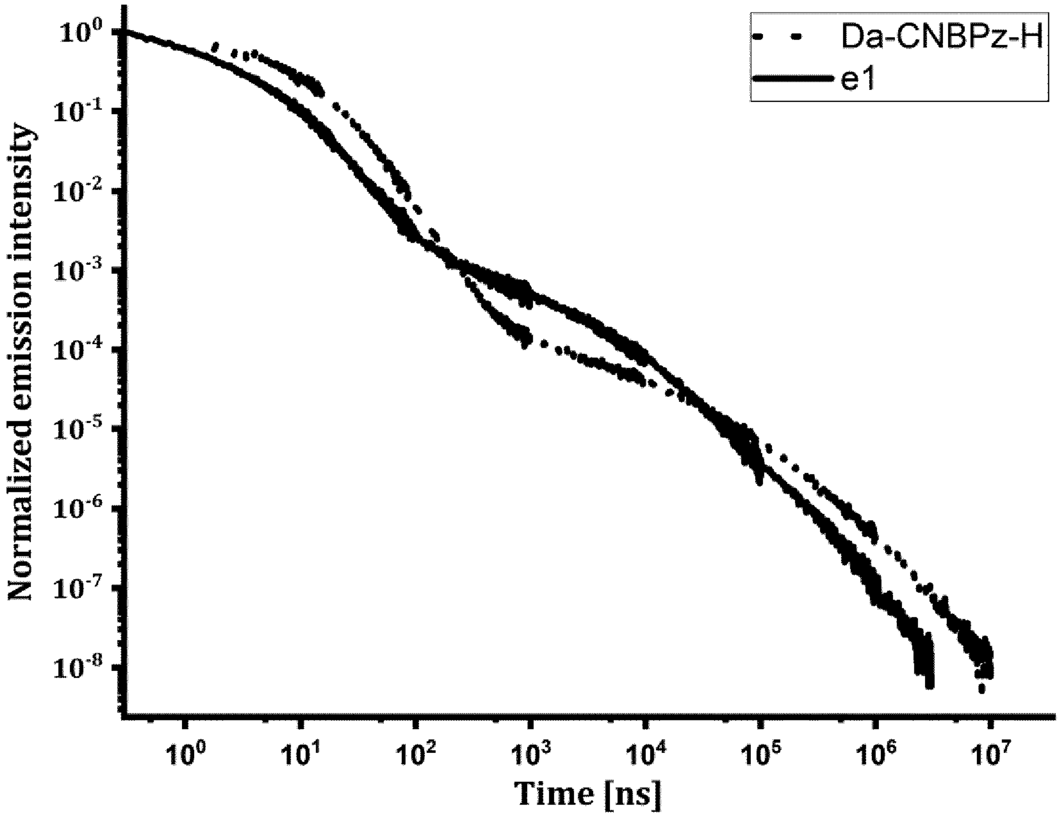


Figure 29. Comparison of PL decay of Da-CNBPz-H and e1.

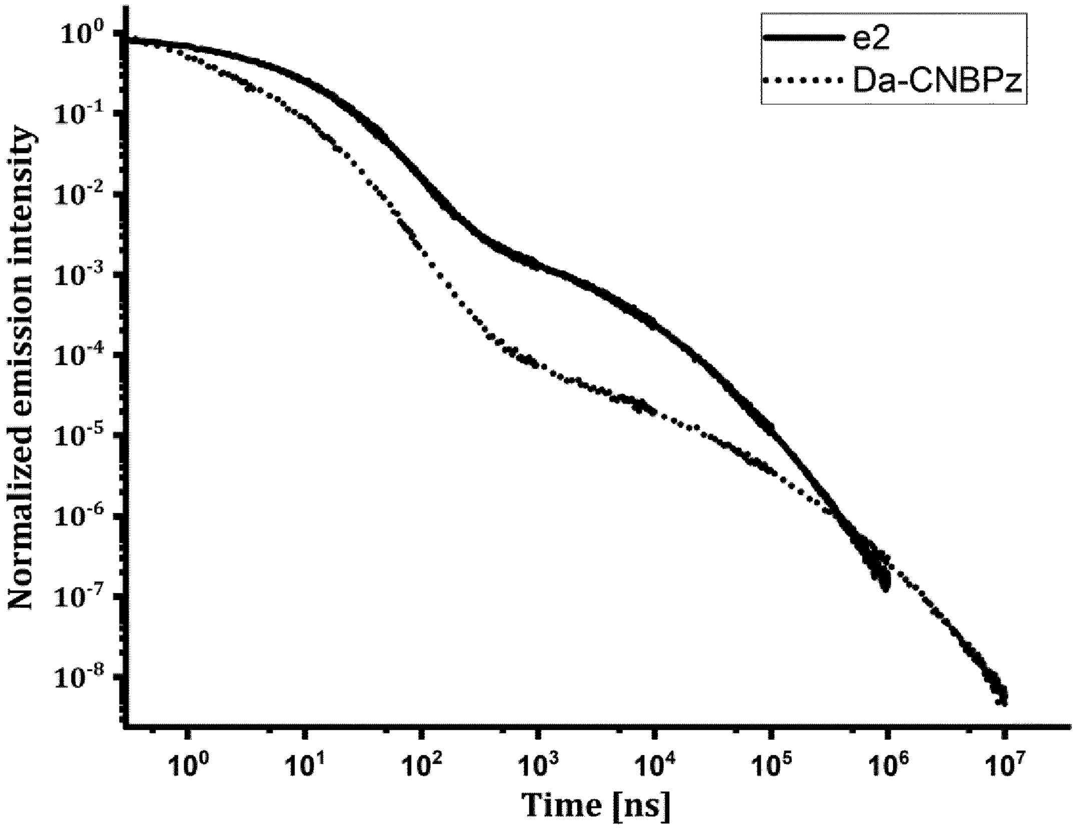

Figure 30. Comparison of PL decay of Da-CNBPz and e2.

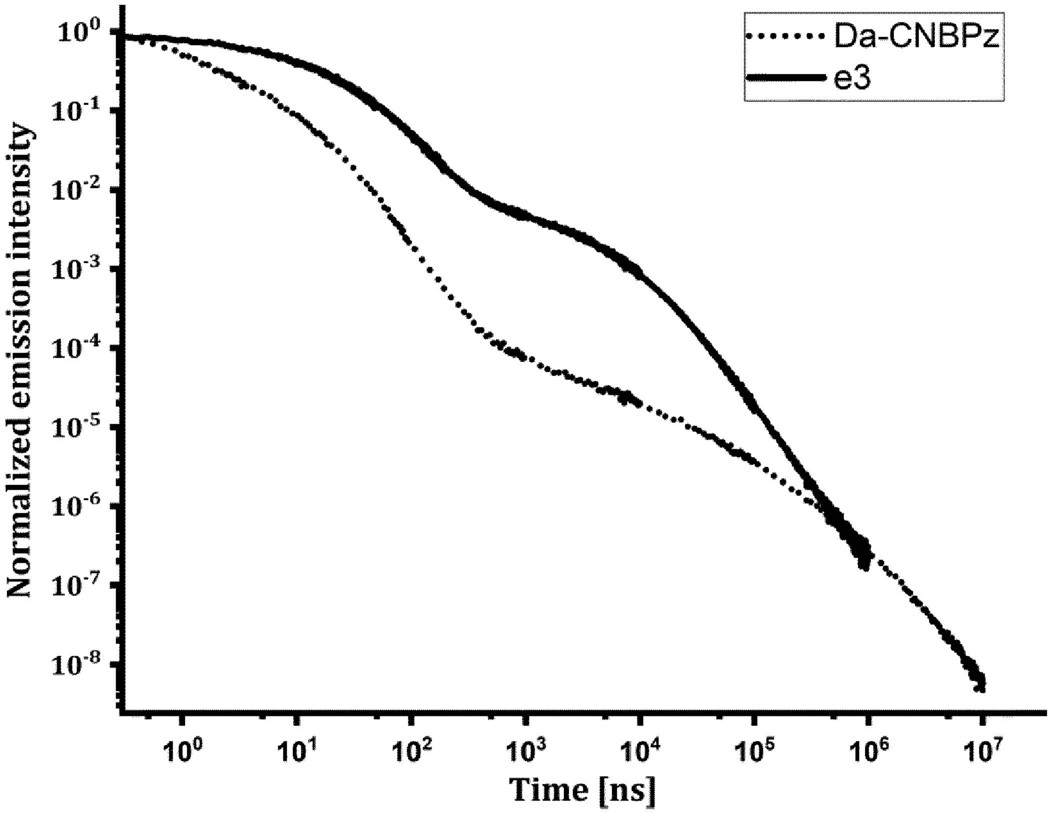


Figure 31. Comparison of PL decay of Da-CNBPz and e3.

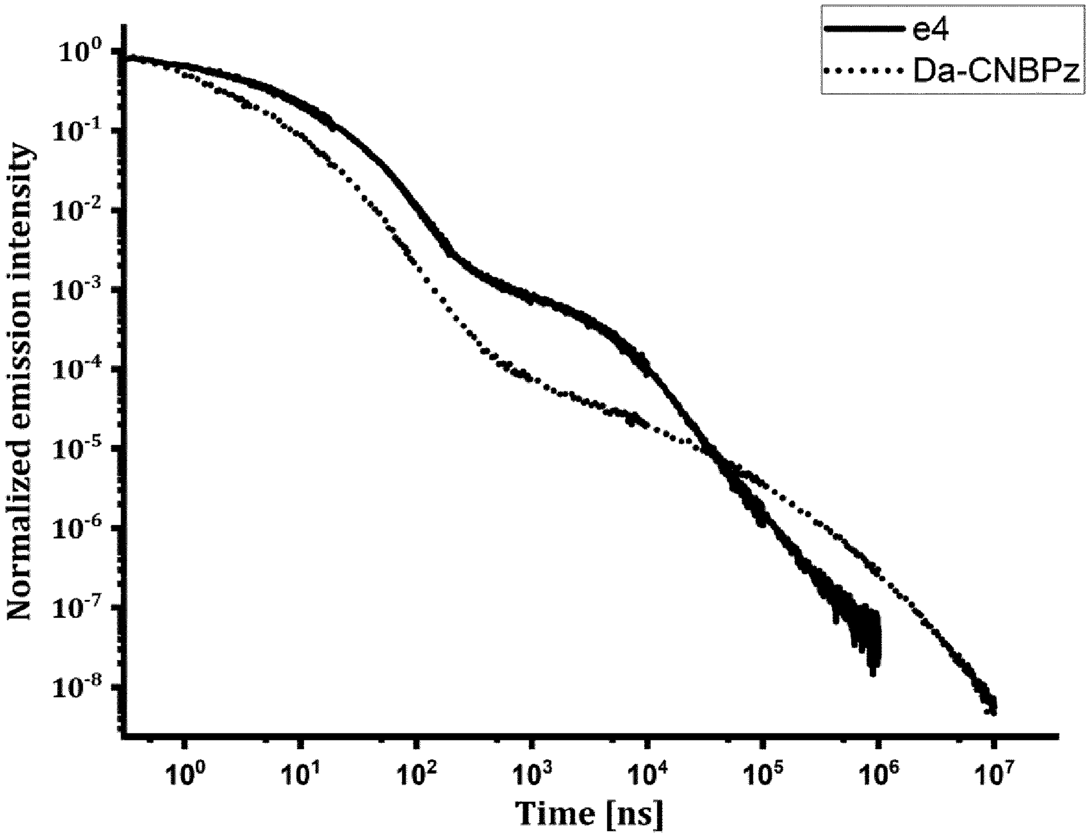


Figure 32. Comparison of PL decay of Da-CNBPz and e4.

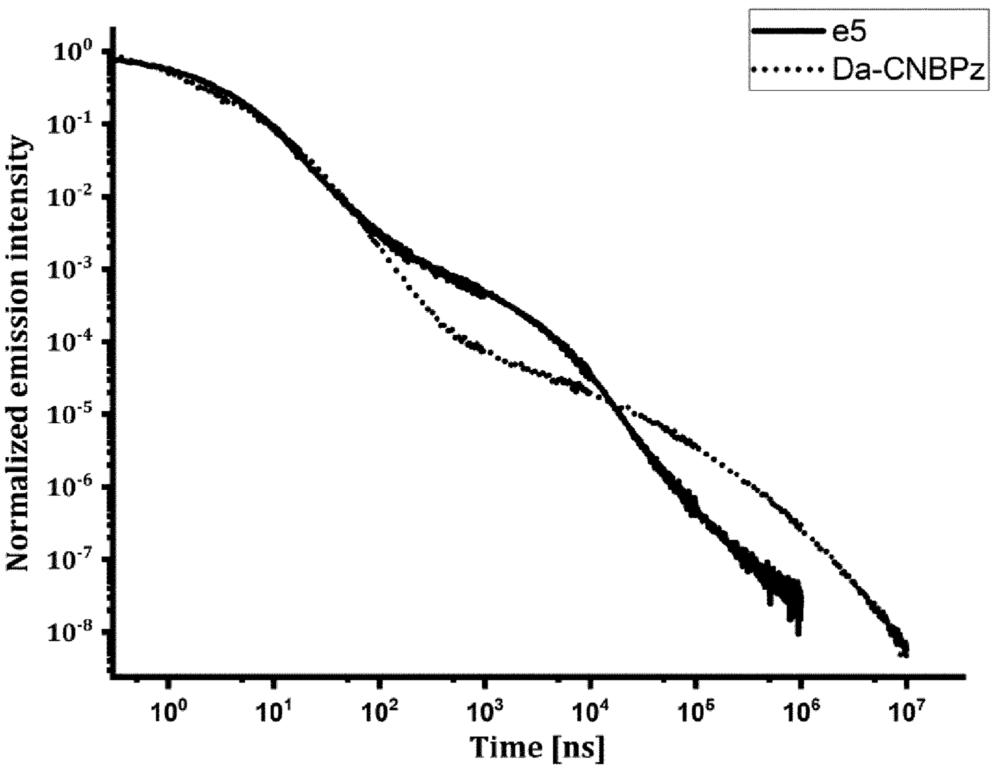


Figure 33. Comparison of PL decay of Da-CNBPz and e5.

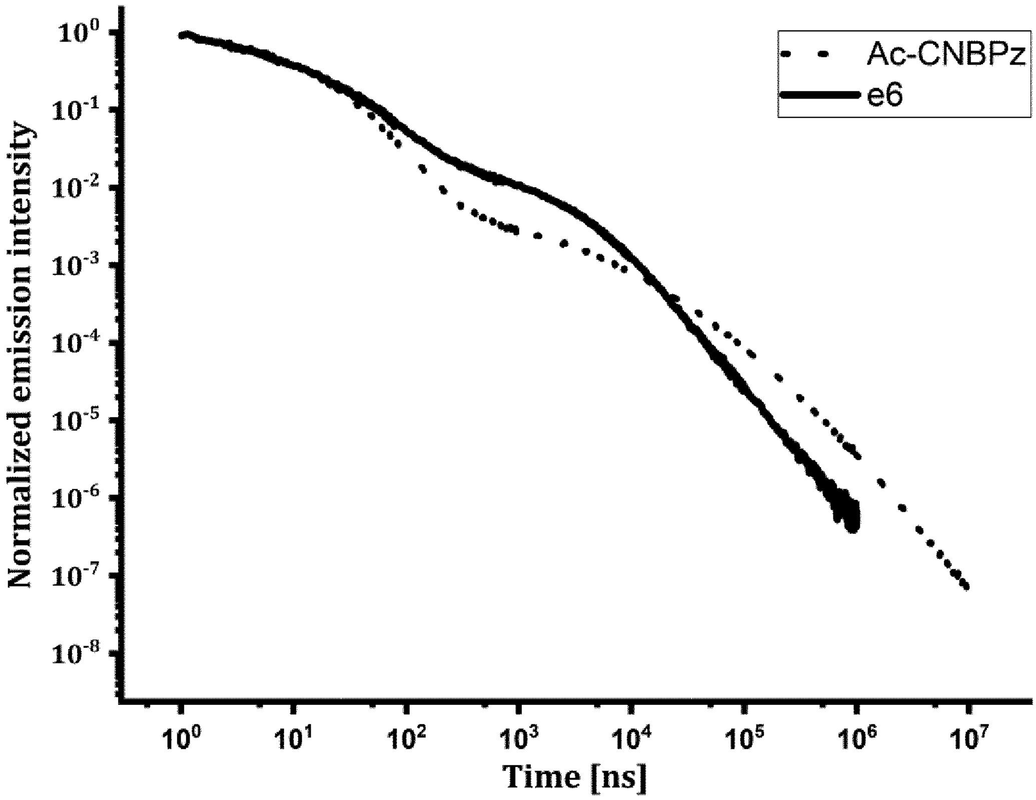


Figure 34. Comparison of PL decay of Ac-CNBPz and e6.

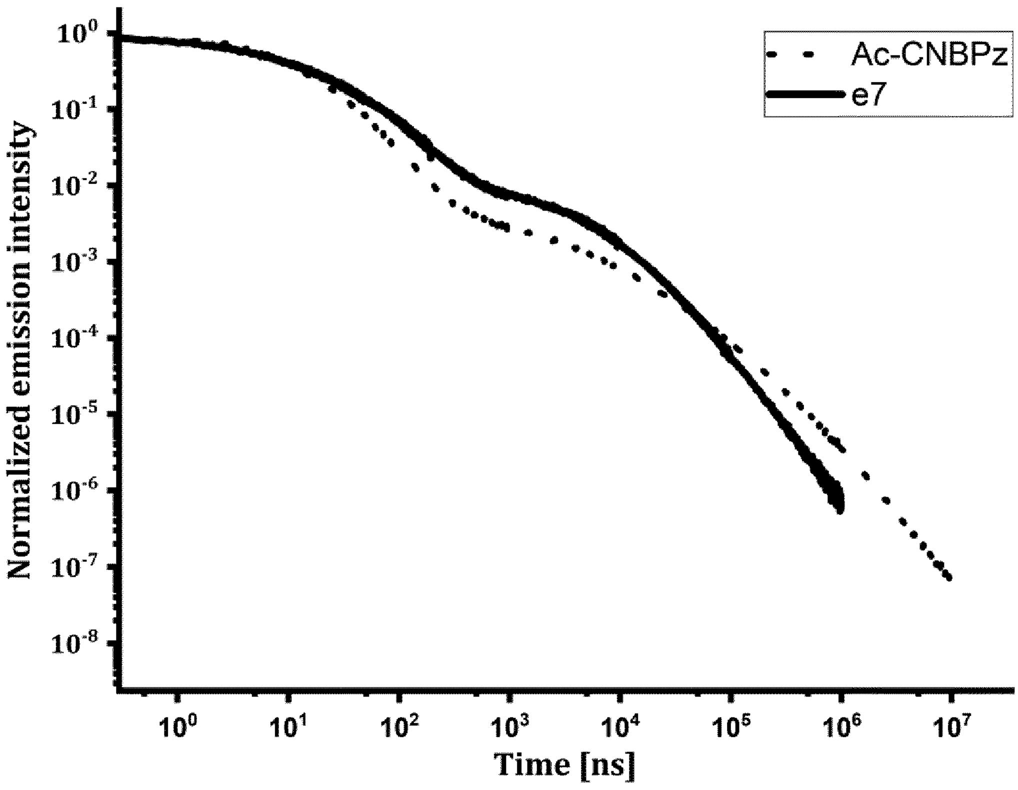

Figure 35. Comparison of PL decay of Ac-CNBPz and e7.

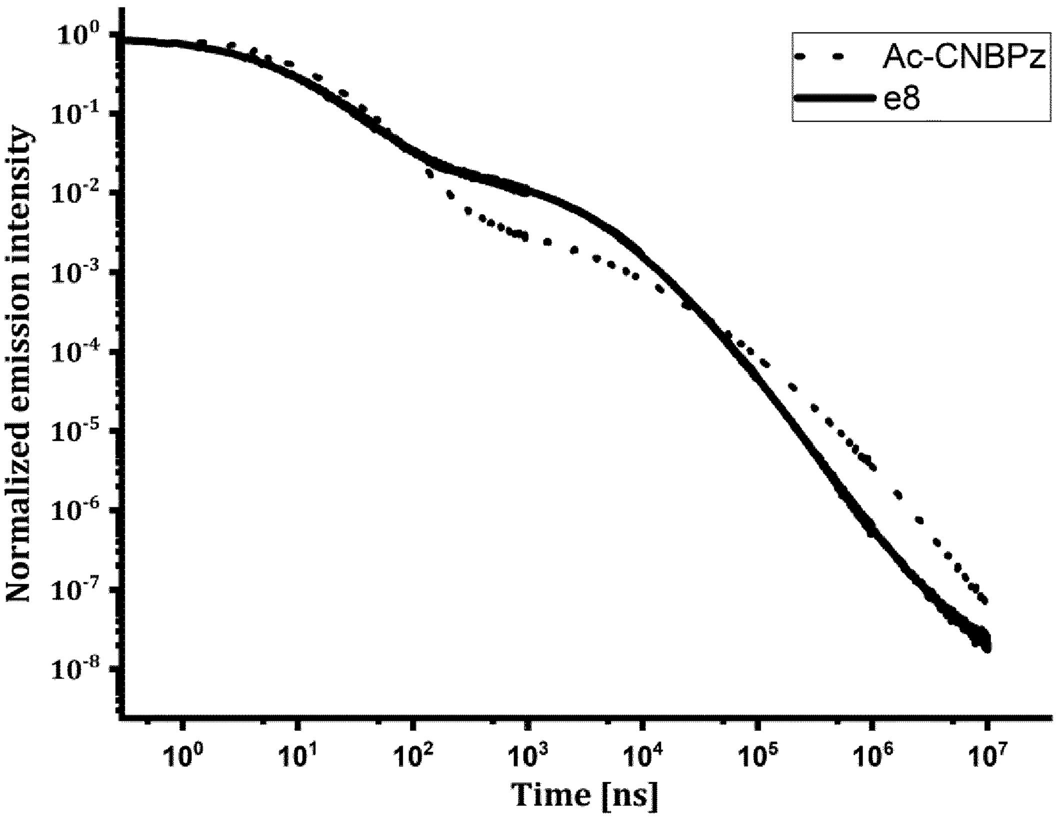


Figure 36. Comparison of PL decay of Ac-CNBPz and e8.

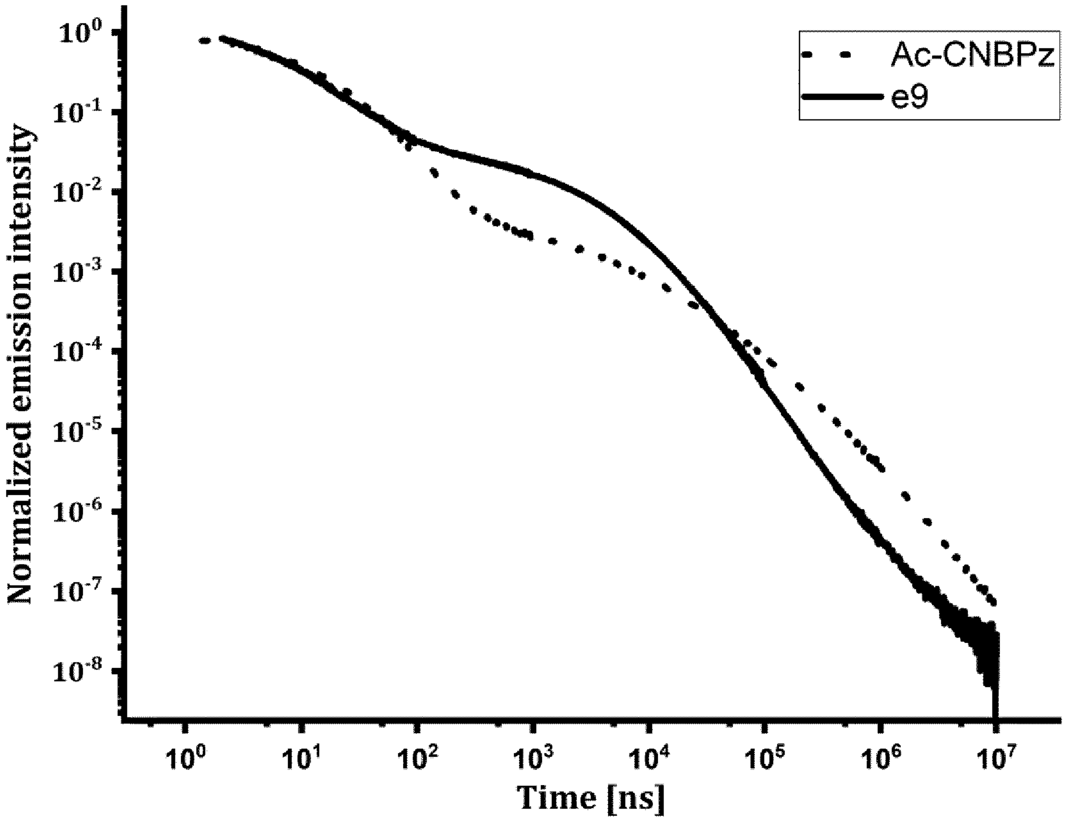


Figure 37. Comparison of PL decay of Ac-CNBPz and e9.

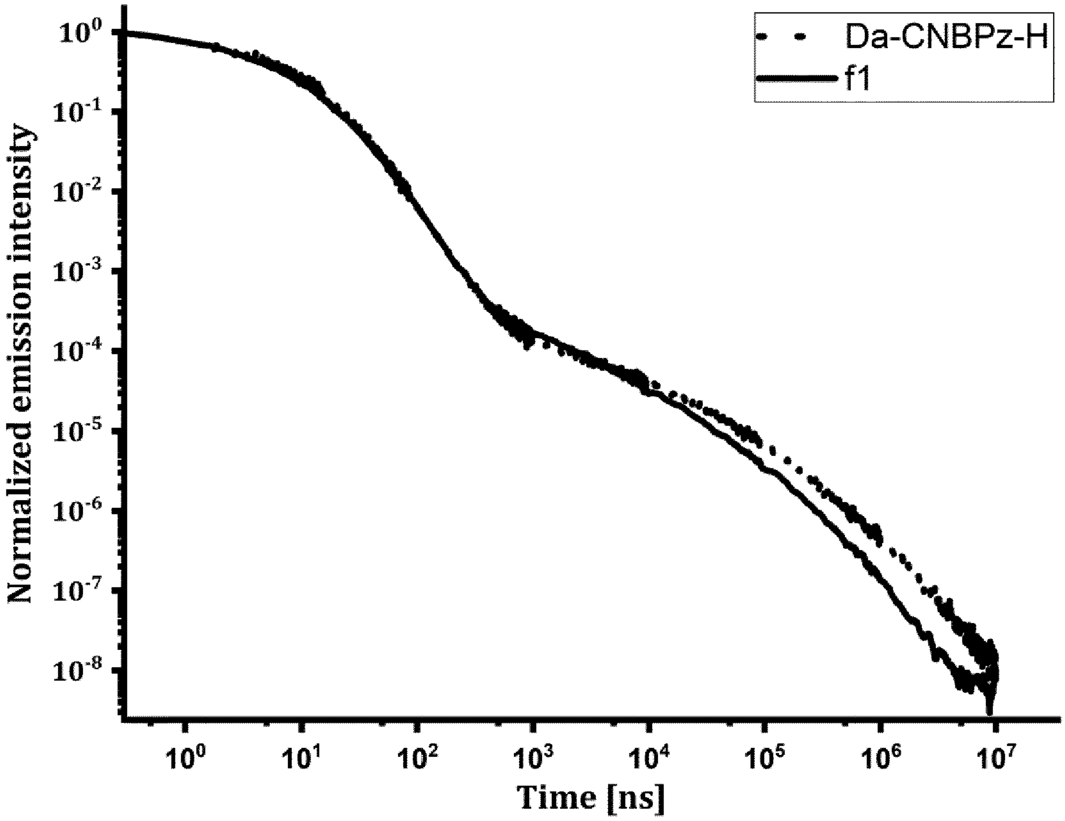

Figure 38. Comparison of PL decay of Da-CNBPz-H and f1.

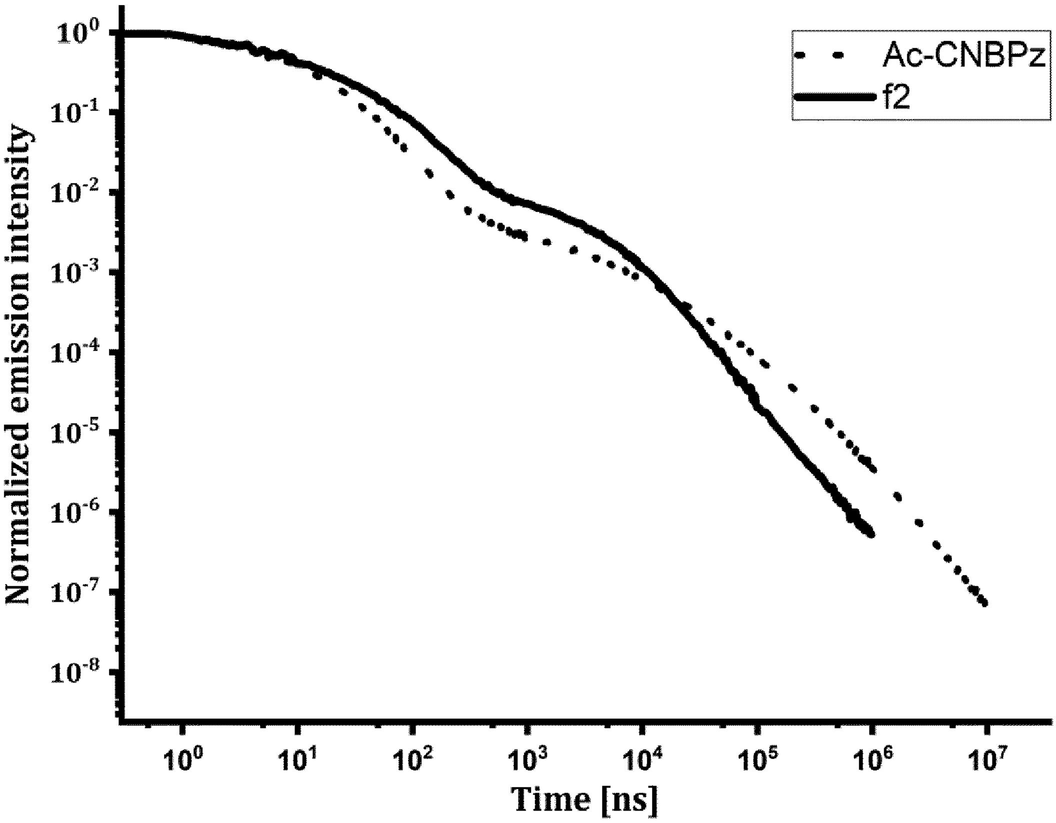


Figure 39. Comparison of PL decay of Ac-CNBPz and f2.

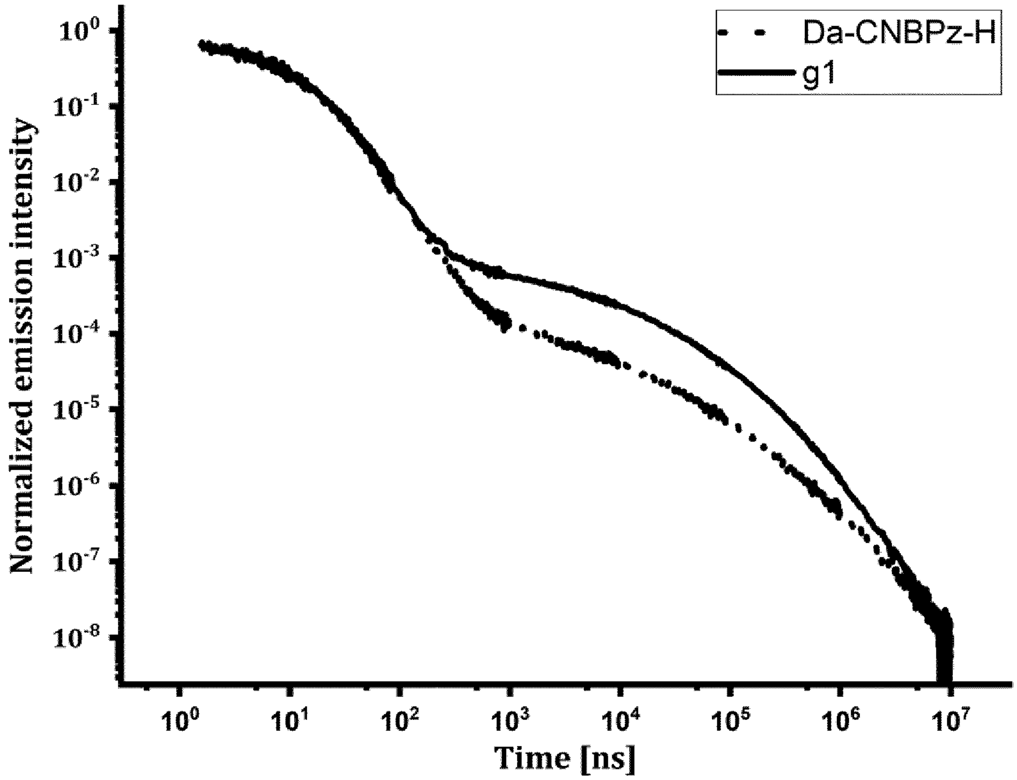


Figure 40. Comparison of PL decay of Da-CNBPz-H and g1.

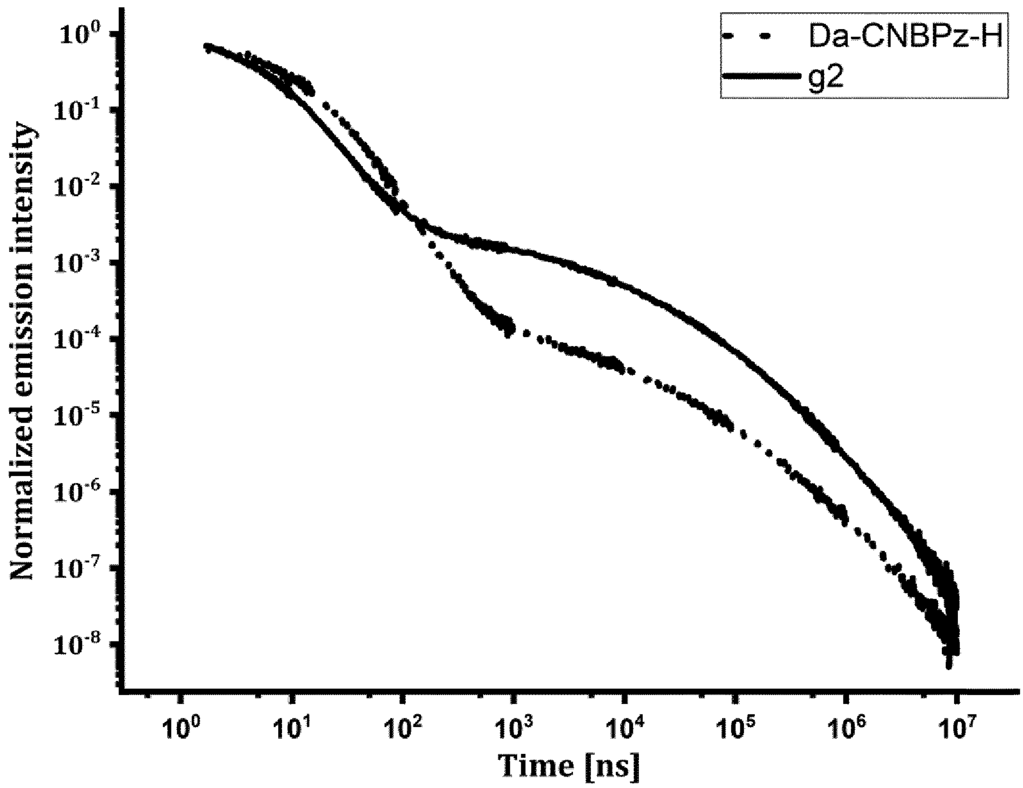


Figure 41. Comparison of PL decay of Da-CNBPz-H and g2.

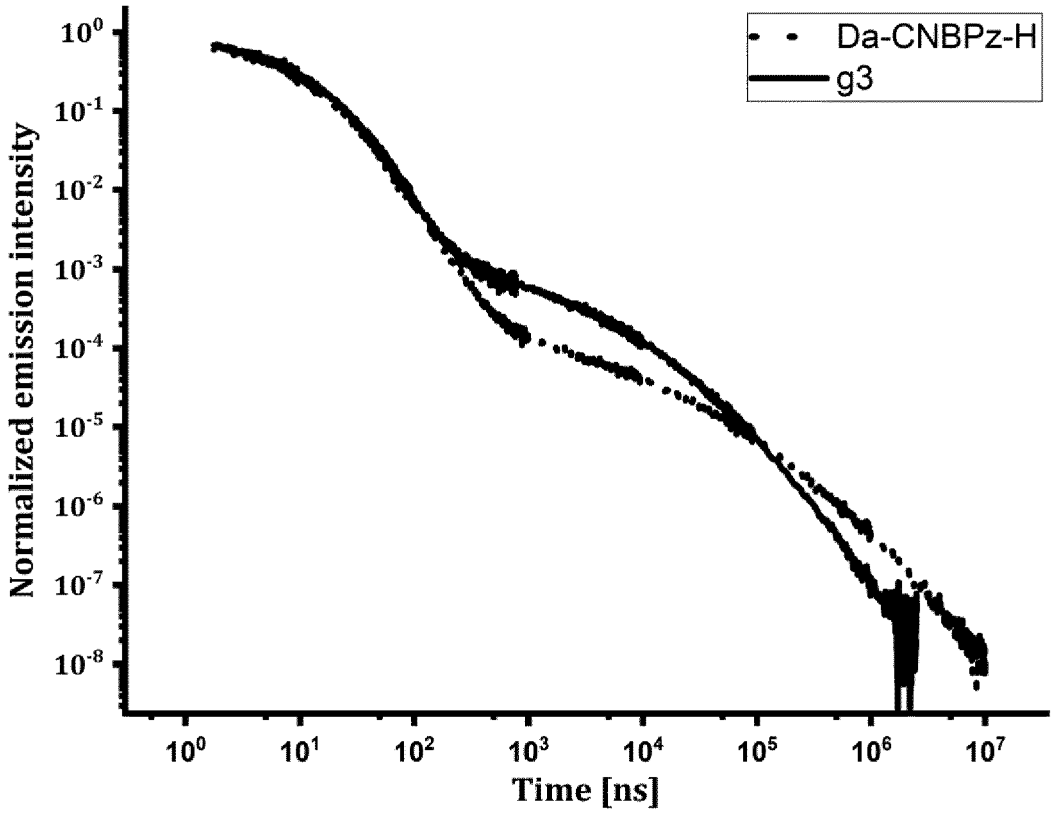


Figure 42. Comparison of PL decay of Da-CNBPz-H and g3.

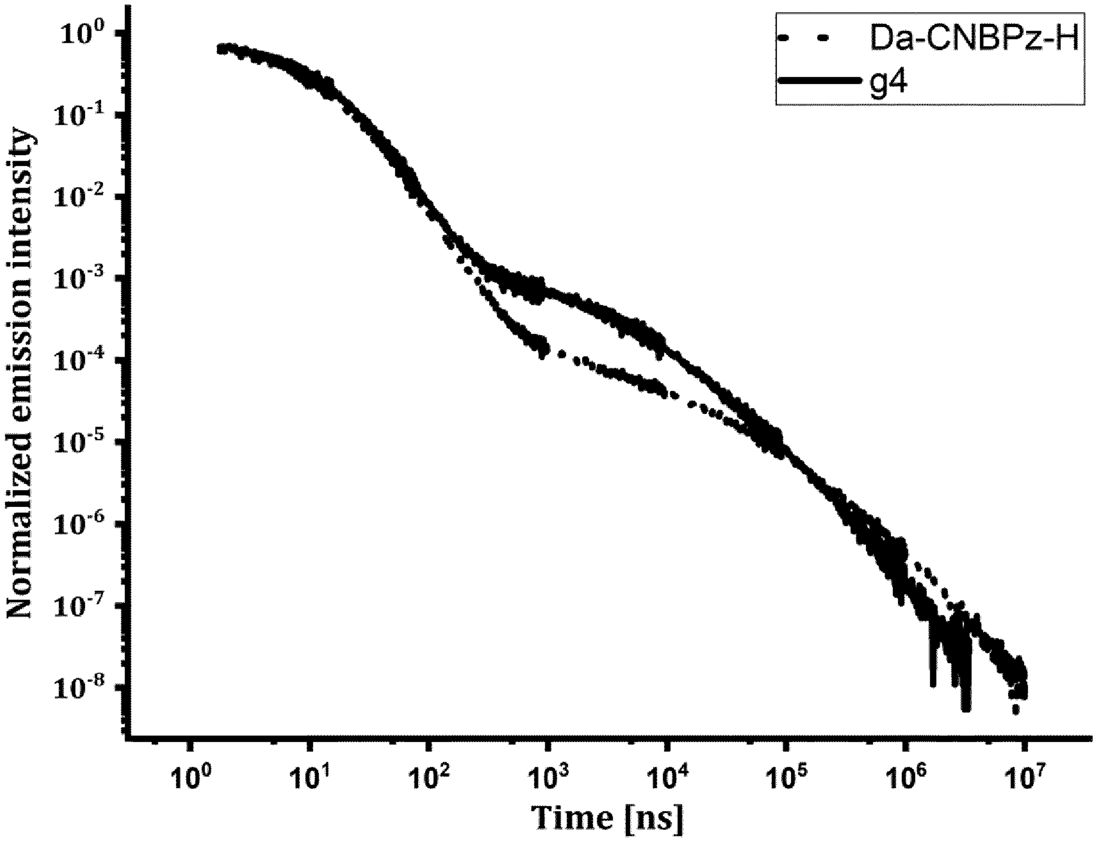


Figure 43. Comparison of PL decay of Da-CNBPz-H and g4.

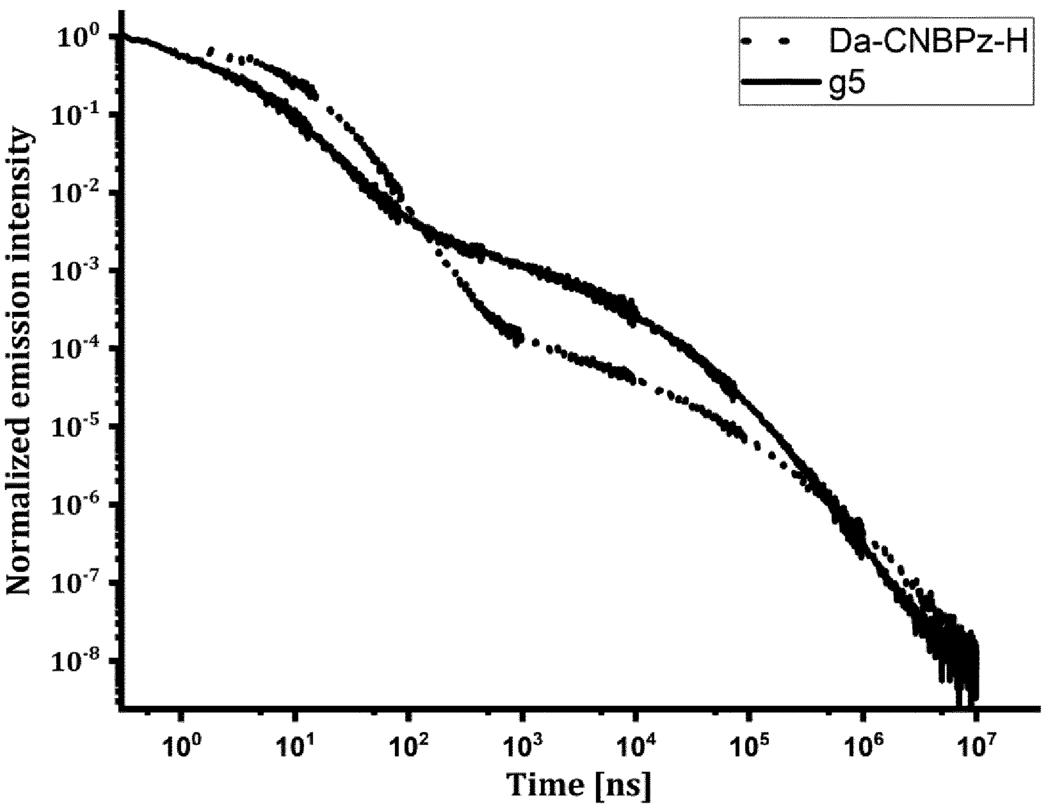

Figure 44. Comparison of PL decay of Da-CNBPz-H and g5.

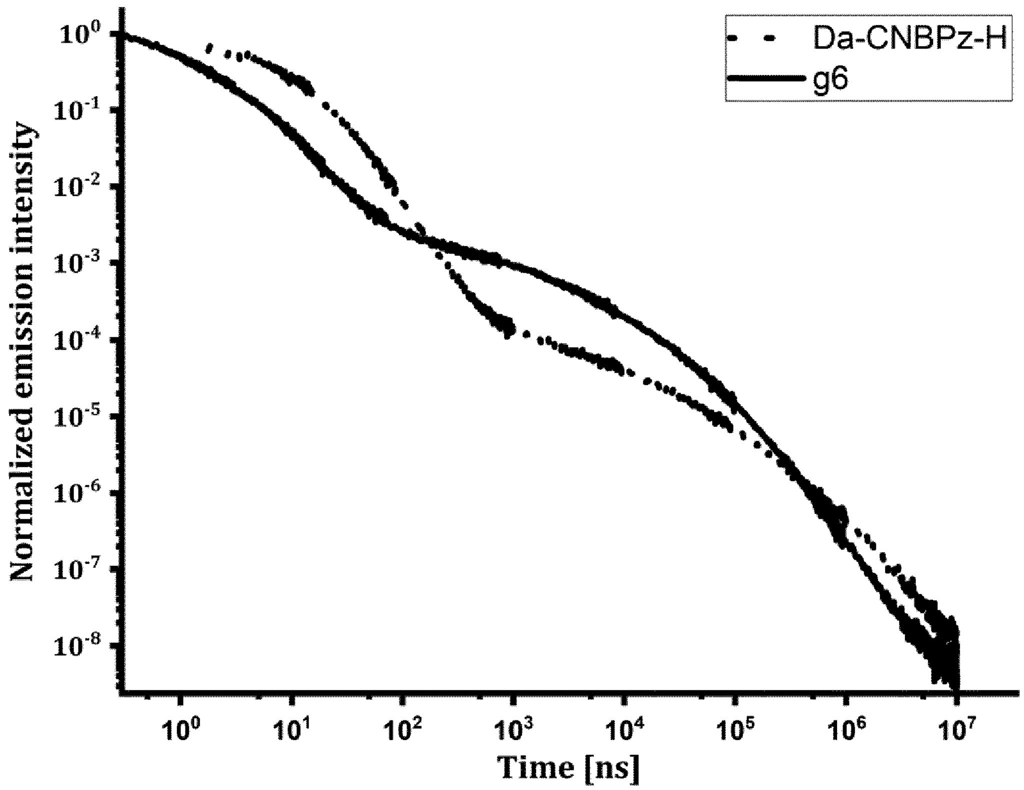

Figure 45. Comparison of PL decay of Da-CNBPz-H and g6.

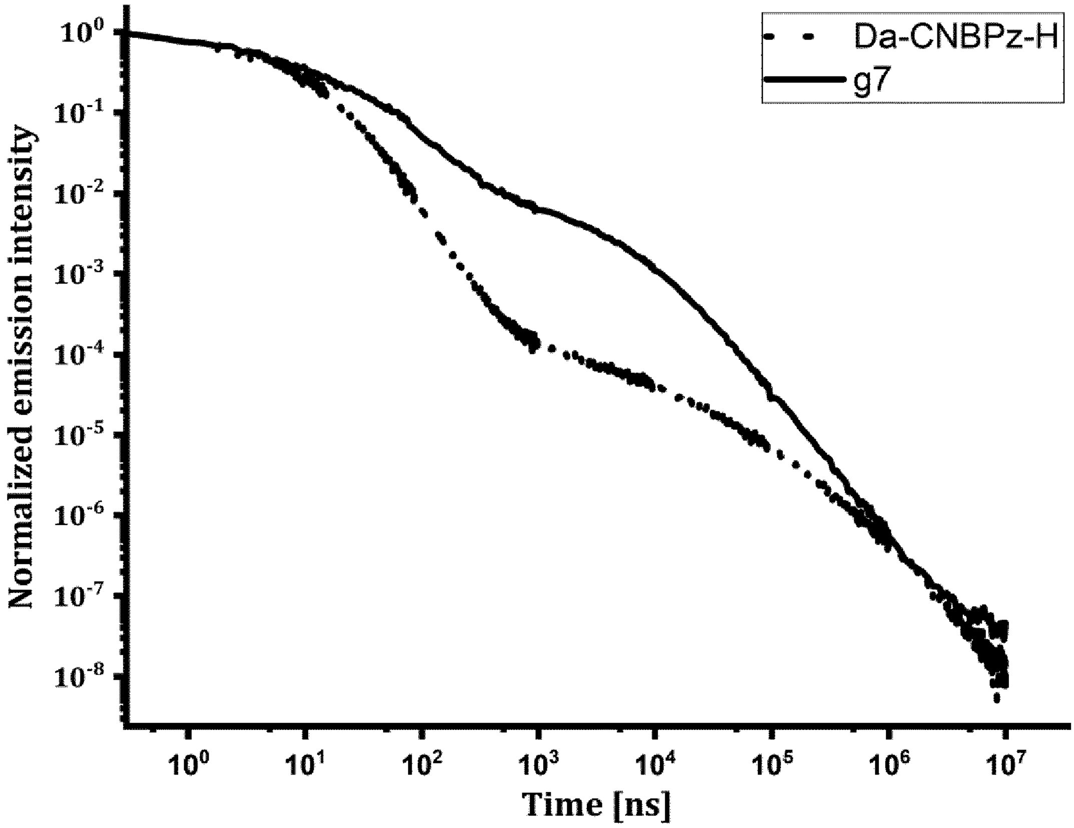


Figure 46. Comparison of PL decay of Da-CNBPz-H and g7.

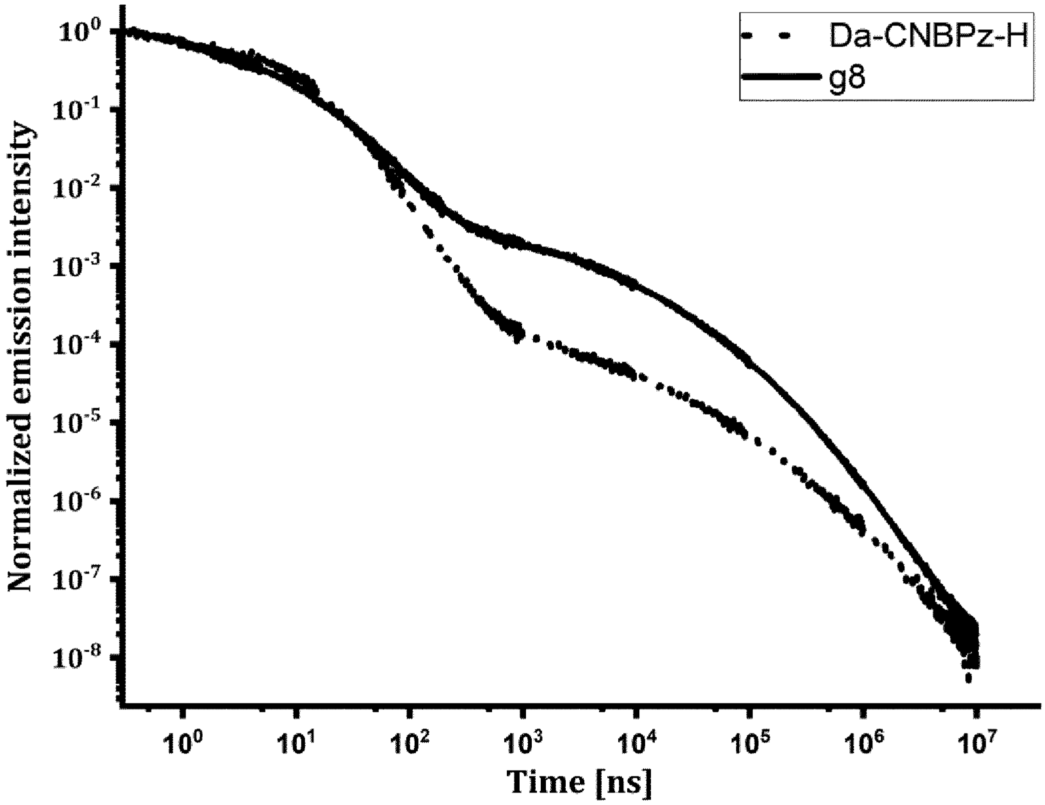


Figure 47. Comparison of PL decay of Da-CNBPz-H and g8.

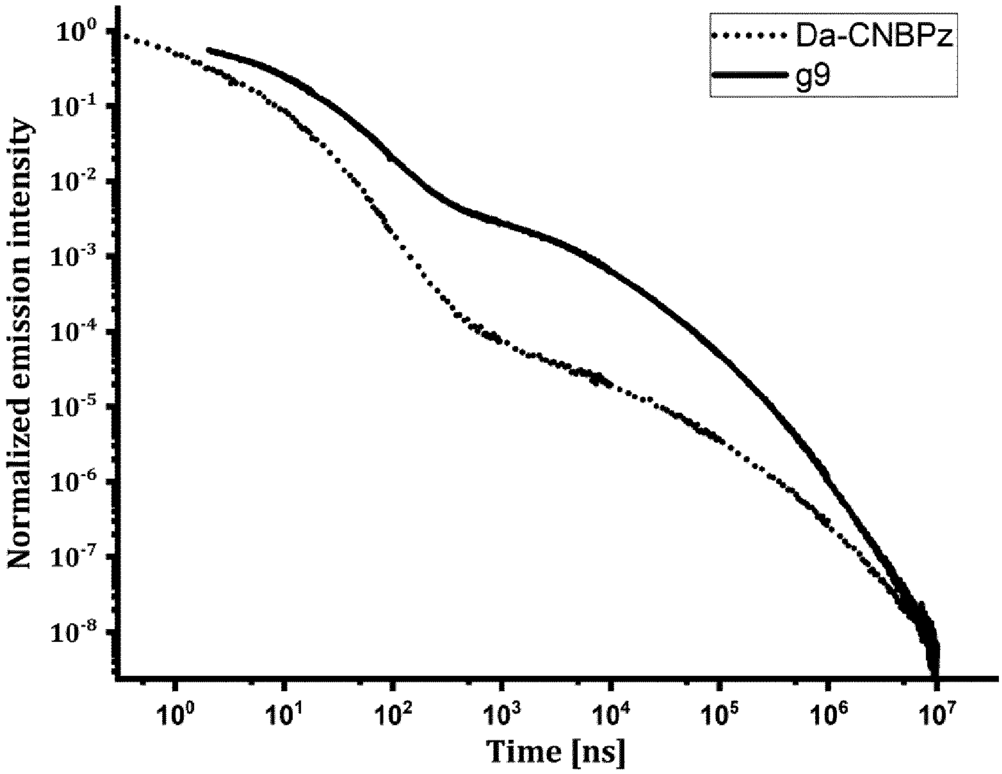


Figure 48. Comparison of PL decay of Da-CNBPz and g9.

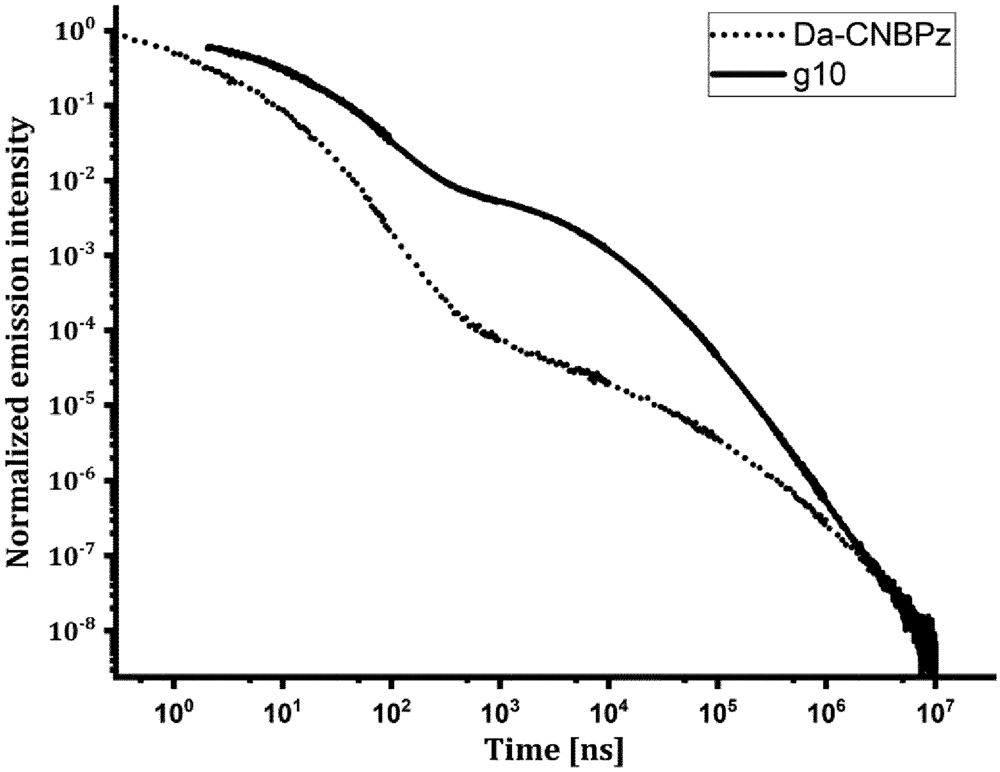


Figure 49. Comparison of PL decay of Da-CNBPz and g10.

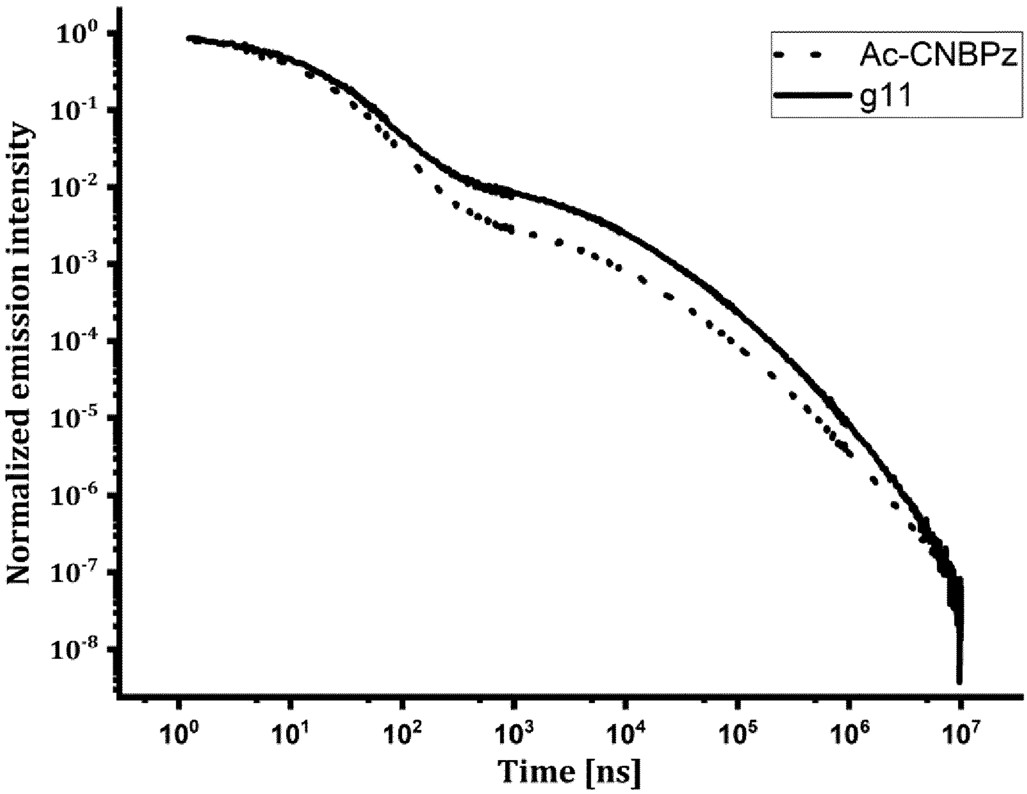


Figure 50. Comparison of PL decay of Ac-CNBPz and g11.

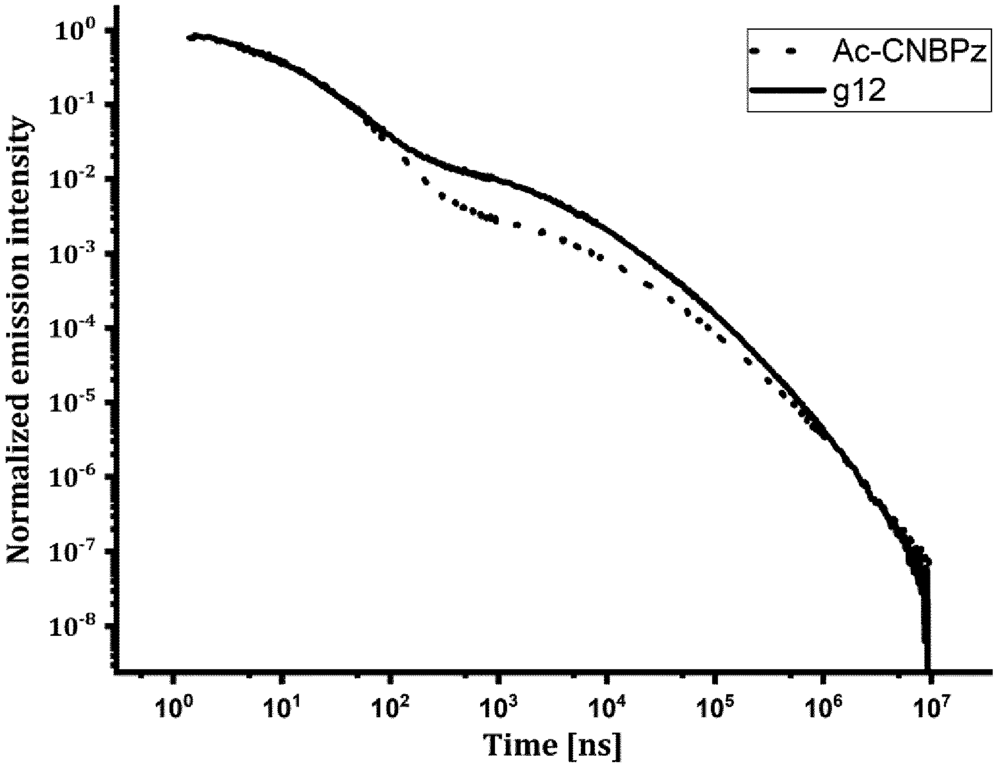


Figure 51. Comparison of PL decay of Ac-CNBPz and g12.

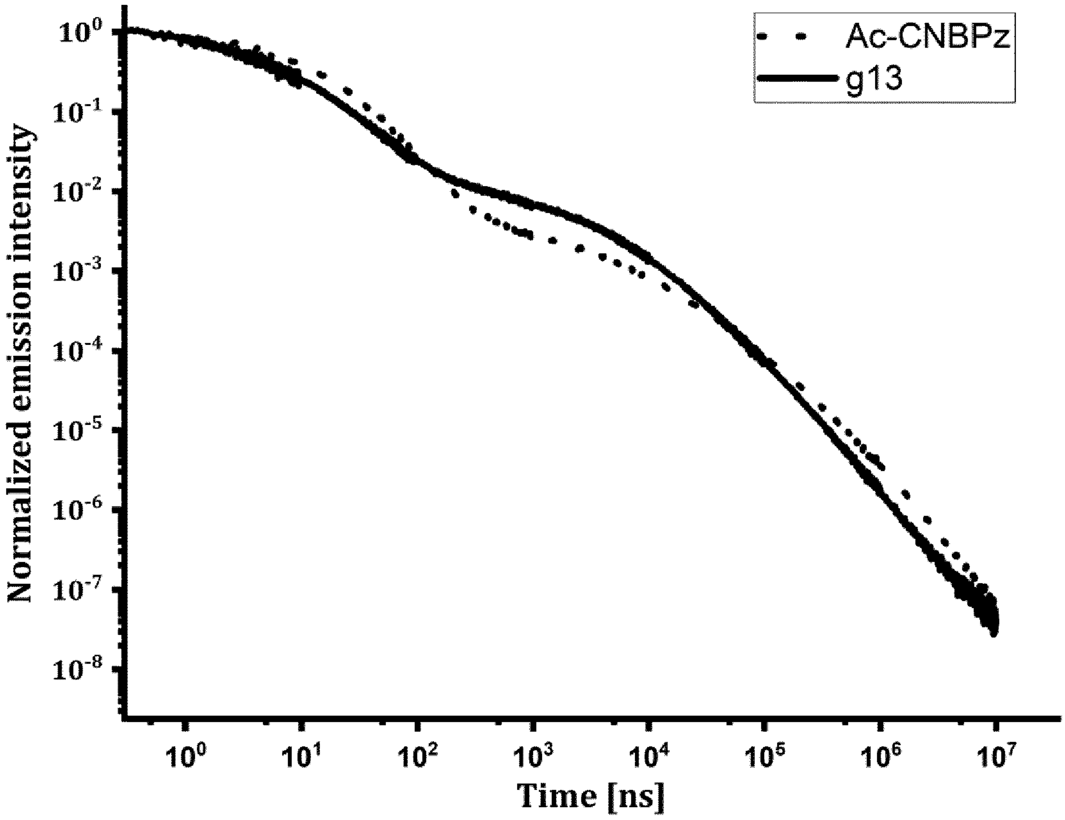

Figure 52. Comparison of PL decay of Ac-CNBPz and g13.

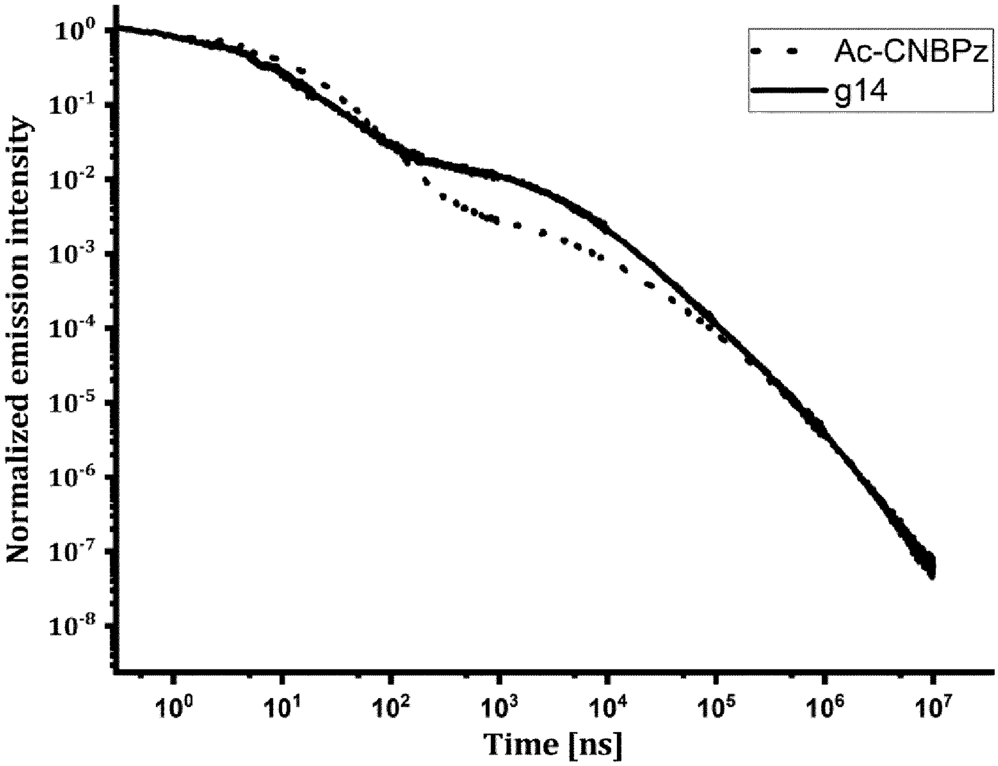


Figure 53. Comparison of PL decay of Ac-CNBPz and g14.

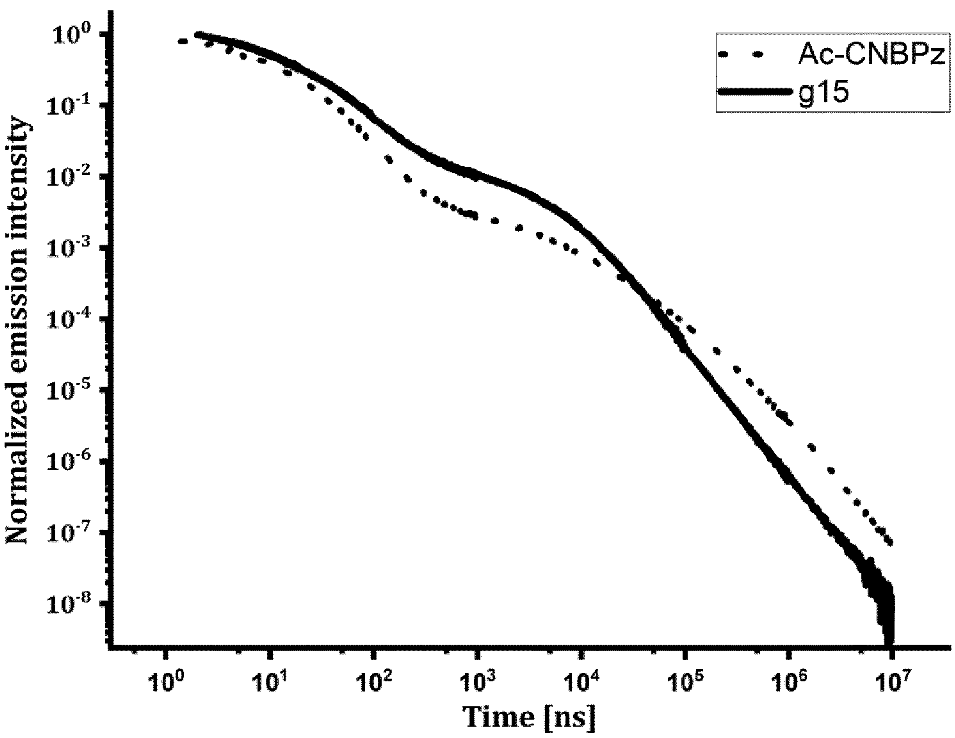


Figure 54. Comparison of PL decay of Ac-CNBPz and g15.

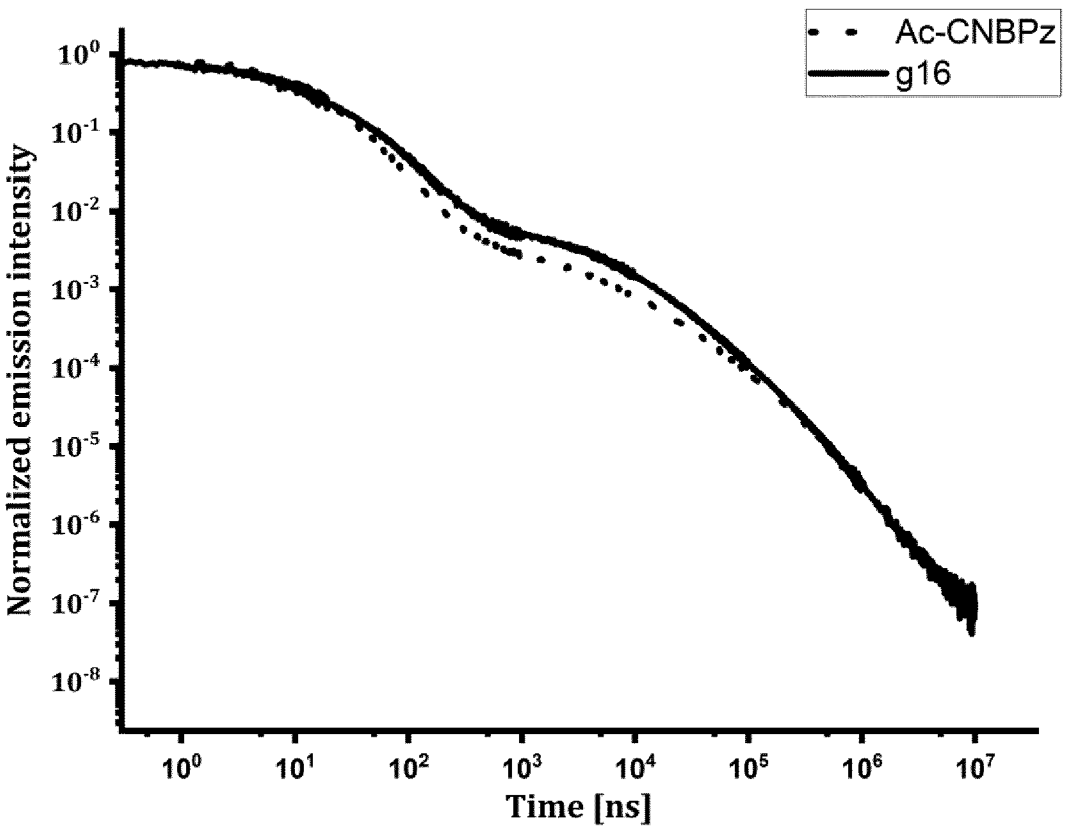


Figure 55. Comparison of PL decay of Ac-CNBPz and g16.

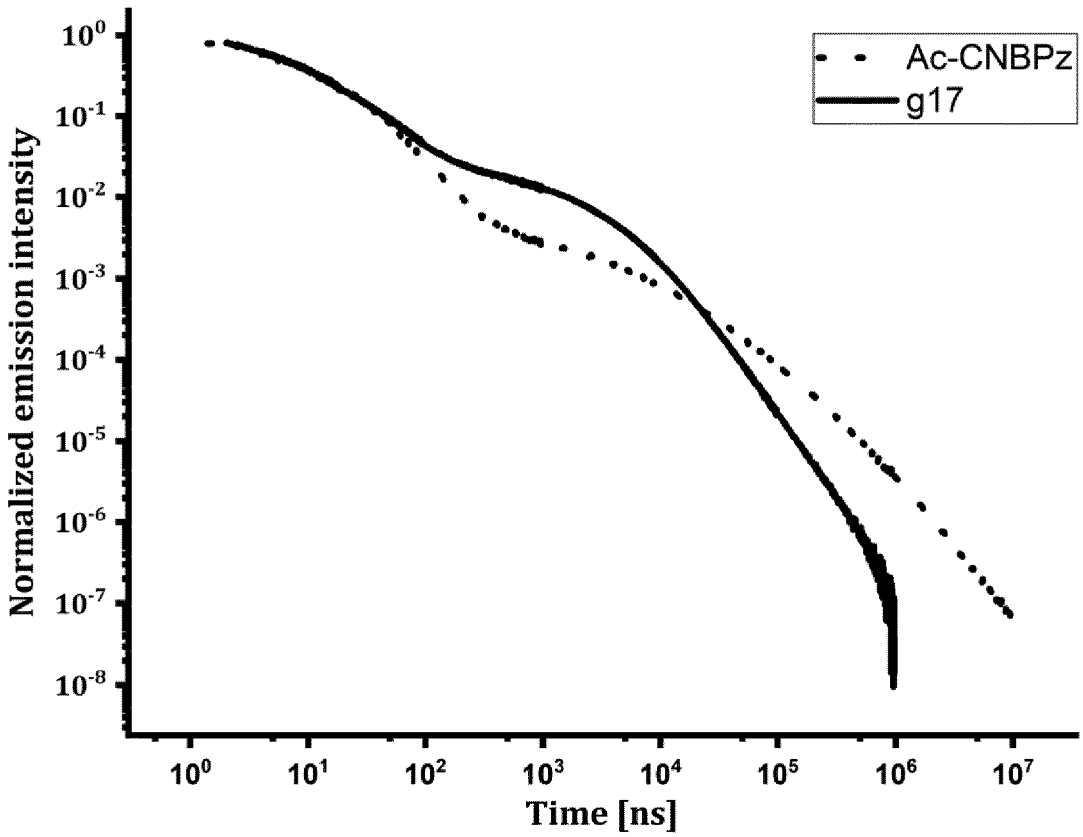


Figure 56. Comparison of PL decay of Ac-CNBPz and g17.

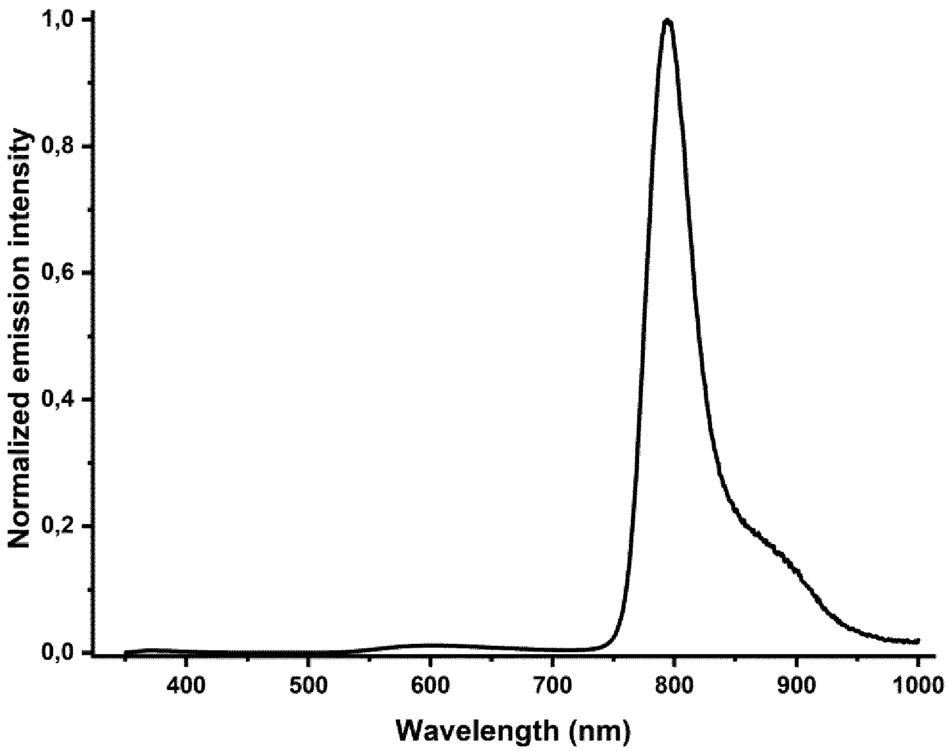

Figure 57. PL spectrum of an amorphous film of the mixture of 0.75% BPPC, 8% e6, and 91% CBP (w/w).

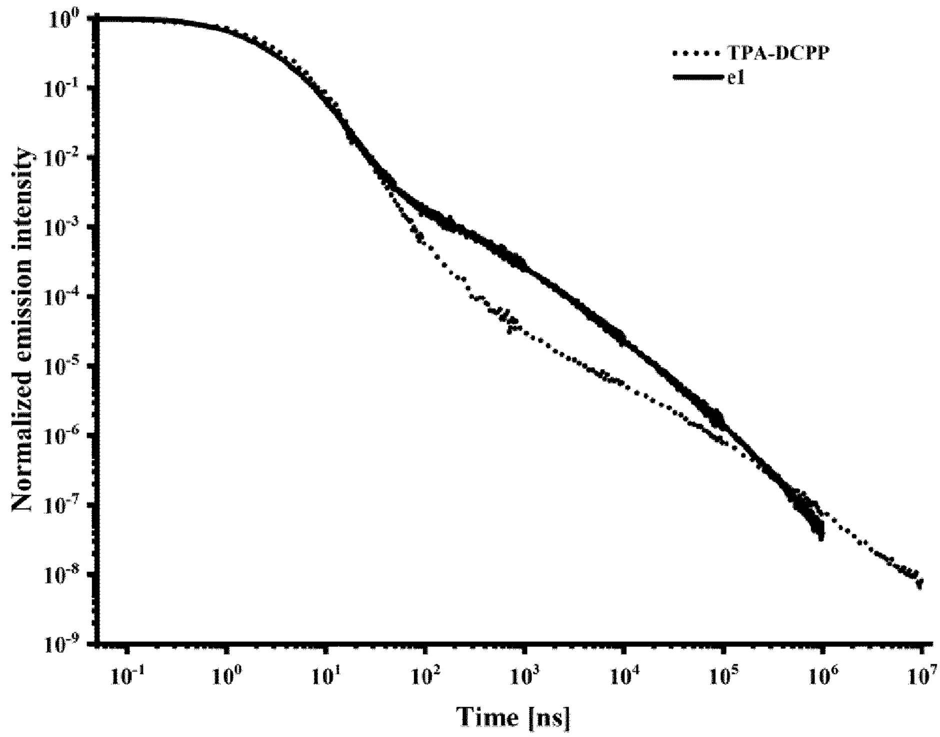


Figure 58. Comparison of PL decay of the amorphous film containing 0.5% BPPC and either 6% of e1 or 10% of TPA-DCPP (*w/w*) in CBP.

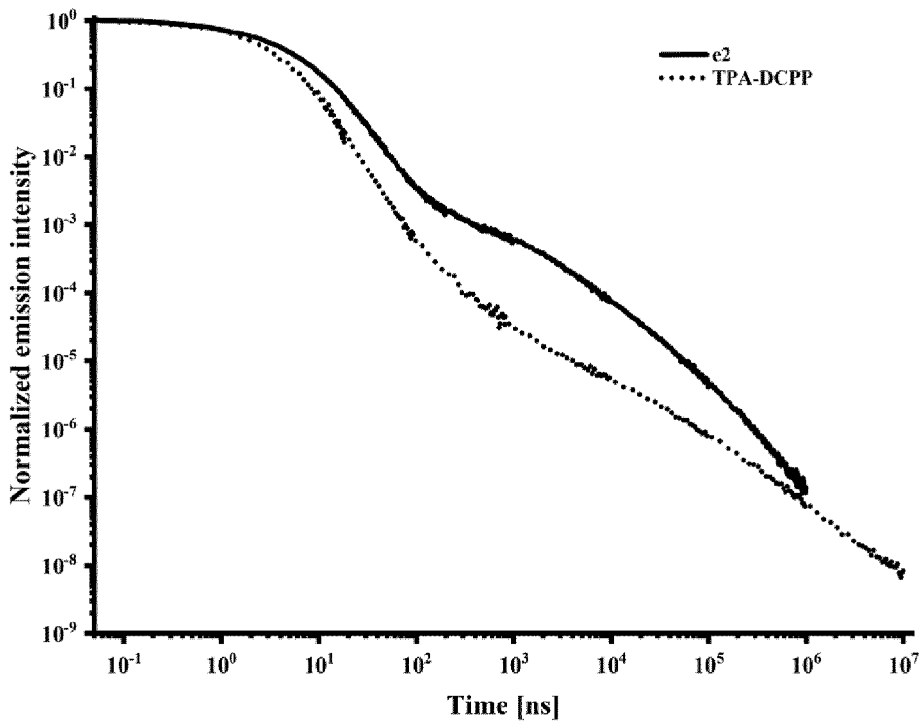

Figure 59. Comparison of PL decay of the amorphous film containing 0.75% BPPC and either 6% of e2 or 10% of TPA-DCPP (*w/w*) in CBP.

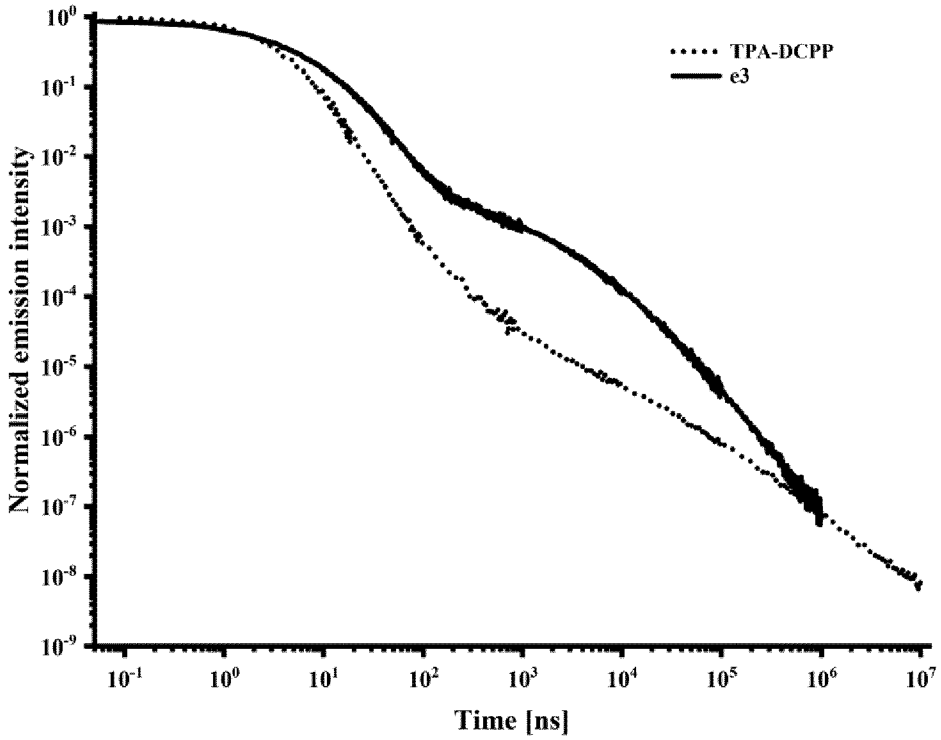


Figure 60. Comparison of PL decay of the amorphous film containing 1% BPPC and either 6% of e3 or 10% of TPA-DCPP (*w/w*) in CBP.

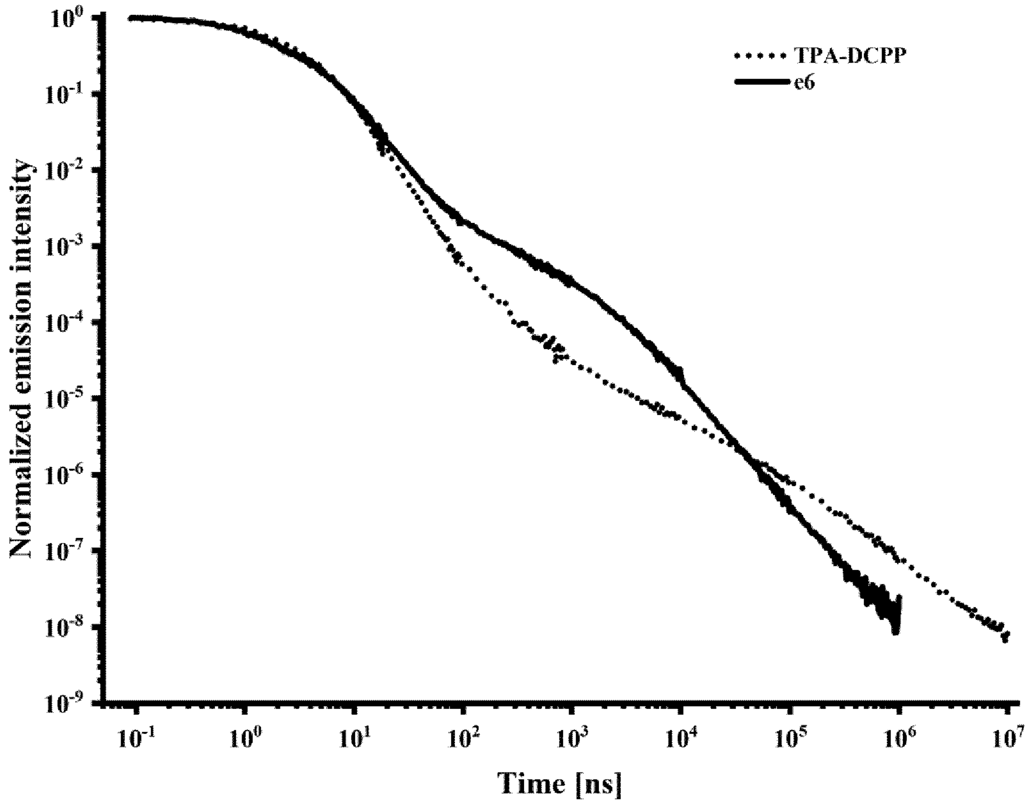


Figure 61. Comparison of PL decay of the amorphous film containing 0.75% BPPC and either 6% of e6 or 10% of TPA-DCPP (*w/w*) in CBP.

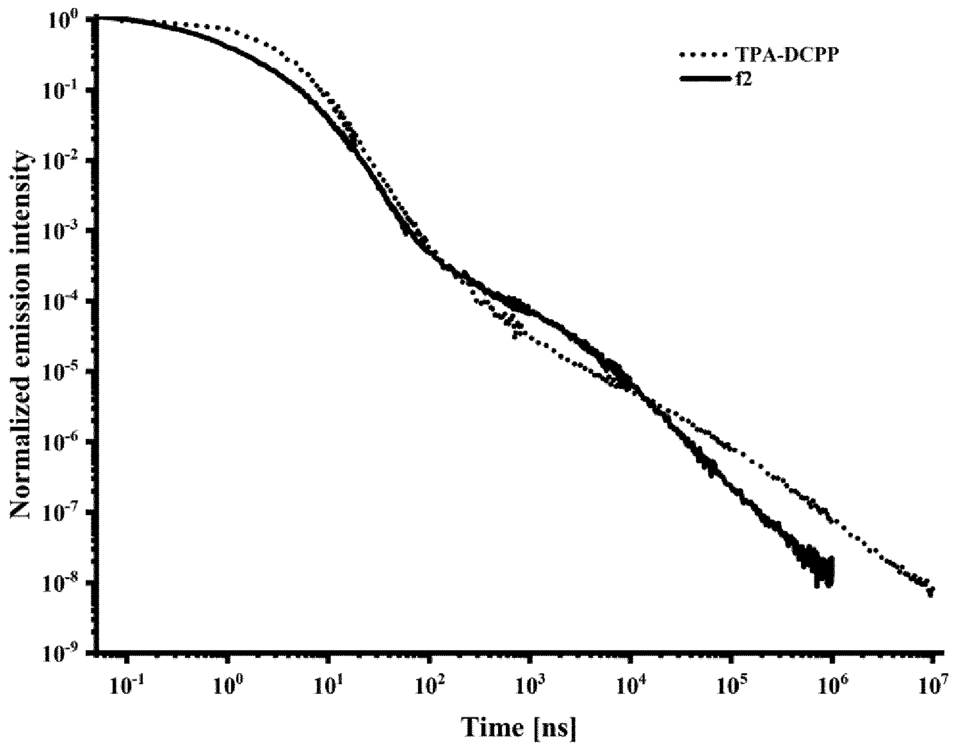


Figure 62. Comparison of PL decay of the amorphous film containing 1% BPPC and either 8% of f2 or 10% of TPA-DCPP (*w/w*) in CBP.

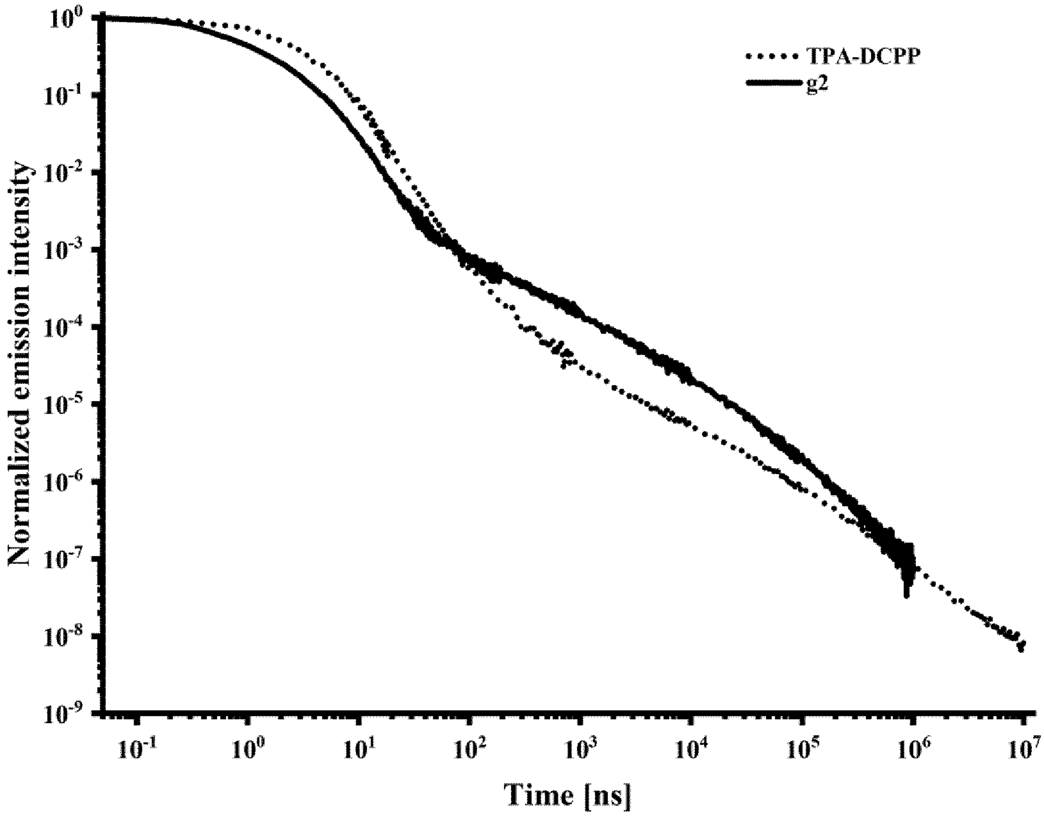


Figure 63. Comparison of PL decay of the amorphous film containing 0.5% BPPC and either 6% of g2 or 10% of TPA-DCPP (*w/w*) in CBP.

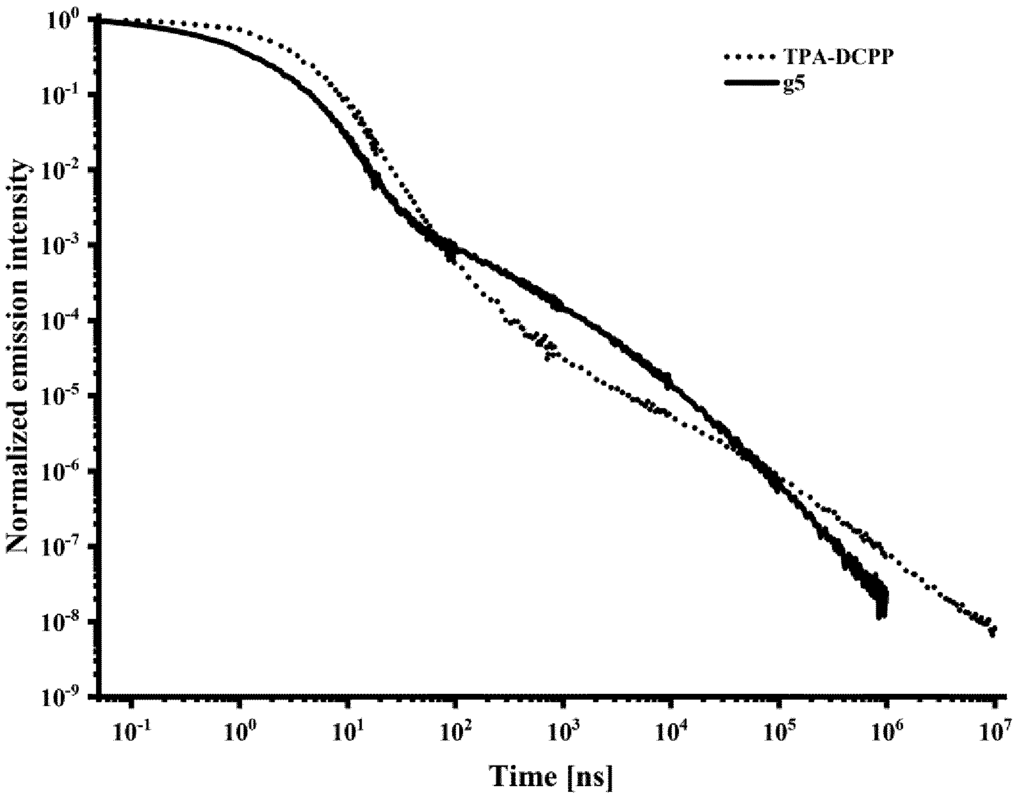

Figure 64. Comparison of PL decay of the amorphous film containing 0.5% BPPC and either 6% of g5 or 10% of TPA-DCPP (*w/w*) in CBP.

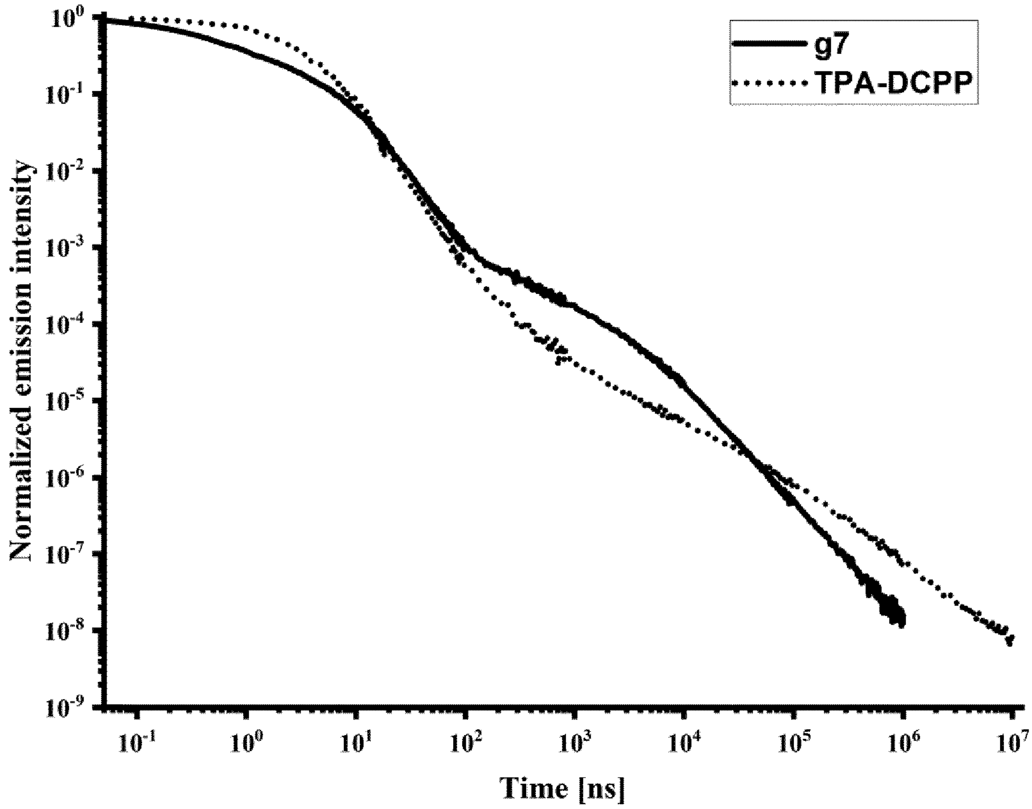


Figure 65. Comparison of PL decay of the amorphous film containing 0.75% BPPC and either 8% of g7 or 10% of TPA-DCPP (*w/w*) in CBP.

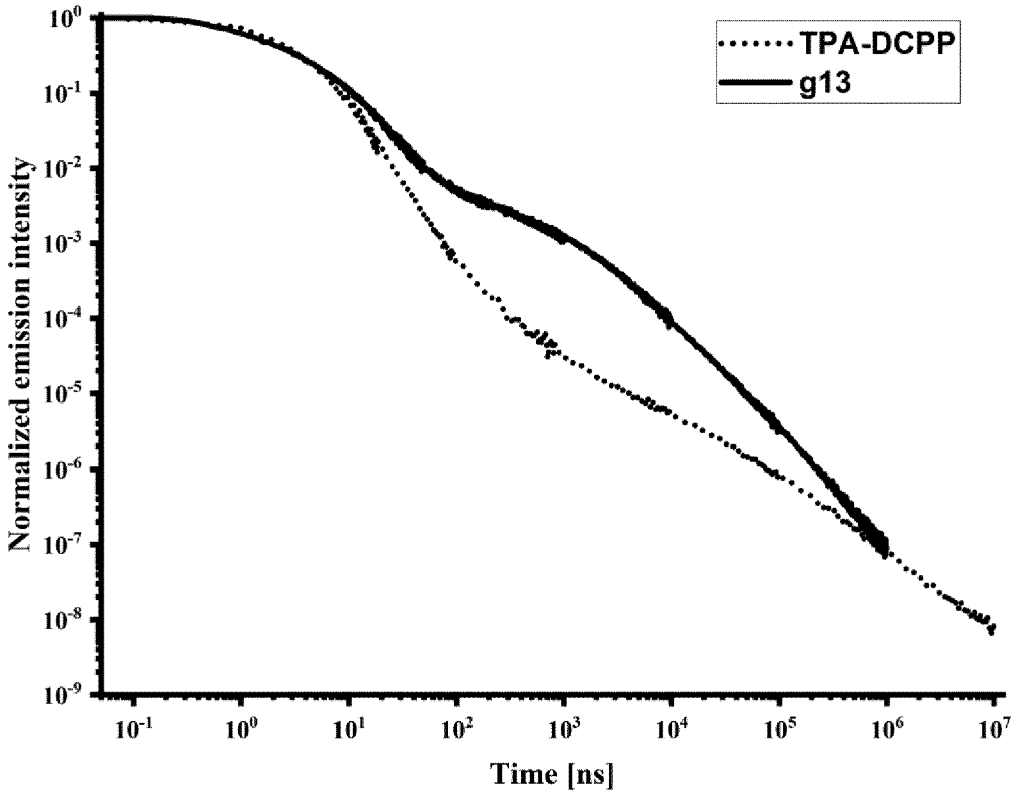


Figure 66. Comparison of PL decay of the amorphous film containing 0.75% BPPC and either 6% of g13 or 10% of TPA-DCPP (*w/w*) in CBP.

Europäisches Patentamt
European Patent Office
Office européen des brevets

# EUROPEAN SEARCH REPORT

Application Number
EP 23 17 5917

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MONKA MICHAL ET AL: "Decisive role of heavy-atom orientation for efficient enhancement of spin-orbit coupling in organic thermally activated delayed fluorescence emitters", JOURNAL OF MATERIALS CHEMISTRY C, vol. 10, no. 32, 1 January 2022 (2022-01-01), pages 11719-11729, XP93101782, GB ISSN: 2050-7526, DOI: 10.1039/D2TC01729F | 1-3 | INV. C07D241/36 C07D403/10 |
| Y | * Experimental part; figures 1,5 * | 1-3 | |
| Y | EP 3 569 599 A1 (UNIV KYUSHU NAT UNIV CORP [JP]; NIPPON SODA CO [JP]) 20 November 2019 (2019-11-20) * Examples; claim 1 * | 1-3 | |
| A | CN 104 830 320 A (UNIV JILIN) 12 August 2015 (2015-08-12) * the whole document * | 1-3 | |

| TECHNICAL FIELDS SEARCHED (IPC) |
|---|
| C07D |

The present search report has been drawn up for all claims

1

| Place of search | Date of completion of the search | Examiner |
|---|---|---|
| Munich | 16 November 2023 | Baston, Eckhard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO.

EP 23 17 5917

This annex lists the patent family members relating to the patent documents cited in the above-mentioned European search report.
The members are as contained in the European Patent Office EDP file on
The European Patent Office is in no way liable for these particulars which are merely given for the purpose of information.

16-11-2023

| Patent document cited in search report | | Publication date | Patent family member(s) | | | Publication date |
|---|---|---|---|---|---|---|
| EP 3569599 | A1 | 20-11-2019 | CN | 110167927 | A | 23-08-2019 |
| | | | EP | 3569599 | A1 | 20-11-2019 |
| | | | JP | 6760616 | B2 | 23-09-2020 |
| | | | JP | WO2018131557 | A1 | 07-11-2019 |
| | | | KR | 20190085138 | A | 17-07-2019 |
| | | | TW | 201831472 | A | 01-09-2018 |
| | | | US | 2019330162 | A1 | 31-10-2019 |
| | | | WO | 2018131557 | A1 | 19-07-2018 |
| CN 104830320 | A | 12-08-2015 | NONE | | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2019330162 A
- WO 2016084008 A


### Non-patent literature cited in the description


- **J. BRODEUR** ; **L. HU** ; **A. MALINGE** ; **E. EIZNER** ; **W. G. SKENE** ; **S. KÉNA-COHEN**. Highly Efficient and Spectrally Narrow Near-Infrared Fluorescent OLEDs Using a TADF-Sensitized Cyanine Dye. *Adv. Opt. Mater.*, 2019, vol. 7, 1901144
- Glossary of terms used in physical organic chemistry. IUPAC Recommendations, PAC, 1994, vol. 66, 1171
- **J. BRODEUR** ; **L. HU** ; **A. MALINGE** ; **E. EIZNER** ; **W. G. SKENE** ; **S. KÉNA-COHEN.** Highly Efficient and Spectrally Narrow Near-Infrared Fluorescent OLEDs Using a TADF-Sensitized Cyanine. *Dye. Adv. Opt. Mater.*, 2019, vol. 7, 1901144
- **J. BRODEUR** ; **L. HU** ; **A. MALINGE** ; **E. EIZNER** ; **W. G. SKENE** ; **S. KÉNA-COHEN.** Highly Efficient and Spectrally Narrow Near-infrared Fluorescent OLEDs Using a TADF-Sensitized Cyanine Dye. *Adv. Opt. Mater.*, 2019, vol. 7, 1901144